# EUROPÄISCHE PATENTSCHRIFT

(11) **EP 1 709 009 B1**
(45) Veröffentlichungstag und Bekanntmachung des Hinweises auf die Patenterteilung: **20.11.2013**
(21) Anmeldenummer: 04804457.2
(22) Anmeldetag: 31.12.2004
(51) Int. Cl.: C07D 239/47, C07D 403/04, C07D 409/06, C07D 409/14, A61K 31/513, A61P 25/28

(54) **6-AMINO-5-CYANO-PYRIMIDIN-4-ONE ZUR VERBESSERUNG VON WAHRNEHMUNG, KONZENTRATIONS-, LERN- UND/ODER GEDÄCHTNISLEISTUNG**
6-AMINO-5-CYANO-PYRIMIDINE-4-ONES USED FOR IMPROVING PERCEPTION, POWER OF CONCENTRATION, LEARNING EFFICIENCY, AND/OR MEMORY POWER
6-AMINO-5-CYANO-PYRIMIDIN-4-ONES POUR AMELIORER LA PERCEPTION ET LES APTITUDES DE CONCENTRATION, D'APPRENTISSAGE ET/OU DE MEMORISATION

(30) Priorität: 14.01.2004 DE 102004001873
(43) Veröffentlichungstag der Anmeldung: 11.10.2006
(73) Patentinhaber: Boehringer Ingelheim International GmbH, 55216 Ingelheim am Rhein (DE)
(72) Erfinder: HENDRIX, Martin, 51519 Odenthal (DE); BÄRFACKER, Lars, 46047 Oberhausen (DE); HECKROTH, Heike, 51519 Odenthal (DE); KARTHAUS, Dagmar, 42697 Solingen (DE); TERSTEEGEN, Adrian, 42115 Wuppertal (DE)
(74) Vertreter: Simon, Elke Anna Maria
(86) Internationale Anmeldenummer: PCT/EP2004/014872
(87) Internationale Veröffentlichungsnummer: WO 2005/068436

(56) Entgegenhaltungen:
- EP-A- 0 130 735
- WO-A-00/18758
- WO-A-95/10506
- WO-A-02/098864
- BAGLI, JEHAN ET AL: "Chemistry and positive inotropic effect of pelrinone and related derivatives. A novel class of 2-methylpyrimidones as inotropic agents" JOURNAL OF MEDICINAL CHEMISTRY , 31(4), 814-23 CODEN: JMCMAR; ISSN: 0022-2623, 1988, XP002300134

## Beschreibung

Die Erfindung betrifft neue Cyanopyrimidinone, Verfahren zu ihrer Herstellung, und ihre Verwendung zur Herstellung von Arzneimitteln zur Verbesserung von Wahrnehmung, Konzentrationsleistung, Lern- und/oder Gedächtnisleistung.

Inhibition von Phosphodiesterasen moduliert die Spiegel der zyklischen Nukleotide 5'-3' zyklisches Adenosinmonophosphat (cAMP) bzw. 5'-3' zyklisches Guanosinmonophosphat (cGMP). Diese zyklischen Nukleotide (cAMP und cGMP) sind wichtige second messenger und spielen daher eine zentrale Rolle in den zellulären Signaltransduktionskaskaden. Beide aktivieren unter anderem, aber nicht ausschließlich, jeweils wieder Proteinkinasen. Die von cAMP aktivierte Proteinkinase wird Proteinkinase A (PKA) genannt, die von cGMP aktivierte Proteinkinase wird Proteinkinase G (PKG) genannt. Aktivierte PKA bzw. PKG können wiederum eine Reihe zellulärer Effektorproteine phosphorylieren (z.B. Ionenkanäle, G-Protein gekoppelte Rezeptoren, Strukturproteine). Auf diese Weise können die second messengers cAMP und cGMP die unterschiedlichsten physiologischen Vorgänge in den verschiedensten Organen kontrollieren. Die zyklischen Nukleotide können aber auch direkt auf Effektormoleküle wirken. So ist z.B. bekannt, dass cGMP direkt auf Ionenkanäle wirken kann und hiermit die zelluläre Ionenkonzentration beeinflussen kann (Übersicht in: Wei et al., Prog. Neurobiol., 1998, 56: 37-64). Ein Kontrollmechanismus, um die Aktivität von cAMP und cGMP und damit diese physiologischen Vorgänge wiederum zu steuern, sind die Phosphodiesterasen (PDE). PDEs hydrolysieren die zyklischen Monophosphate zu den inaktiven Monophosphaten AMP und GMP. Es sind mittlerweile mindestens 21 PDE-Gene beschrieben (Exp. Opin. Investig. Drugs 2000, 9, 1354-3784). Diese 21 PDE-Gene lassen sich aufgrund ihrer Sequenzhomologie in 11 PDE-Familien einteilen (Nomenklatur-Vorschlag siehe http://depts.washington.edu/pde/Nomenclature.html.). Einzelne PDE-Gene innerhalb einer Familie werden durch Buchstaben unterschieden (z.B. PDE1A und PDE1B). Falls noch unterschiedliche Splice-Varianten innerhalb eines Genes vorkommen, wird dies dann durch eine zusätzliche Nummerierung nach dem Buchstaben angegeben (z.B. PDE1A1).

Die humane PDE9A wurde 1998 kloniert und sequenziert. Die Aminosäurenidentität zu anderen PDEs liegt bei maximal 34% (PDE8A) und minimal 28% (PDE5A). Mit einer Michaelis-Menten-Konstante (Km-Wert) von 170 nM ist PDE9A hochaffin für cGMP. Darüber hinaus ist PDE9A selektiv für cGMP (Km-Wert für cAMP = 230 µM). PDE9A weist keine cGMP-Bindungsdomäne auf, die auf eine allosterische Enzymregulation durch cGMP schließen ließe. In einer Western Blot Analyse wurde gezeigt, dass die PDE9A im Mensch unter anderem in Hoden, Gehirn, Dünndarm, Skelettmuskulatur, Herz, Lunge, Thymus und Milz exprimiert wird. Die höchste Expression wurde in Gehirn, Dünndarm, Herz und Milz gefunden (Fisher et al., J. Biol. Chem., 1998, 273 (25): 15559-15564). Das Gen für die humane PDE9A liegt auf Chromosom 21q22.3 und enthält 21 Exons. Bislang wurden 4 alternative Splice-Varianten der PDE9A identifiziert (Guipponi et al., Hum. Genet., 1998, 103: 386-392). Klassische PDE-Inhibitoren hemmen die humane PDE9A nicht. So zeigen IBMX, Dipyridamole, SKF94120, Rolipram und Vinpocetin in Konzentrationen bis 100 µM keine Inhibition am isolierten Enzym. Für Zaprinast wurde ein IC₅₀-Wert von 35 µM nachgewiesen (Fisher et al., J. Biol. Chem., 1998, 273 (25): 15559-15564).

Die Maus-PDE9A wurde 1998 von Soderling et al. (J. Biol. Chem., 1998, 273 (19): 15553-15558) kloniert und sequenziert. Diese ist wie die humane Form hochaffin für cGMP mit einem Km von 70 nM. In der Maus wurde eine besonders hohe Expression in der Niere, Gehirn, Lunge und Herz gefunden. Auch die Maus-PDE9A wird von IBMX in Konzentrationen unter 200 µM nicht gehemmt; der IC₅₀-Wert für Zaprinast liegt bei 29 µM (Soderling et al., J. Biol. Chem., 1998, 273 (19): 15553-15558). Im Rattengehim wurde gezeigt, dass PDE9A in einigen Hirnregionen stark exprimiert wird. Dazu zählen der Bulbus olfactorius, Hippocampus, Cortex, Basalganglien und basales Vorderhirn (Andreeva et al., J. Neurosci., 2001, 21 (22): 9068-9076). Insbesondere Hippocampus, Cortex und basales Vorderhirn spielen eine wichtige Rolle an Lern- und Gedächtnisvorgängen.

Wie oben bereits erwähnt, zeichnet sich PDE9A durch eine besonders hohe Affinität für cGMP aus. Deshalb ist PDE9A im Gegensatz zu PDE2A (Km = 10 µM; Martins et al., J. Biol. Chem., 1982, 257: 1973-1979), PDE5A (Km = 4 µM; Francis et al., J. Biol. Chem., 1980, 255: 620-626), PDE6A (Km = 17 µM; Gillespie and Beavo, J. Biol. Chem., 1988, 263 (17): 8133-8141) und PDE11A (Km = 0.52 µM; Fawcett et al., Proc. Nat. Acad. Sci., 2000, 97 (7): 3702-3707) schon bei niedrigen physiologischen Konzentrationen aktiv. Im Gegensatz zu PDE2A (Murashima et al., Biochemistry, 1990, 29: 5285-5292) wird die katalytische Aktvität von PDE9A nicht durch cGMP gesteigert, da es keine GAF-Domäne (cGMP-Bindedomäne, über die die PDE Aktivität allosterisch gesteigert wird) aufweist (Beavo et al., Current Opinion in Cell Biology, 2000, 12: 174-179). PDE9A-Inhibitoren können deshalb zu einer Erhöhung der basalen cGMP Konzentration führen.

Die US 5,002,949 offenbart Cyanopyrimidinone zur Inhibierung von weißen Thrombusformationen.

In WO 02/06288 werden Cyanopyrimidinone mit mGluR antagonistischer Wirkung beschrieben.

In WO 95/10506 offenbart Cyanopyrimidinone zur Behandlung von Depression und der Alzheimer'schen Krankheit.

In EP 130735 werden Cyanopyrimidine als cardiotonische Reagentien beschrieben.

US 5,256,668 und WO 99/41253 offenbaren Cyanopyrimidine mit antiviraler Wirkung.

Die vorliegende Erfindung betrifft Verbindungen der Formel 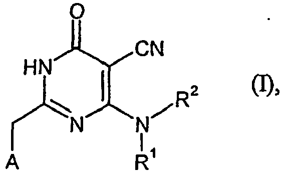 in welcher
- A: Phenyl, Heteroaryl oder eine Gruppe der Formel 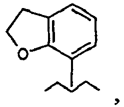 wobei Phenyl und Heteroaryl gegebenenfalls mit bis zu 2 Resten unabhängig voneinander ausgewählt aus der Gruppe Heteroaryl, Halogen, C₁-C₆-Alkyl, C₁-C₆-Alkoxy, Trifluormethyl, Trifluormethoxy, Benzyloxy und Benzyl substituiert sind,
wobei C₁-C₆-Alkyl gegebenenfalls mit einer Gruppe der Formel -NR³R⁴, in welcher R³ C₁-C₆-Alkyl und R⁴ Wasserstoff oder C₁-C₆-Alkoxy(C₁-C₆)-alkyl bedeuten, und
Heteroaryl gegebenenfalls mit C₁-C₆-Alkoxy substituiert ist,
- R¹: C₃-C₈-Cycloalkyl, C₁-C₆-Alkyl, C₁-C₆-Alkoxy(C₁-C₆)alkyl, Benzyl oder eine Gruppe der 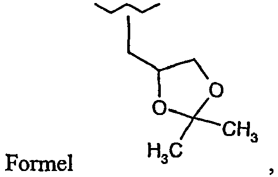 wobei C₃-C₈-Cycloalkyl gegebenenfalls mit Hydroxy, C₁-C₆-Alkyl oder Trifluormethyl,
C₁-C₆-Alkyl gegebenenfalls mit Heteroaryl, C₃-C₈-Cycloalkyl oder Hydroxy,
und Benzyl gegebenenfalls mit C₁-C₆-Alkoxy oder Halogen substituiert ist,
- R²: Wasserstoff,
oder
- R¹ und R²: zusammen mit dem Stickstoffatom, an dem sie gebunden sind, ein 5- bis 6-gliedriges Heterocyclyl bilden, welches gegebenenfalls mit bis zu 2 Substituenten unabhängig voneinander ausgewählt aus der Gruppe C₁-C₆-Alkyl, Hydroxy, Cyano, Oxo, Heteroaryl, Benzyl, Formyl, C₁-C₆-Alkylcarbonyl und einer der folgenden Gruppen 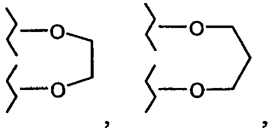 die über die beiden Sauerstoffatome an eines der Kohlenstoffatome im Heterocyclus gebunden sind, substituiert ist,
wobei C₁-C₆-Alkyl gegebenenfalls mit Hydroxy oder Heteroaryl substituiert ist,
bedeuten, sowie deren Salze, Solvate und/oder Solvate der Salze, wobei die Verbindung 2-Benzyl-4-oxo-6[(pyridin-3-ylmethyl)-amino]-4,5-dihydro-pyrimidin-5-carbonitril ausgeschlossen ist:
Die erfindungsgemäßen Verbindungen können in Abhängigkeit von ihrer Struktur in stereoisomeren Formen (Enantiomere, Diastereomere) und tautomeren Formen existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Als Salze sind im Rahmen der Erfindung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Dehydroabietylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:
C₁₋C₆-Alkyl steht für einen geradkettigen oder verzweigten Alkylrest mit 1 bis 6, bevorzugt 1 bis 4 Kohlenstoffatomen. Bevorzugte Beispiele umfassen Methyl, Ethyl, n-Propyl, Isopropyl, 2-Butyl, tert.-Butyl, 2-Pentyl, 3-Pentyl und n-Hexyl.
C₁-C₆-Alkoxy steht für einen geradkettigen oder verzweigten Alkoxyrest mit 1 bis 6, bevorzugt 1 bis 4, besonders bevorzugt mit 1 bis 3 Kohlenstoffatomen. Bevorzugte Beispiele umfassen Methoxy, Ethoxy, n-Propoxy, Isopropoxy, tert.-Butoxy, n-Pentoxy und n-Hexoxy.
C₁-C₆-Alkoxy(C₁-C₆)-alkyl steht für einen geradkettigen oder verzweigten Alkoxyrest mit 1 bis 6, bevorzugt 1 bis 4, besonders bevorzugt mit 1 bis 3 Kohlenstoffatomen, der an einen geradkettigen oder verzweigten Alkylrest mit 1 bis 6, bevorzugt 1 bis 4, besonders bevorzugt mit 2 bis 3 Kohlenstoffatomen gebunden ist. Bevorzugte Beispiele umfassen Methoxymethyl, 2-Methoxyethyl, Ethoxymethyl und 2-Ethoxyethyl.
C₁-C₆-Alkylcarbonyl steht für einen geradkettigen oder verzweigten Alkylcarbonylrest mit 1 bis 6, bevorzugt 1 bis 4 und besonders bevorzugt 1 bis 3 Kohlenstoffatomen. Bevorzugte Beispiele umfassen Methylcarbonyl, Ethylcarbonyl, n-Propylcarbonyl, Isopropylcarbonyl und tert.-Butylcarbonyl.
3- bis 8-gliedriges Cycloalkyl steht für gesättigte Cycloalkylreste mit 3 bis 8, bevorzugt 3 bis 6 und besonders bevorzugt 5 bis 6 Kohlenstoffatomen im Cyclus. Bevorzugte Beispiele umfassen Cyclopropyl, Cyclobutyl, Cyclopentyl, Cyclohexyl und Cycloheptyl.
Halogen steht für Fluor, Chlor, Brom und Iod. Bevorzugt sind Fluor, Chlor, Brom, besonders bevorzugt Fluor und Chlor.
Heteroaryl steht für einen aromatischen, monocyclischen Rest mit 5 bis 6 Ringatomen und bis zu 3 Heteroatomen aus der Reihe S, O und/oder N. Bevorzugt sind 5- bis 6-gliedrige Heteroaryle mit bis zu 2 Heteroatomen. Der Heteroarylrest kann über ein Kohlenstoff- oder Stickstoffatom gebunden sein. Bevorzugte Beispiele umfassen Thienyl, Furyl, Pyrrolyl, Thiazolyl, Oxazolyl, Imidazolyl, Pyridyl, Pyrimidinyl und Pyridazinyl.
5- bis 6-gliedriges Heterocyclyl steht für einen monocyclischen, gesättigten oder partiell ungesättigten heterocyclischen Rest mit 5 bis 6 Ringatomen und bis zu 2 Heteroatomen aus der Reihe N, O, S. Als Heteroatome sind N und O bevorzugt. Bevorzugte Beispiele umfassen Pyrrolidinyl, Pyrrolinyl, Tetrahydrofuranyl, Tetrahydrothienyl, Pyranyl, Thiopyranyl, Piperidinyl, Morpholinyl, Thiomorpholinyl und Piperazinyl.

Wenn Reste in den erfindungsgemäßen Verbindungen gegebenenfalls substituiert sind, ist, soweit nicht anders spezifiziert, eine Substitution mit bis zu drei gleichen oder verschiedenen Substituenten bevorzugt.

Eine weitere Ausführungsform der Erfindung betrifft Verbindungen der Formel (I), in welcher
- A: Phenyl, Heteroaryl oder eine Gruppe der Formel 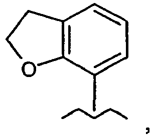 wobei Phenyl und Heteroaryl gegebenenfalls mit bis zu 2 Resten unabhängig voneinander ausgewählt aus der Gruppe Heteroaryl, Halogen, C₁-C₄-Alkyl, C₁-C₄-Alkoxy, Trifluormethyl, Trifluormethoxy, Benzyloxy und Benzyl substituiert sind,
wobei C₁-C₄-Alkyl gegebenenfalls mit einer Gruppe der Formel NR³R⁴, in welcher R³ C₁-C₄-Alkyl und R⁴ Wasserstoff oder C₁-C₄-Alkoxy(C₁-C₄)-alkyl bedeuten, und
Heteroaryl gegebenenfalls mit C₁-C₄-Alkoxy substituiert ist,
- R¹: C₃-C₆-Cycloalkyl, C₁-C₄-Alkyl, C₁-C₄-Alkoxy(C₁-C₄)alkyl, Benzyl oder eine Gruppe der 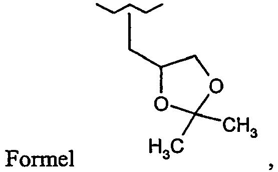 wobei C₃-C₆-Cycloalkyl gegebenenfalls mit Hydroxy, C₁-C₄-Alkyl oder Trifluormethyl,
C₁-C₄-Alkyl gegebenenfalls mit Heteroaryl, C₃-C₆-Cycloalkyl oder Hydroxy,
und Benzyl gegebenenfalls mit C₁-C₄-Alkoxy oder Halogen substituiert ist,
- R²: Wasserstoff,
oder
- R¹ und R²: zusammen mit dem Stickstoffatom, an dem sie gebunden sind, ein 5- bis 6-gliedriges Heterocyclyl bilden, welches gegebenenfalls mit bis zu 2 Substituenten unabhängig voneinander ausgewählt aus der Gruppe C₁-C₄-Alkyl, Hydroxy, Cyano, Oxo, Heteroaryl, Benzyl, Formyl, C₁-C₄-Alkylcarbonyl und einer der folgenden Gruppen 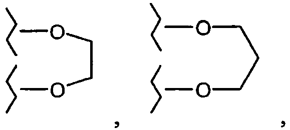 die über die beiden Sauerstoffatome an eines der Kohlenstoffatome im Heterocyclus gebunden sind, substituiert ist,
wobei C₁-C₄-Alkyl gegebenenfalls mit Hydroxy oder Heteroaryl substituiert ist,
bedeuten, sowie deren Salze, Solvate und/oder Solvate der Salze.

Eine weitere Ausführungsform der Erfindung betrifft Verbindungen der Formel (I), in welcher
- A: Phenyl, Thienyl oder eine Gruppe der Formel 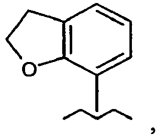 wobei Phenyl und Thienyl gegebenenfalls mit bis zu 2 Resten unabhängig voneinander ausgewählt aus der Gruppe Pyridyl, Fluor, Chlor, Brom, C₁-C₄-Alkyl, C₁-C₄-Alkoxy, Trifluormethyl, Trifluormethoxy, Benzyloxy und Benzyl substituiert sind,
wobei C₁-C₄-Alkyl gegebenenfalls mit einer Gruppe der Formel -NR³R⁴, in welcher R³ C₁-C₄-Alkyl und R⁴ Wasserstoff oder C₁-C₄-Alkoxy(C₁-C₄)-alkyl bedeuten, und
Pyridyl gegebenenfalls mit C₁-C₄-Alkoxy substituiert ist,
- R¹: C₃-C₆-Cycloalkyl, C₁-C₄-Alkyl, C₁-C₄-Alkoxy(C₁-C₄)alkyl, Benzyl oder eine Gruppe der 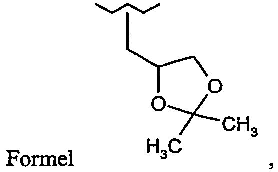 wobei C₃-C₆-Cycloalkyl gegebenenfalls mit Hydroxy, C₁-C₄-Alkyl oder Trifluormethyl,
C₁-C₄-Alkyl gegebenenfalls mit Pyridyl, C₃-C₆-Cycloalkyl oder Hydroxy,
und Benzyl gegebenenfalls mit C₁-C₄-Alkoxy, Fluor, Chlor oder Brom substituiert ist,
- R²: Wasserstoff,
oder
- R¹ und R²: zusammen mit dem Stickstoffatom, an dem sie gebunden sind, ein 5- bis 6-gliedriges Heterocyclyl ausgewählt aus der Gruppe Pyrrolidinyl, Piperidinyl, Piperazinyl und Morpholinyl bilden, welches gegebenenfalls mit bis zu 2 Substituenten unabhängig voneinander ausgewählt aus der Gruppe C₁-C₄-Alkyl, Hydroxy, Cyano, Oxo, Heteroaryl, Benzyl, Formyl, C₁-C₄-Alkylcarbonyl und einer der folgenden Gruppen 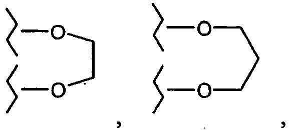 die über die beiden Sauerstoffatome an eines der Kohlenstoffatome im Heterocyclus gebunden sind, substituiert ist,
wobei C₁-C₄-Alkyl gegebenenfalls mit Hydroxy oder Pyridyl substituiert ist,
bedeuten, sowie deren Salze, Solvate und/oder Solvate der Salze.

Eine weitere Ausführungsform der Erfindung betrifft Verbindungen der Formel (I), in welcher
- A: Phenyl, Thienyl oder eine Gruppe der Formel 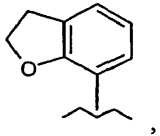 wobei Phenyl gegebenenfalls mit bis zu 2 Resten unabhängig voneinander ausgewählt aus der Gruppe Pyridyl, Fluor, Chlor, Methyl, Methoxy, Ethoxy, Trifluormethyl, Trifluormethoxy, Benzyloxy und Benzyl substituiert ist,
wobei Methyl gegebenenfalls mit einer Gruppe der Formel -NR³R⁴, in welcher R³ Methyl und R⁴ Wasserstoff oder 2-Methoxyethyl bedeuten, und
Pyridyl gegebenenfalls mit Methoxy substituiert ist,
- R¹: C₃-C₆-Cycloalkyl, Methyl, Ethyl, Propyl, 2-Methoxyethyl, Benzyl oder eine Gruppe der 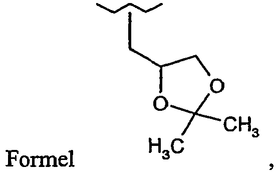 wobei C₃-C₆-Cycloalkyl gegebenenfalls mit Hydroxy, Methyl oder Trifluormethyl,
Methyl, Ethyl, Propyl gegebenenfalls mit Pyridyl, Cyclopropyl oder Hydroxy,
und Benzyl gegebenenfalls mit Methoxy, Ethoxy, Fluor oder Chlor substituiert ist,
- R²: Wasserstoff,
oder
- R¹ und R²: zusammen mit dem Stickstoffatom, an dem sie gebunden sind, ein 5- bis 6-gliedriges Heterocyclyl ausgewählt aus der Gruppe Pyrrolidinyl, Piperidinyl, Piperazinyl und Morpholinyl bilden, welches gegebenenfalls mit bis zu 2 Substituenten unabhängig voneinander ausgewählt aus der Gruppe Methyl, Ethyl, Propyl, tert.-Butyl, Hydroxy, Cyano, Oxo, Pyridyl, Benzyl, Formyl, Methylcarbonyl, Ethylcarbonyl, Propylcarbonyl und einer der folgenden Gruppen 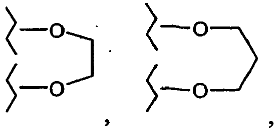 die über die beiden Sauerstoffatome an eines der Kohlenstoffatome im Heterocyclus gebunden sind, substituiert ist,
wobei Methyl, Ethyl und Propyl gegebenenfalls mit Hydroxy oder Pyridyl substituiert sind,
bedeuten, sowie deren Salze, Solvate und/oder Solvate der Salze.

Außerdem wurde ein Verfahren zur Herstellung der erfindungsgemäßen Verbindungen der Formel (I) gefunden, dadurch gekennzeichnet, dass man entweder
[A] eine Verbindung der Formel 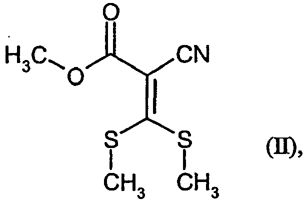 zunächst mit einer Verbindung der Formel

   HNR¹R² (III),

   in welcher
   R¹ und R² die oben angegebenen Bedeutungen aufweisen,
   bei erhöhter Temperatur in einem inerten Lösemittel oder auch in Abwesenheit eines Lösemittels in eine Verbindung der Formel 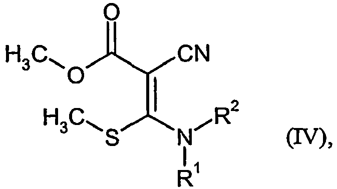 in welcher
   R¹ und R² die oben angegebenen Bedeutungen aufweisen,
   überführt und diese dann in einem inerten Lösemittel in Gegenwart einer Base mit einer Verbindung der Formel 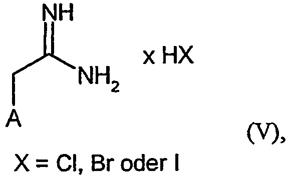 in welcher
   - A: die oben angegebenen Bedeutungen aufweist,
   umsetzt
   oder in veränderter Reihenfolge der Reaktionspartner
[B] eine Verbindung der Formel (II) zunächst mit einer Verbindung der Formel (V) in einem inerten Lösemittel in Gegenwart einer Base in eine Verbindung der Formel 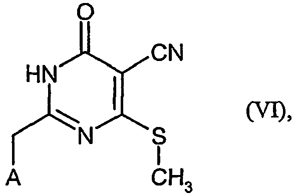 in welcher
   - A: die oben angegebenen Bedeutungen aufweist,
   überführt und diese dann bei erhöhter Temperatur in einem inerten Lösemittel oder auch in Abwesenheit eines Lösemittels mit einer Verbindung der Formel (III) umsetzt,
und die jeweils resultierenden Verbindungen der Formel (I) gegebenenfalls mit den entsprechenden (i) Lösemitteln und/oder (ii) Basen oder Säuren zu ihren Solvaten, Salzen und/oder Solvaten der Salze umsetzt.

Die Verbindung der Formel (II) ist literaturbekannt (R. Gompper, W. Toepfl, Chem. Ber. 1962, 95, 2861-2870). Die Verbindungen der Formeln (III) und (V) sind kommerziell erhältlich, literaturbekannt oder können in Analogie zu literaturbekannten Verfahren hergestellt werden (siehe z.B. H. Gielen, C. Alonso-Alija, M. Hendrix, U. Niewöhner, D. Schauß, Tetrahedron Lett. 2002, 43, 419-421).

Für den Verfahrensschritt (II) + (III) → (IV) eignen sich hochsiedende, inerte organische Lösemittel, die sich unter den Reaktionsbedingungen nicht verändern. Hierzu gehören bevorzugt Toluol, Acetonitril, Dimethylformamid, Dimethylsulfoxid oder Sulfolan. Ebenso ist es möglich, die Reaktion ohne Lösemittel in der Schmelze durchzuführen. Besonders bevorzugt ist eine Reaktionsführung ohne Lösemittel oder in Dimethylformamid, Acetonitril oder Toluol.

Die Reaktion erfolgt im Allgemeinen in einem Temperaturbereich von +70°C bis +200°C, bevorzugt in einem Temperaturbereich von +100°C bis +150°C. Die Umsetzung kann bei normalem, erhöhtem oder bei erniedrigtem Druck durchgeführt werden (z.B. von 0.5 bis 5 bar). Im Allgemeinen arbeitet man bei Normaldruck.

Die Verbindung der Formel (III) wird hierbei in einer Menge von 1 bis 2 Mol, bevorzugt in einer äquivalenten Menge von 1 Mol, bezogen auf 1 Mol der Verbindung der Formel (II), eingesetzt.

Für den Verfahrensschritt (VI) + (III) → (I) eignen sich übliche organische Lösemittel, die sich unter den Reaktionsbedingungen nicht verändern. Hierzu gehören bevorzugt Dimethylformamid, Dimethylsulfoxid oder Acetonitril. Ebenso ist es möglich, die Reaktion ohne Lösemittel durchzuführen. Besonders bevorzugt ist eine Reaktionsführung ohne Lösemittel oder in Acetonitril.

Die Reaktion erfolgt im Allgemeinen in einem Temperaturbereich von +50°C bis +150°C, bevorzugt in einem Temperaturbereich von +70°C bis +100°C. Die Umsetzung kann bei normalem, erhöhtem oder bei erniedrigtem Druck durchgeführt werden (z.B. von 0.5 bis 5 bar). Im Allgemeinen arbeitet man bei Normaldruck.

Die Verbindung der Formel (III) wird hierbei in einer Menge von 1 bis 10 Mol, bevorzugt in einem Überschuss von 3 bis 10 Mol, bezogen auf 1 Mol der Verbindung der Formel (VI), eingesetzt.

Für den Verfahrensschritt (IV) + (V) → (I) bzw. (II) + (V) → (VI) eignen sich übliche organische Lösemittel, die sich unter den Reaktionsbedingungen nicht verändern. Hierzu gehören bevorzugt Dimethylformamid, Dimethylsulfoxid, Acetonitril, Dioxan oder Alkohole wie Methanol, Ethanol, Propanol, Isopropanol, n-Butanol oder tert.-Butanol. Ebenso ist es möglich, Gemische der genannten Lösemittel einzusetzen. Besonders bevorzugt ist für den Verfahrensschritt (IV) + (V) → (I) Dimethylformamid oder Acetonitril und für den Verfahrensschritt (II) + (V) → (VI) Ethanol.

Die Reaktion erfolgt im Allgemeinen in einem Temperaturbereich von +50°C bis +150°C, bevorzugt in einem Temperaturbereich von +70°C bis +100°C. Die Umsetzung kann bei normalem, erhöhtem oder bei erniedrigtem Druck durchgeführt werden (z.B. von 0.5 bis 5 bar). Im Allgemeinen arbeitet man bei Normaldruck.

Als Basen für den Verfahrensschritt (IV) + (V) → (I) bzw. (II) + (V) → (VI) eignen sich bevorzugt Alkalicarbonate wie Lithium-, Natrium-, Kalium- oder Cäsiumcarbonat oder organische Amin-Basen wie beispielsweise Pyridin, Triethylamin, Ethyldiisopropylamin, *N*-Methylmorpholin oder *N-*Methylpiperidin. Besonders bevorzugt ist Kaliumcarbonat oder Triethylamin.

Die Base wird hierbei in einer Menge von 1.5 bis 4 Mol, bevorzugt in einer Menge von 1.5 bis 2 Mol, bezogen auf 1 Mol der Verbindung der Formel (IV) bzw. (II), eingesetzt. Die Verbindung der Formel (V) wird in einer Menge von 1 bis 1.5 Mol, bevorzugt in einer Menge von 1.2 Mol, bezogen auf 1 Mol der Verbindung der Formel (IV) bzw. (II), eingesetzt.

Das erfindungsgemäße Verfahren kann durch die folgenden Formelschemata beispielhaft erläutert werden: 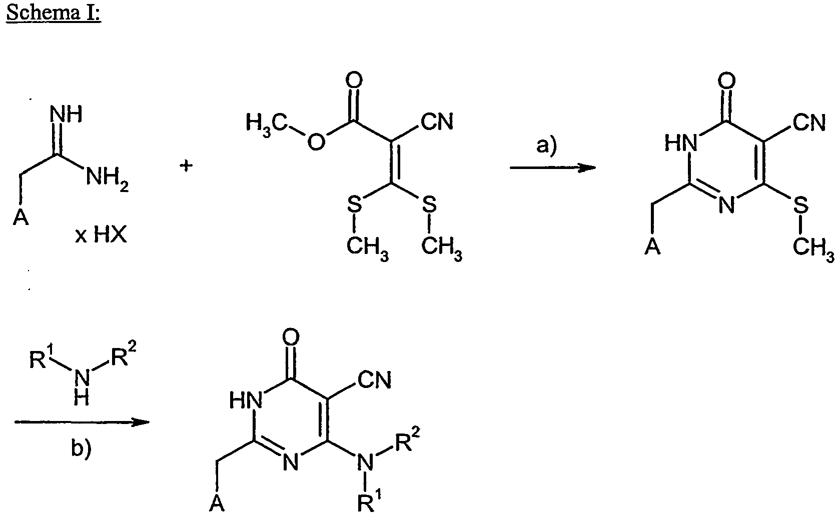 X = Cl, Br
a) Ethanol, Triethylamin, 5-16 h Rückfluss; b) Acetonitril, 85-90°C, 1-7 Tage. 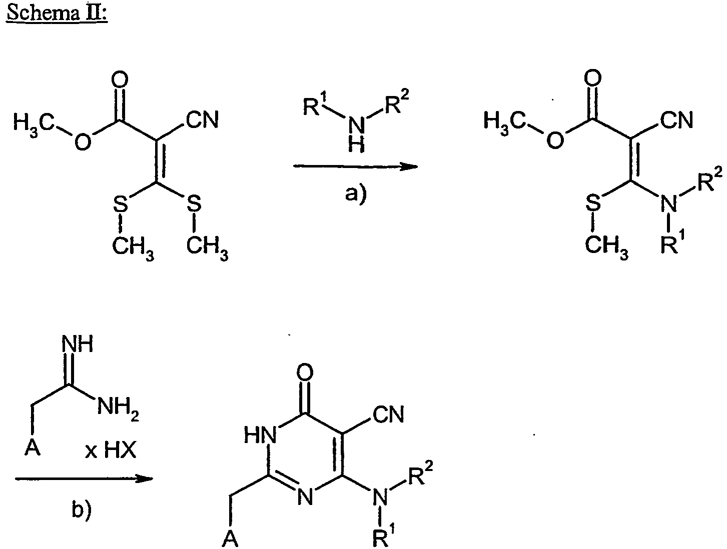 X = Cl, Br
a) 1. Toluol, Bortrifluorid-Etherat, RT, 30 min.; 2. Amin-Komponente R¹R²NH, 150°C, 16 h; oder: Schmelze der Ausgangsverbindungen bei 150°C, 1-16 h; b) DMF, Triethylamin, 100°C, 16 h oder DMF, Kaliumcarbonat, 90°C, 16 h.

Die erfindungsgemäßen Verbindungen zeigen ein nicht vorhersehbares, wertvolles pharmakologisches Wirkspektrum. Sie zeichnen sich insbesondere durch eine Inhibition von PDE9A aus.

Überraschenderweise wurde gefunden, dass die erfindungsgemäßen Verbindungen zur Herstellung von Arzneimitteln zur Verbesserung der Wahrnehmung, Konzentrationsleistung, Lernleistung oder Gedächtnisleistung geeignet sind.

Die erfindungsgemäßen Verbindungen können aufgrund ihrer pharmakologischen Eigenschaften allein oder in Kombination mit anderen Arzneimitteln zur Verbesserung von Wahrnehmung, Konzentrationsleistung, Lern- und/oder Gedächtnisleistung eingesetzt werden.

Besonders eignen sich die erfindungsgemäßen Verbindungen zur Verbesserung der Wahrnehmung, Konzentrationsleistung, Lemleistung, oder Gedächtnisleistung nach kognitiven Störungen, wie sie insbesondere bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatische Demenz, allgemeine Konzentrationsstörungen, Konzentrationsstörungen in Kindern mit Lern- und Gedächtnisproblemen, Alzheimer'sche Krankheit, Demenz mit Lewy-Körperchen, Demenz mit Degeneration der Frontallappen einschließlich des Pick's Syndroms, Parkinson'sche Krankheit, Progressive nuclear palsy, Demenz mit corticobasaler Degeneration, Amyotrope Lateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsakoff-Psychose.

Die *in vitro*-Wirkung der erfindungsgemäßen Verbindungen kann mit folgenden biologischen Assays gezeigt werden:

### PDE-Inhibition

Rekombinante PDE1C (GenBank/EMBL Accession Number: NM_005020, Loughney et al. J. Biol. Chem. 1996 271, 796-806), PDE2A (GenBank/EMBL Accession Number: NM_002599, Rosman et al. Gene 1997 191, 89-95), PDE3B (GenBank/EMBL Accession Number: NM_000922, Miki et al. Genomics 1996, 36, 476-485), PDE4B (GenBank/EMBL Accession Number: NM_002600, Obernolte et al. Gene. 1993, 129, 239-247), PDE5A (GenBank/EMBL Accession Number: NM_001083, Loughney et al. Gene 1998, 216, 139-147), PDE7B (GenBank/EMBL Accession Number: NM_018945, Hetman et al. Proc. Natl. Acad. Sci. U.S.A. 2000, 97, 472-476), PDE8A (GenBank/EMBL Accession Number: AF_056490, Fisher et al. Biochem. Biophys. Res. Commun. 1998 246, 570-577), PDE9A (Fisher et al., J. Biol. Chem, 1998, 273 (25): 15559-15564), PDE10A (GenBank/EMBL Accession Number: NM_06661, Fujishige et al. J Biol Chem. 1999, 274, 18438-45), PDE11A (GenBank/EMBL Accession Number: NM_016953, Fawcett et al. Proc. Natl. Acad. Sci. 2000, 97, 3702-3707) wurden mit Hilfe des pFASTBAC Baculovirus Expressionssystems (GibcoBRL) in Sf9-Zellen exprimiert.

Die Testsubstanzen werden zur Bestimmung ihrer *in vitro* Wirkung an PDE9A in 100% DMSO aufgelöst und seriell verdünnt. Typischerweise werden Verdünnungsreihen von 200 µM bis 1.6 µM hergestellt (resultierende Endkonzentrationen im Test: 4 µM bis 0.032 µM). Jeweils 2 µL der verdünnten Substanzlösungen werden in die Vertiefungen von Mikrotiterplatten (Isoplate; Wallac Inc., Atlanta, GA) vorgelegt. Anschließend werden 50 µL einer Verdünnung des oben beschriebenen PDE9A-Präparates hinzugefügt. Die Verdünnung des PDE9A-Präparates wird so gewählt, dass während der späteren Inkubation weniger als 70% des Substrates umgesetzt wird (typische Verdünnung: 1:10000; Verdünnungspuffer: 50 mM Tris/HCl pH 7.5, 8.3 mM MgCl₂, 1.7 mM EDTA, 0.2% BSA). Das Substrat, [8-³H] guanosine 3', 5'-cyclic phosphate (1 µCi/µL; Amersham Pharmacia Biotech., Piscataway, NJ) wird 1:2000 mit Assaypuffer (50 mM Tris/HCl pH 7.5, 8.3 mM MgCl₂, 1.7 mM EDTA) auf eine Konzentration von 0.0005 µCi/µL verdünnt.

Durch Zugabe von 50 µL (0.025 µCi) des verdünnten Substrates wird die Enzymreaktion schließlich gestartet. Die Testansätze werden für 60 min bei Raumtemperatur inkubiert und die Reaktion durch Zugabe von 25 µl eines in Assaypuffer gelösten PDE9A-Inhibitors (z.B. der Inhibitor aus Herstellbeispiel 1, 10 µM Endkonzentration) gestoppt. Direkt im Anschluss werden 25 µL einer Suspension mit 18 mg/mL Yttrium Scintillation Proximity Beads (Amersham Pharmacia Biotech., Piscataway, NJ.) hinzugefügt. Die Mikrotiterplatten werden mit einer Folie versiegelt und für 60 min bei Raumtemperatur stehengelassen. Anschließend werden die Platten für 30 s pro Vertiefung in einem Microbeta Szintillationzähler (Wallac Inc., Atlanta, GA) vermessen. IC₅₀-Werte werden anhand der graphischen Auftragung der Substanzkonzentration gegen die prozentuale Inhibition bestimmt.

Die *in vitro* Wirkung von Testsubstanzen an rekombinanter PDE3B, PDE4B, PDE7B, PDE8A, PDE10A und PDE11A wird nach dem oben für PDE9A beschriebenen Testprotokoll mit folgenden Anpassungen bestimmt: Als Substrat wird [5',8-³H] adenosine 3', 5'-cyclic phosphate (1 µCi/µL; Amersham Pharmacia Biotech., Piscataway, NJ) verwendet. Die Zugabe einer Inhibitorlösung zum Stoppen der Reaktion ist nicht notwendig. Stattdessen wird in Anschluss an die Inkubation von Substrat und PDE direkt mit der Zugabe der Yttrium Scintillation Proximity Beads wie oben beschrieben fortgefahren und dadurch die Reaktion gestoppt. Für die Bestimmung einer entsprechenden Wirkung an rekombinanter PDE1C, PDE2A und PDE5A wird das Protokoll zusätzlich wie folgt angepasst: Bei PDE1C werden zusätzlich Calmodulin 10⁻⁷ M und CaCl₂ 3 mM zum Reaktionsansatz gegeben. PDE2A wird im Test durch Zugabe von cGMP 1 µM stimuliert und mit einer BSA-Konzentration von 0.01% getestet. Für PDE1C und PDE2A wird als Substrat [5',8-³H] adenosine 3', 5'-cyclic phosphate (1 µCi/µL; Amersham Pharmacia Biotech., Piscataway, NJ), für PDE5A [8-³H] guanosine 3', 5'-cyclic phosphate (1 µCi/µL; Amersham Pharmacia Biotech., Piscataway, NJ) eingesetzt.

Repräsentative Beispiele für die selektiv PDE9A-inhibierende Wirkung der erfindungsgemäßen Verbindungen sind anhand der IC₅₀-Werte in den Tabellen 1 bis 3 aufgeführt:
Tabellen 1-3: Inhibition von PDE-Isoenzymen (human) durch Beispiele 38, 112 und 113

**Tabelle 1: Beispiel 38**

| **Isoenzym** | **IC₅₀ [nM]** |
|---|---|
| PDE1C | > 4000 |
| PDE2A | > 4000 |
| PDE3B | > 4000 |
| PDE4B | > 4000 |
| PDE7B | 2200 |
| PDE8A | 4000 |
| PDE9A | 5 |
| PDE10A | > 4000 |
| PDE11 | > 4000 |

**Tabelle 2: Beispiel 112**

| **Isoenzym** | **IC₅₀ [nM]** |
|---|---|
| PDE1C | > 4000 |
| PDE2A | > 4000 |
| PDE3B | > 4000 |
| PDE4B | > 4000 |
| PDE7B | > 4000 |
| PDE8A | > 4000 |
| PDE9A | 5 |
| PDE10A | > 4000 |
| PDE11 | > 4000 |

**Tabelle 3: Beispiel 113**

| **Isoenzym** | **IC₅₀ [nM]** |
|---|---|
| PDE1C | > 4000 |
| PDE2A | > 4000 |
| PDE3B | > 4000 |
| PDE4B | > 4000 |
| PDE7B | > 4000 |
| PDE8A | > 4000 |
| PDE9A | 15 |
| PDE10A | > 4000 |
| PDE11 | 1500 |

### Langzeitpotenzierung

Langzeitpotenzierung wird als ein zelluläres Korrelat für Lern- und Gedächtnisvorgänge angesehen. Zur Bestimmung, ob PDE9-Inhibition einen Einfluss auf Langzeitpotenzierung hat, kann folgende Methode angewandt werden:
Rattenhippokampi werden in einen Winkel von etwa 70 Grad im Verhältnis zur Schnittklinge plaziert (Chopper). In Abständen von 400 µm wird der Hippokampus zerschnitten. Die Schnitte werden mit Hilfe eines sehr weichen, stark benetzten Pinsels (Marderhaar) von der Klinge genommen und in ein Glasgefäß mit carbogenisierter gekühlter Nährlösung (124 mM NaCl, 4.9 mM KCl, 1.3 mM MgSO₄ x 7 H₂O, 2.5 mM CaCl₂ wasserfrei, 1.2 mM KH₂PO₄, 25.6 mM NaHCO₃, 10 mM Glucose, pH 7.4) überführt. Während der Messung befinden sich die Schnitte in einer temperierten Kammer unter einem Flüssigkeitsspiegel von 1-3 mm Höhe, Die Durchflussrate beträgt 2.5 ml/min. Die Vorbegasung erfolgt unter geringen Überdruck (etwa 1 atm) sowie über eine Mikrokanüle in der Vorkammer. Die Schnittkammer ist mit der Vorkammer so verbunden, dass eine Minizirkulation aufrechterhalten werden kann. Als Antrieb der Minizirkulation wird das durch die Mikrokanüle ausströmende Carbogen eingesetzt. Die frisch präparierten Hippokampusschnitte werden mindestens 1 Stunde bei 33°C in der Schnittkammer adaptiert.

Die Reizstärke wird so gewählt, dass die fokalen exzitatiorischen postsynaptischen Potentiale (fEPSP) 30 % des maximalen exzitatorischen postsynaptischen Potentials (EPSP) betragen. Mit Hilfe einer monopolaren Stimulationselektrode, die aus lackiertem Edelstahl besteht und eines stromkonstanten, biphasischen Reizgenerators (AM-Systems 2100), werden lokal die Schaffer-Kollateralen erregt (Spannung: 1-5 V, Impulsbreite einer Polarität 0.1 ms, Gesamtimpuls 0.2 ms). Mit Hilfe von Glaselektroden (Borosilikatglas mit Filament, 1-5 MOhm, Durchmesser: 1.5 mm, Spitzendurchmesser: 3-20 µm), die mit normaler Nährlösung gefüllt sind, werden aus dem Stratum radiatum die exzitatorischen postsynaptischen Potentiale (fEPSP) registriert. Die Messung der Feldpotentiale geschieht gegenüber einer chlorierten Referenzelektrode aus Silber, die sich am Rande der Schnittkammer befindet, mit Hilfe eines Gleichspannungsverstärkers. Das Filtern der Feldpotentiale erfolgt über einen Low-Pass Filter (5 kHz). Für die statistische Analyse der Experimente wird der Anstieg (slope) der fEPSPs (fEPSP-Anstieg) ermittelt. Die Aufnahme, Analyse und Steuerung des Experimentes erfolgt mit Hilfe eines Softwareprogrammes (PWIN), welches in der Abteilung Neurophysiologie entwickelt worden ist. Die Mittelwertbildung der fEPSP-Anstiegswerte zu den jeweiligen Zeitpunkten und die Konstruktion der Diagramme erfolgt mit Hilfe der Software EXCEL, wobei ein entsprechendes Makro die Aufnahme der Daten automatisiert.

Superfusion der Hippokampusschnitte mit einer 10 µM Lösung der erfindungsgemäßen Verbindungen führt zu einer signifikanten Steigerung der LTP.

Die in vivo-Wirkung der erfindungsgemäßen Verbindungen kann zum Beispiel wie folgt gezeigt werden:

### Sozialer Wiedererkennungstest

Der Soziale Wiedererkennungstest ist ein Lern- und Gedächtnistest. Er misst die Fähigkeit von Ratten, zwischen bekannten und unbekannten Artgenossen zu unterscheiden. Deshalb eignet sich dieser Test zur Prüfung der lern- oder gedächtnisverbessemden Wirkung der erfindungsgemäßen Verbindungen.

Adulte Ratten, die in Gruppen gehalten werden, werden 30 min vor Testbeginn einzeln in Testkäfige gesetzt. Vier min vor Testbeginn wird das Testtier in eine Beobachtungsbox gebracht. Nach dieser Adaptationszeit wird ein juveniles Tier zu dem Testtier gesetzt und 2 min lang die absolute Zeit gemessen, die das adulte Tier das Junge inspiziert (Trial 1). Gemessen werden alle deutlich auf das Jungtier gerichteten Verhaltensweisen, d.h. ano-genitale Inspektion, Verfolgen sowie Fellpflege, bei denen das Alttier einen Abstand von höchstens 1 cm zu dem Jungtier hatte. Danach wird das Juvenile herausgenommen, das Adulte mit einer erfindungsgemäßen Verbindung oder Vehikel behandelt und anschließend in seinen Heimkäfig zurückgesetzt Nach einer Retentionszeit von 24 Stunden wird der Test wiederholt (Trial 2). Eine verringerte Soziale Interaktionszeit im Vergleich zu Trial 1 zeigt an, dass die adulte Ratte sich an das Jungtier erinnert.

Die adulten Tiere werden entweder in einem festgelegten Zeitabstand (z.B. 1 Stunde) vor Trial 1 oder direkt im Anschluss an Trial 1 entweder mit Vehikel (10 % Ethanol, 20 % Solutol, 70 % physiologische Kochsalzlösung) oder 0.1 mg/kg, 0.3 mg/kg, 1.0 mg/kg bzw. 3.0 mg/kg erfindungsgemäßer Verbindung, gelöst in 10 % Ethanol, 20 % Solutol, 70 % physiologische Kochsalzlösung intraperitoneal injiziert. Vehikel behandelte Ratten zeigen keine Reduktion der sozialen Interaktionszeit in Trial 2 verglichen mit Trial 1. Sie haben folglich vergessen, dass sie schon einmal Kontakt mit dem Jungtier hatten. Überraschenderweise ist die soziale Interaktionszeit im zweiten Durchgang nach Behandlung mit den erfindungsgemäßen Verbindungen signifikant gegenüber den Vehikel behandelten reduziert. Dies bedeutet, dass die substanzbehandelten Ratten sich an das juvenile Tier erinnert haben und somit die erfindungsgemäßen Verbindungen eine verbessernde Wirkung auf Lernen und Gedächtnis aufweist.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0.5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise durch Verstrecken der Wirkstoffe mit Lösungsmitteln und/oder Trägerstoffen, gegebenenfalls unter Verwendung von Emulgiermitteln und/oder Dispergiermitteln hergestellt, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös. Sie kann aber auch durch Inhalation über Mund oder Nase, beispielsweise mit Hilfe eines Sprays, oder topisch über die Haut erfolgen.

Im Allgemeinen hat es sich als vorteilhaft erwiesen, Mengen von etwa 0.001 bis 10, bei oraler Anwendung vorzugsweise etwa 0.005 bis 3 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Soweit nicht anders angegeben, beziehen sich alle Mengenangaben auf Gewichtsprozente. Lösungsmittelverhältnisse, Verdünnungsverhältnisse und Konzentrationsangaben von flüssig/-flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

### Abkürzungen:

- DCI: direkte chemische Ionisation (bei MS)
- DMSO: Dimethylsulfoxid
- d. Th.: der Theorie (bei Ausbeute)
- ESI: Elektrospray-Ionisation (bei MS)
- Fp.: Schmelzpunkt
- h: Stunde(n)
- HPLC: Hochdruck-, Hochleistungsflüssigchromatographie
- LC-MS: Flüssigchromatographie-gekoppelte Massenspektroskopie
- min: Minute(n)
- MS: Massenspektroskopie
- NMR: Kernresonanzspektroskopie
- R_{f}: Retentionsindex (bei DC)
- RT: Raumtemperatur
- Rₜ: Retentionszeit (bei HPLC)

### LC-MS- und HPLC-Methoden:

### Methode 1:

Instrument: Micromass Platform LCZ mit HPLC Agilent Serie 1100; Säule: Grom-Sil 120 ODS-4 HE, 50 mm x 2.0 mm, 3 µm; Eluent A: 11 Wasser + 1 ml 50 %-ige Ameisensäure, Eluent B: 1 l Acetonitril + 1 ml 50 %-ige Ameisensäure; Gradient: 0.0 min 100 % A → 0.2 min 100 % A → 2.9 min 30 % A → 3.1 min 10 % A → 4.5 min 10 % A; Ofen: 55°C; Fluss: 0.8 ml/min; UV-Detektion: 208-400 nm.

### Methode 2:

Instrument: Micromass Quattro LCZ mit HPLC Agilent Serie 1100; Säule: Grom-Sil 120 ODS-4 HE, 50 mm x 2.0 mm, 3 µm; Eluent A: 1 l Wasser + 1 ml 50 %-ige Ameisensäure, Eluent B: 1 l Acetonitril + 1 ml 50 %-ige Ameisensäure; Gradient: 0.0 min 100 % A → 0.2 min 100 % A → 2.9 min 30 % A → 3.1 min 10 % A → 4.5 min 10% A; Ofen: 55°C; Fluss: 0.8 ml/min; UV-Detektion: 208-400 nm.

### Methode 3:

Instrument: Micromass Platform LCZ mit HPLC Agilent Serie 1100; Säule: Phenomenex Synergi 2µ Hydro-RP Mercury 20 mm x 4 mm; Eluent A: 1 1 Wasser + 0.5 ml 50 %-ige Ameisensäure, Eluent B: 1 l Acetonitril + 0.5 ml 50 %-ige Ameisensäure; Gradient: 0.0 min 90 % A, Fluss 1 ml/min → 2.5 min 30 % A, Fluss 2 ml/min → 3.0 min 5 % A, Fluss 2 ml/min → 4.5 min 5 % A, Fluss 2 ml/min; Ofen: 50°C; UV-Detektion: 210 nm.

### Methode 4:

Instrument: Micromass Quattro LCZ mit HPLC Agilent Serie 1100; Säule: Phenomenex Synergi 2µ Hydro-RP Mercury 20 mm x 4 mm; Eluent A: 1 l Wasser + 0.5 ml 50 %-ige Ameisensäure, Eluent B: 1 l Acetonitril + 0.5 ml 50 %-ige Ameisensäure; Gradient: 0.0 min 90 % A, Fluss 1 ml/min → 2.5 min 30 % A, Fluss 2 ml/min → 3.0 min 5 % A, Fluss 2 ml/min → 4.5 min 5 % A, Fluss 2 ml/min; Ofen: 50°C; UV-Detektion: 208- 400 nm.

### Methode 5:

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 l Wasser + 0.5 ml 50 %-ige Ameisensäure, Eluent B: 1 l Acetonitril + 0.5 ml 50 %-ige Ameisensäure; Gradient: 0.0 min 90 % A, Fluss 1 ml/min → 2.5 min 30 % A, Fluss 2 ml/min → 3.0 min 5 % A, Fluss 2 ml/min → 4.5 min 5 % A, Fluss 2 ml/min; Ofen: 50°C; UV-Detektion: 210 nm.

### Methode 6:

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50 %-ige Ameisensäure, Eluent B: 1 l Acetonitril + 0.5 ml 50 %-ige Ameisensäure; Gradient: 0.0 min 90 % A, Fluss 1 ml/min → 2.5 min 30 % A, Fluss 2 ml/min → 3.0 min 5 % A, Fluss 2 ml/min → 4.5 min 5 % A, Fluss 2 ml/min; Ofen: 50°C; UV-Detektion: 210 nm.

### Methode 7:

Gerätetyp MS: Micromass ZQ; Gerätetyp HPLC: HP 1100 Series; UV DAD; Säule: Grom-Sil 120 ODS-4 HE 50 mm x 2 mm, 3.0 µm; Eluent A: Wasser + 500 µl 50 %-ige Ameisensäure /1, Eluent B: Acetonitril + 500 µl 50 %-ige Ameisensäure / 1; Gradient: 0.0 min 0 % B → 2.9 min 70 % B → 3.1 min 90 % B → 4.5 min 90 % B; Ofen: 50°C; Fluss: 0.8 ml/min; UV-Detektion: 210 nm.

### Methode 8:

Gerätetyp MS: Micromass ZQ; Gerätetyp HPLC: Waters Alliance 2790; Säule: Grom-Sil 120 ODS-4 HE 50 mm x 2 mm, 3.0 µm; Eluent A: Wasser + 500 µl 50 %-ige Ameisensäure /l, Eluent B: Acetonitril + 500 µl 50 %-ige Ameisensäure /l; Gradient: 0.0 min 5 % B → 2.0 min 40 % B → 4.5 min 90 % B → 5.5 min 90 % B; Fluss: 0.0 min 0.75 ml/min → 4.5 min 0.75 ml/min → 5.5 min 1.25 ml/min; Ofen: 45°C; UV-Detektion: 210 nm.

### Methode 9:

Gerätetyp MS: Micromass ZQ; Gerätetyp HPLC: Waters Alliance 2790; Säule: Grom-Sil 120 ODS-4 HE 50 mm x 2 mm, 3.0 µm; Eluent A: Wasser + 500 µl 50 %-ige Ameisensäure /l, Eluent B: Acetonitril + 500 µl 50 %-ige Ameisensäure / l; Gradient: 0.0 min 0 % B → 0.2 min 0 % B → 2.9 min 70 % B → 3.1 min 90 % B → 4.5 min 90 % B; Ofen: 45°C; Fluss: 0.8 ml/min; UV-Detektion: 210 nm.

### Methode 10:

Gerätetyp MS: Micromass ZQ; Gerätetyp HPLC: Waters Alliance 2795; Säule: Merck Chromolith SpeedROD RP-18e 50 mm x 4.6 mm; Eluent A: Wasser + 500 µl 50 %-ige Ameisensäure / l, Eluent B: Acetonitril + 500 µl 50 %-ige Ameisensäure / l; Gradient: 0.0 min 10 % B → 3.0 min 95 % B → 4.0 min 95 % B; Ofen: 35°C; Fluss: 0.0 min 1.0 ml/min → 3.0 min 3.0 ml/min → 4.0 min 3.0 ml/min; UV-Detektion: 210 nm.

### Methode 11:

Gerätetyp MS: Micromass ZQ; Gerätetyp HPLC: Waters Alliance 2790; Säule: Uptisphere C 18 50 mm x 2.0 mm, 3.0 µm; Eluent B: Acetonitril + 0.05 % Ameisensäure, Eluent A: Wasser + 0.05 % Ameisensäure; Gradient: 0.0 min 5 % B → 2.0 min 40 % B → 4.5 min 90 % B → 5.5 min 90 % B; Ofen: 45°C; Fluss: 0.0 min 0.75 ml/min → 4.5 min 0.75 ml/min → 5.5 min 1.25 ml/min; UV-Detektion: 210 nm.

### Methode 12:

Instrument: HP 1100 mit DAD-Detektion; Säule: Kromasil RP-18, 60 mm x 2 mm, 3.5 µm; Eluent A: 5 ml HClO₄ / l Wasser, Eluent B: Acetonitril; Gradient: 0 min 2% B → 0.5 min 2% B → 4.5 min 90 % B → 6.5 min 90 % B; Fluss: 0.75 ml/min; Temperatur: 30°C; UV-Detektion: 210 nm.

### Methode 13:

Instrument: HP 1100 mit DAD-Detektion; Säule: Kromasil RP-18, 125 mm x 4 mm, 5 µm; Eluent A: 5 ml HClO₄ / 1 Wasser, Eluent B: Acetonitril; Gradient: 0 min 2 % B → 0.5 min 2 % B → 4.5 min 90 % B → 6.5 min 90% B; Fluss: 0.75 ml/min; Temperatur: 30°C; UV-Detektion: 210 nm.

### Ausgangsverbindungen:

Die für die folgenden Umsetzungen benötigten Amidine werden aus den entsprechenden Nitrilen bzw. Estern gemäß Gielen H., Alonso-Alija C., Hendrix M., Niewöhner U., Schauß D., Tetrahedron Lett. 43, 419-421 (2002) hergestellt.

### Beispiel 1A

### 2-(3,4-Dichlorphenyl)ethanamidin-Hydrochlorid

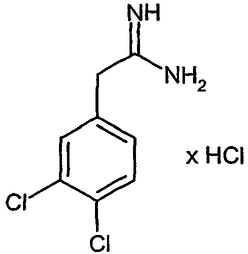

Unter einer Argonatmosphäre werden 2.88 g (54 mmol) Ammoniumchlorid in 50 ml Toluol suspendiert und auf 0°C abgekühlt. Nach Zutropfen von 27 ml einer 2 M Trimethylaluminium-Lösung in Toluol wird das Gemisch auf Raumtemperatur erwärmt und 1.5 h nachgerührt. Es werden 5 g (27 mmol) 3,4-Dichlorphenylacetonitril zugegeben und der Ansatz über Nacht bei 80°C gerührt. Nach Abkühlen auf 0°C werden 50 ml Methanol zugetropft. Das Produkt wird vom ausgefallenen Feststoff durch Absaugen getrennt und der Filterkuchen mehrfach mit Methanol ge-waschen. Die vereinigten Filtrate werden bis zur Trockene eingeengt, der Rückstand dann in Dichlormethan/Methanol 10:1 aufgeschlämmt und wiederum abgesaugt. Das Filtrat ergibt nach Einengen 6.2 g (77% d. Th.) der Titelverbindung.
MS (ESIpos): m/z = 203 [M+H]⁺.

### Beispiel 2A

### 6-Methoxypyridin-3-ylboronsäure

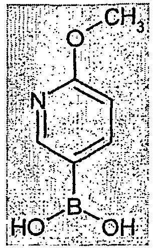

1 g (5.32 mmol) 5-Brom-2-methoxypyridin wird in 10 ml absolutem Tetrahydrofuran gelöst und auf -78°C abgekühlt. Nach Zugabe von 0.4 g (6.38 mmol) einer 1.6 M n-Butyllithium-Lösung in Hexan entsteht eine gelbe Lösung, die 30 min bei der gegebenen Temperatur gerührt wird. Nach Zugabe von 3 g (15.9 mmol) Triisopropylborat wird eine weitere Stunde gerührt, wobei sich die Lösung auf -20°C erwärmt. Man versetzt mit Wasser und rührt über Nacht nach. Die Rohlösung wird mit 1 N Salzsäure auf pH 5 angesäuert und zweimal mit Ethylacetat extrahiert. Die organische Phase wird über Natriumsulfat getrocknet, filtriert und eingeengt, wobei ein hellbrauner Feststoff erhalten wird, der mit Diethylether aufgeschlämmt und filtriert wird. Es werden 0.38 g (47 % d. Th.) des Produktes isoliert.
MS (ESIpos): m/z = 154 [M+H]⁺
¹H-NMR (DMSO-d₆, 300 MHz): δ = 3.83 (s, 3H), 6.76 (d, 1H), 8.0 (dd, 1H), 8.52 (s, 1H).

### Beispiel 3A

### Methyl [2-(6-methoxypyridin-3-yl)phenyl]acetat

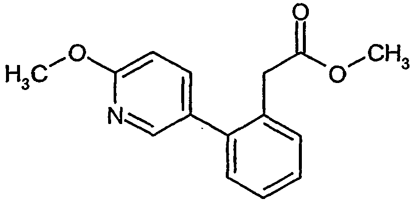

1.35 g (5.89 mmol) Methyl (2-bromphenyl)acetat werden mit 1 g (6.55 mmol) 6-Methoxypyridin, 3-ylboronsäure und 1.98 g (13.09 mmol) Cäsiumfluorid unter Argon in 20 ml 1,2-Dimethoxyethan vorgelegt. Nach Zugabe von 0.22 g (0.19 mmol) Tetrakis(triphenylphosphin)palladium(0) wird die Reaktionsmischung 4 h bei 100°C gerührt. Nach Abkühlen auf Raumtemperatur wird eine Mischung aus Ethylacetat und Wasser zugesetzt und mit Ethylacetat extrahiert. Nach Trocknen der organischen Phase über Magnesiumsulfat und Entfernen des Lösungsmittels im Vakuum wird der Rückstand säulenchromatographisch an Kieselgel gereinigt (Laufmittel: Cyclohexan/Ethylacetat 9:1). Es werden 1.1 g (68% d. Th.) des Produktes erhalten.
LC-MS (Methode 5): Rₜ = 2.1 min., MS (ESIpos): m/z = 258 [M+H]⁺
¹H-NMR (DMSO-d₆, 300 MHz): δ = 3.51 (s, 3H), 3.63 (s, 2H), 3.9 (s, 3H), 6.89 (d, 1H), 7.26 (m, 1H), 7.38 (m, 3H), 7.63 (dd, 1H), 8.08 (m, 1H).

### Beispiel 4A

### 2-[2-(6-Methoxypyridin-3-yl)phenyl]ethanimidamid-Hydrochlorid

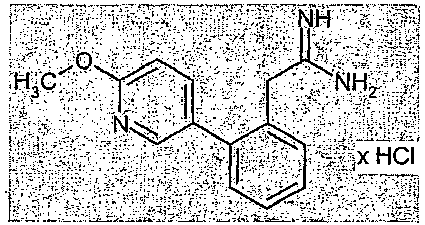

Unter einer Argonatmosphäre werden 1.14 g (21.37 mmol) Ammoniumchlorid in 20 ml Toluol suspendiert und auf 0°C abgekühlt. Nach Zutropfen von 10.7 ml einer 2 M Trimethylaluminium-Lösung in Toluol wird das Gemisch auf Raumtemperatur erwärmt und 1.5 h nachgerührt. Es werden 1.1 g (4.27 mmol) Methyl [2-(6-methoxypyridin-3-yl)phenyl]acetat zugegeben und der Ansatz zwei Tage bei 80°C gerührt Nach Abkühlen auf 5°C werden 50 ml Methanol zugetropft. Das Produkt wird vom ausgefallenen Feststoff durch Absaugen getrennt und der Filterkuchen mehrfach mit Methanol gewaschen. Die vereinigten Filtrate werden bis zur Trockene eingeengt, der Rückstand dann in Dichlormethan/Methanol 10:1 aufgeschlämmt und wiederum abgesaugt. Das Filtrat ergibt nach Einengen 0.5 g (46% d. Th.) der Titelverbindung.
LC-MS (Methode 5): Rₜ = 0.94 min., MS (ESIpos): m/z = 242 [M+H]⁺
¹H-NMR (DMSO-d₆, 300 MHz): δ = 3.79 (s, 2H), 3.9 (s, 3H), 6.89 (d, 1H), 7.31 (m, 2H), 7.46 (m, 2H), 7.68 (dd, 1H), 8.12 (m, 1H), 8.72 (s, 1H), 8.8 (s, 2H).

### Beispiel 5A

### 2-[2-(6-Methoxypyridin-3-yl)benzyl]-4-(methylsulfanyl)-6-oxo-1,6-dihydropyrimidin-5-carbonitril

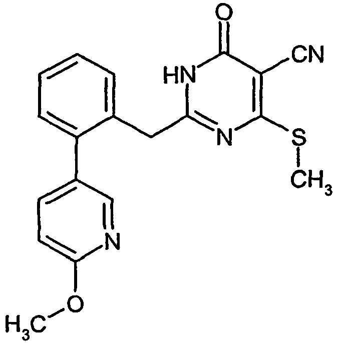

0.55 g (1.96 mmol) 2-[2-(6-Methoxypyridin-3-yl)phenyl]ethanimidamid-Hydrochlorid werden mit 0.4 g (1.96 mmol) Methyl 2-cyano-3,3-dimethylthioprop-2-enoat und 0.79 g (7.81 mmol) Triethylamin in 20 ml Dioxan gelöst und über Nacht bei 90°C gerührt. Anschließend wird das Lösungsmittel bis auf ca. 2 ml im Vakuum entfernt und die verbleibende Lösung mit Acetonitril versetzt, wobei das Produkt ausfällt. Nach Filtration wird mit Acetonitril und Methanol gewaschen und das Produkt im Hochvakuum getrocknet. Man erhält 276 mg (38% d. Th.) der Titelverbindung.
LC-MS (Methode 5): Rₜ = 2.08 min., MS (ESIpos): m/z = 365 [M+H]⁺
¹H-NMR (DMSO-d₆, 300 MHz): δ = 2.31 (s, 3H), 3.9 (s, 3H), 3.98 (s, 2H), 6.89 (d, 1H), 7.28 (m, 1H), 7.39 (m, 3H), 7.63 (dd, 1H), 8.08 (m, 1H).

### Beispiel 6A

### 2-Benzyl-4-(methylsulfanyl)-6-oxo-1,6-dihydro-5-pyrimidincarbonitril

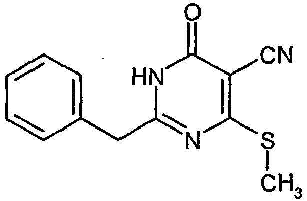

100 mg (0.59 mmol) 2-Phenylethanamidin-Hydrochlorid werden mit 119 mg (0.59 mmol) Methyl 2-cyano-3,3-dimethylthioprop-2-enoat und 237 mg (2.34 mmol) Triethylamin in 2 ml Ethanol gelöst und 5 h bei 70°C gerührt. Anschließend wird das Lösungsmittel im Vakuum entfernt, der Rückstand in 50 ml Dichlormethan aufgenommen und mit 2 M Salzsäure gewaschen. Nach Trocknen der organischen Phase über Magnesiumsulfat wird das Lösungsmittel im Vakuum entfernt und der Rückstand an Kieselgel flash-chromatographiert (Laufmittel: Dichlormethan/ Methanol 200:1, 100:1). Man erhält 75 mg (50% d. Th.) der Titelverbindung.
HPLC (Methode 12): Rₜ = 42 min.
MS (ESIpos): m/z = 258 [M+H]⁺.

### Beispiel 7A

### 2-(3-Methylbenzyl)-4-(methylsulfanyl)-6-oxo-1,6-dihydro-5-pyrimidincarbonitril

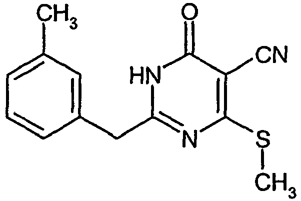

1.45 g (9.83 mmol) 2-(3-Methylphenyl)ethanamidin-Hydrochlorid werden mit 2 g (9.83 mmol) Methyl 2-cyano-3,3-dimethylthioprop-2-enoat und 2 g (19.67 mmol) Triethylamin in 40 ml Ethanol gelöst und 5 h bei 70°C gerührt. Anschließend wird das Lösungsmittel im Vakuum entfernt und der Rückstand mittels präparativer HPLC gereinigt. Es werden 0.4 g (15 % d. Th.) des Produkts erhalten.
LC-MS (Methode 2): Rₜ = 2.83 min., MS (ESIpos): m/z = 272 [M+H]⁺
¹H-NMR (DMSO-d₆, 300 MHz): δ = 2.25 (s, 3H), 2.50 (s, 3H), 3.91 (s, 2H), 7.19 (m, 4H).

### Beispiel 8A

### 2-(2-Methylbenzyl)-4-(methylsulfanyl)-6-oxo-1,6-dihydro-5-pyrimidincarbonitril

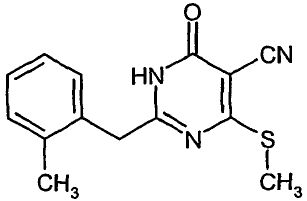

3 g (16.45 mmol) 2-(2-Methylphenyl)ethanamidin-Hydrochlorid werden mit 3.3 g (16.45 mmol) Methyl 2-cyano-3,3-dimethylthioprop-2-enoat und 6.6 g (64.9 mmol) Triethylamin in 60 ml Dioxan gelöst und über Nacht bei 90°C gerührt. Nach dem Abfiltrieren der Triethylammoniumsalze wird das Filtrat eingeengt und der Rückstand mit Dichlormethan verrieben. Es werden 3.6 g (81 % d. Th.) des Produkts erhalten.

LC-MS (Methode 10): Rₜ = 2.13 min., MS (ESIpos): m/z = 272 [M+H]⁺.

### Beispiel 9A

### 2-(2-Fluorbenzyl)-4-(methylsulfanyl)-6-oxo-1,6-dihydro-5-pyrimidincarbonitril

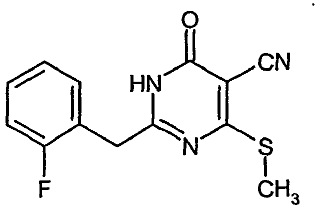

3.5 g (18.5 mmol) 2-(2-Fluorphenyl)ethanamidin-Hydrochlorid werden mit 3.8 g (18.5 mmol) Methyl 2-cyano-3,3-dimethylthioprop-2-enoat und 7.5 g (74.2 mmol) Triethylamin in 50 ml Dioxan gelöst und über Nacht bei 90°C gerührt. Nach dem Abfiltrieren der Triethylammoniumsalze wird das Filtrat eingeengt und der Rückstand in Ethylacetat aufgenommen. Das Produkt wird durch Zugabe von 1 N Salzsäure und Wasser ausgefällt. Es werden 3.8 g (75 % d. Th.) der Titelverbindung erhalten.
LC-MS (Methode 10): Rₜ = 2.03 min., MS (ESIpos): m/z = 276 [M+H]⁺
¹H-NMR (DMSO-d₆, 300 MHz): δ = 2.31 (s, 3H), 4.06 (s, 2H), 7.19 (m, 2H), 7.41 (m, 2H).

### Beispiel 10A

### 2-(2-Ethoxybenzyl)-4-(methylsulfanyl)-6-oxo-1,6-dihydro-5-pyrimidincarbonitril

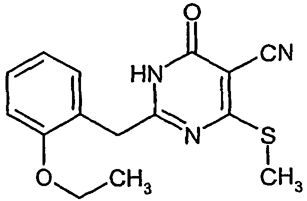

4.2 g (19.7 mmol) 2-(2-Ethoxyphenyl)ethanamidin-Hydrochlorid werden mit 4.0 g (19.7 mmol) Methyl 2-cyano-3,3-dimethylthioprop-2-enoat und 7.9 g (78.7 mmol) Triethylamin in 80 ml Dioxan gelöst und über Nacht bei 90°C gerührt. Nach dem Abfiltrieren der Triethylammonium-salze wird das Filtrat eingeengt und der Rückstand in Ethylacetat aufgenommen. Das Produkt wird durch Zugabe von 1 N Salzsäure und Wasser ausgefällt. Es werden 5.3 g (90 % d. Th.) der Titelverbindung erhalten.
LC-MS (Methode 3): Rₜ = 2.32 min., MS (ESIpos): m/z = 302 [M+H]⁺
¹H-NMR (DMSO-d₆, 300 MHz): δ = 1.19 (t, 3H), 2.30 (s, 3H), 3.99 (q, 2H + s, 2H), 6.93 (m, 2H), 7.27 (m, 2H).

### Beispiel 11A

### 2-(3-Chlorbenzyl)-4-(methylsulfanyl)-6-oxo-1,6-dihydropyrimidin-5-carbonitril

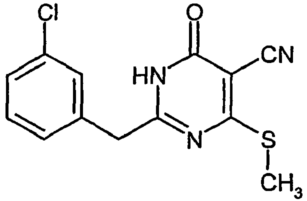

Analog zur Darstellung von Beispiel 2A werden 0.5 g (2.00 mmol) 2-(3-Chlorphenyl)ethanamidin-Hydrobromid mit 0.41 g (2.00 mmol) Methyl 2-cyano-3,3-dimethylthioprop-2-enoat und 0.81 g (8.01 mmol) Triethylamin zu 0.5 g (86 % d. Th.) der Titelverbindung umgesetzt.
HPLC (Methode 12): Rₜ = 4.4 min.
MS (DCI, NH₃): m/z = 292 [M+H]⁺, 309 [M+NH₄]⁺.

### Beispiel 12A

### 2-(4-Chlorbenzyl)-4-(methylsulfanyl)-6-oxo-1,6-dihydropyrimidin-5-carbonitril

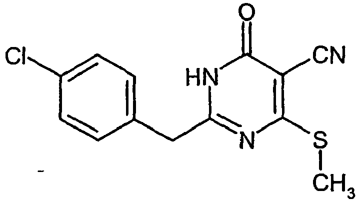

Analog zur Darstellung von **Beispiel 2A** werden 10 g (48.8 mmol) 2-(4-Chlorphenyl)ethanamidin-Hydrochlorid mit 9.91 g (48.8 mmol) Methyl 2-cyano-3,3-dimethylthioprop-2-enoat und 19.7 g (195 mmol) Triethylamin zu 7.00 g (49 % d. Th.) der Titelverbindung umgesetzt.
HPLC (Methode 12): Rₜ = 4.35 min.
MS (ESIpos): m/z = 292 [M+H]⁺.

### Beispiel 13A

### 2-(3,4-Dichlorbenzyl)-4-(methylsulfanyl)-6-oxo-1,6-dihydropyrimidin-5-carbonitril

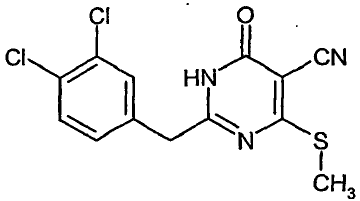

Analog zur Darstellung von Beispiel 2A werden 1.00 g (4.17 mmol) 2-(3,4-Dichlorphenyl)ethan-amidin-Hydrochlorid mit 0.85 g (4.17 mmol) Methyl 2-cyano-3,3-dimethylthioprop-2-enoat und 1.69 g (16.7 mmol) Triethylamin zu 0.6 g (44 % d. Th.) der Titelverbindung umgesetzt.
HPLC (Methode 12): Rₜ = 4.7 min.
MS (DCI, NH₃): m/z = 343 [M+NH₄]⁺.

### Beispiel 14A

### 2-(3-Fluorbenzyl)-4-(methylsulfanyl)-6-oxo-1,6-dihydropyrimidin-5-carbonitril

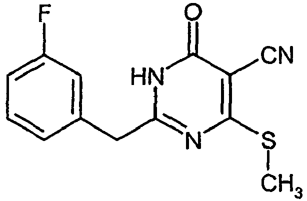

Analog zur Darstellung von Beispiel 2A werden 100 mg (0.43 mmol) 2-(3-Fluorphenyl)ethan-amidin-Hydrochlorid mit 87 mg (0.43 mmol) Methyl 2-cyano-3,3-dimethylthioprop-2-enoat und 174 mg (1.72 mmol) Triethylamin zu 28 mg (24% d. Th.) der Titelverbindung umgesetzt.
HPLC (Methode 12): Rₜ = 4.2 min.
MS (ESIpos): m/z = 276 [M+H]⁺.

### Beispiel 15A

### 4-(Methylsulfanyl)-6-oxo-2-[3-(trifluormethyl)benzyl]-1,6-dihydropyrimidin-5-carbonitril

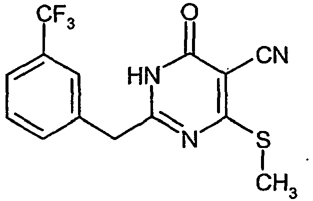

Analog zur Darstellung von Beispiel 2A werden 0.5 g (2.10 mmol) 2-[3-(Trifluormethyl)phenyl]-ethanamidin-Hydrochlorid mit 0.43 g (2.10 mmol) Methyl 2-cyano-3,3-dimethylthioprop-2-enoat und 0.85 g (8.38 mmol) Triethylamin zu 0.4 g (59% d. Th.) der Titelverbindung umgesetzt.
HPLC (Methode 12): Rₜ = 4.4 min.
MS (ESIpos): m/z = 326 [M+H]⁺.

### Beispiel 16A

### 4-(Methylsulfanyl)-6-oxo-2-(3-thienylmethyl)-1,6-dihydropyrimidin-5-carbonitril

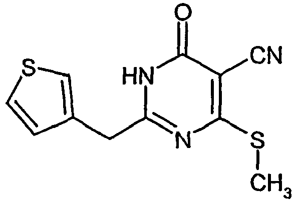

Analog zur Darstellung von Beispiel 2A werden 3.10 g (17.6 mmol) 2-(3-Thienyl)ethanamidin-Hydrochlorid mit 3.57 g (17.6 mmol) Methyl 2-cyano-3,3-dimethylthioprop-2-enoat und 7.10 g (70.2 mmol) Triethylamin zu 2.19 g (47% d. Th.) der Titelverbindung umgesetzt.
HPLC (Methode 12): Rₜ = 4.1 min.
MS (ESIpos): m/z = 263.9 [M+H]⁺.

### Beispiel 17A

### Methyl (2E/Z)-2-cyano-3-(cyclohexylamino)-3-methylthio-prop-2-enoat

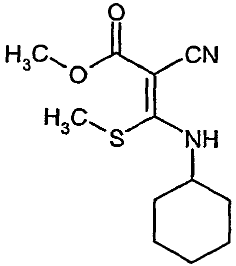

0.6 g (2.9 mmol) Methyl 3,3-bis(methylthio)-2-cyanoacrylat werden mit 0.29 g (2.9 mmol) Cyclohexylamin in 20 ml Acetonitril 1 h bei Raumtemperatur gerührt. Die flüchtigen Bestandteile werden im Vakuum entfernt. Es werden 0.74 g (98 % d. Th.) des Produkts als gelbes Öl erhalten.
HPLC (Methode 12): Rₜ = 4.85 min.
MS (DCI, NH₃): m/z = 254.9 [M+H]⁺, 272 [M+NH₄]⁺.

### Beispiel 18A

### Methyl (2E/Z)-2-cyano-3-(cyclopentylamino)-3-methylthio-prop-2-enoat

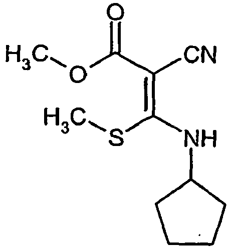

0.3 g (1.47 mmol) Methyl 3,3-bis(methylthio)-2-cyanoacrylat werden mit 0.13 g (1.47 mmol) Cyclopentylamin in 3 ml Acetonitril 30 min auf 70°C erhitzt. Anschließend werden die flüchtigen Bestandteile im Vakuum entfernt. Man erhält 0.35 g (98 % d. Th.) des Produkts als gelbes Öl.
HPLC (Methode 12): Rₜ = 4.6 min.
MS (DCI, NH₃): m/z = 241 [M+H]⁺, 258 [M+NH₄]⁺.

### Ausführüngsbeispiele:

Die nachfolgenden Verbindungen werden nach dem in Schema I dargestellten allgemeinen Syntheseweg hergestellt: 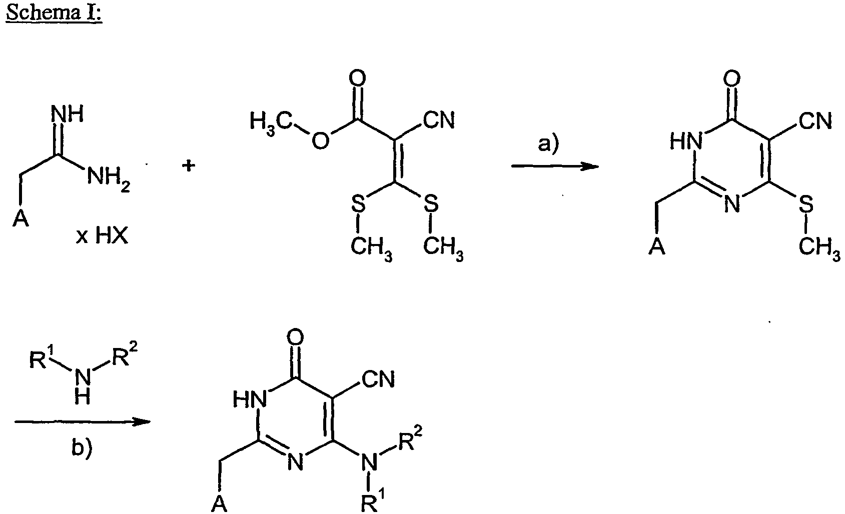 X = Cl, Br
a) Ethanol, Triethylamin, 5-16 h Rückfluss; b) Acetonitril, 85-90°C, 1-7 Tage.

### Beispiel 1

### 2-(3,4-Dichlorbenzyl)-6-oxo-4-(1-piperidinyl)-1,6-dihydropyrimidin-5-carbonitril

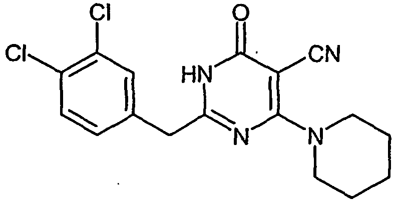

100 mg (0.34 mmol) 2-(3,4-Dichlorbenzyl)-4-(methylsulfanyl)-6-oxo-1,6-dihydropyrimidin 5-carbonitril werden mit 261 mg (3.07 mmol) Piperidin 16 h bei 85°C gerührt. Nach Entfernen der flüchtigen Bestandteile im Vakuum wird der Rückstand mittels präparativer HPLC gereinigt. Es werden 42 mg (38 % d. Th.) der Titelverbindung erhalten.
HPLC (Methode 12): Rₜ = 4.8 min.
MS (ESIpos): m/z = 363 [M+H]⁺
¹H-NMR (DMSO-d₆, 200 MHz): δ = 1.45-1.70 (m, 6H), 3.73-3.89 (m, 6H), 7.35 (dd, 1H), 7.57-7.66 (m, 2H), 11.1 (s, 1H).

### Beispiel 2

### 2-Benzyl-6-oxo-4-(1-piperidinyl)-1,6-dihydropyrimidin-5-carbonitril

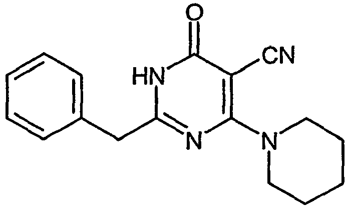

43 mg (0.17 mmol) 2-Benzyl-4-(methylsulfanyl)-6-oxo-1,6-dihydropyrimidin-5-carbonitril werden in 0.3 ml Acetonitril suspendiert und mit 42.7 mg (0.50 mmol) Piperidin 16 h bei 85°C gerührt. Im Anschluss wird das entstandene Rohprodukt mittels präparativer HPLC gereinigt. Es werden 11 mg (22 % d. Th.) der Titelverbindung erhalten.
HPLC (Methode 12): Rₜ = 4.3 min.
MS (ESIpos): m/z = 295 [M+H]⁺
¹H-NMR (CD₃OD, 300 MHz): δ = 1.59-1.77 (m, 6H), 3.83 (s, 2H), 3.93 (t, 4H), 7.24-7.34 (m, 5H).

### Beispiel 3

### 4-[4-(2-Hydroxyethyl)-1-piperidinyl]-2-(3-methylbenzyl)-6-oxo-1,6-dihydro-5-pyrimidincarbonitril

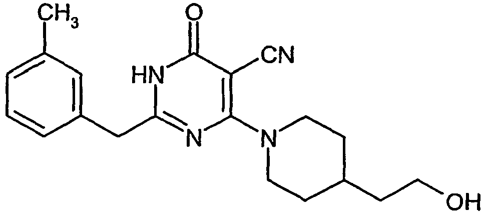

0.1 g (0.37 mmol) 2-(3-Methylbenzyl)-4-(methylsulfanyl)-6-oxo-1,6-dihydro-5-pyrimidincarbonitril werden unter Argon mit 0.142 g (1.16 mmol) 2-(4-Piperidinyl)ethan-1-ol in 3 ml Acetonitril sieben Tage auf 90°C erhitzt. Nach Abkühlen auf Raumtemperatur wird das Rohprodukt mittels präparativer HPLC gereinigt. Es werden 0.047 g (36 % d. Th.) der Titelverbindung als farbloser Feststoff erhalten.
LC-MS (Methode 7): Rₜ = 3.01 min., MS (ESIpos): m/z = 353 [M+H]⁺
¹H-NMR (DMSO-d₆, 300 MHz): δ = 1.05 (m, 2H), 1.36 (m, 2H), 1.69 (m, 3H), 2.25 (s, 3H), 2.89 (t, 2H), 3.42 (t, 2H), 3.68 (s, 2H), 4.51 (d, 2H), 7.18 (m, 4H).

### Beispiel 4

### 4-(Cyclopentylamino)-2-(3-methylbenzyl)-6-oxo-1,6-dihydro-5-pyrimidincarbonitril

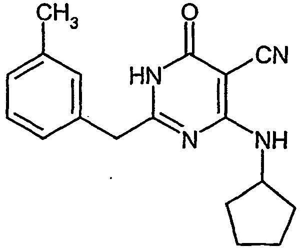

0.1 g (0.37 mmol) 2-(3-Methylbenzyl)-4-(methylsulfanyl)-6-oxo-1,6-dihydro-5-pyrimidincarbonitril werden unter Argon mit 0.31 g (3.65 mmol) Cyclopentylamin in 3 ml Acetonitril über Nacht auf 90°C erhitzt. Nach Abkühlen auf Raumtemperatur wird das Rohprodukt mittels präparativer HPLC gereinigt. Es werden 0.03 g (26 % d. Th.) der Titelverbindung als farbloser Feststoff erhalten.
LC-MS (Methode 10): Rₜ = 2.27 min., MS (ESIpos): m/z = 309 [M+H]⁺.

### Beispiel 5

### 4-[(2S)-2-(Hydroxymethyl)-1-pyrrolidinyl]-2-(3-methylbenzyl)-6-oxo-1,6-dihydro-5-pyrimidin-carbonitril

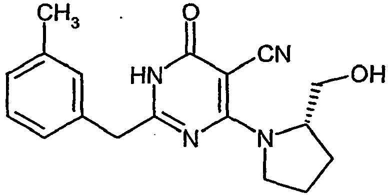

0.1 g (0.37 mmol) 2-(3-Methylbenzyl)-4-(methylsulfanyl)-6-oxo-1,6-dihydro-5-pyrimidincarbonitril werden unter Argon mit 0.11 g (1.1 mmol) (5)-(+)-2-Pyrrolidinmethanol in 3 ml Acetonitril fünf Tage auf 90°C erhitzt. Nach Abkühlen auf Raumtemperatur wird das Rohprodukt mittels präparativer HPLC gereinigt Es werden 0.035 g (29 % d. Th.) der Titelverbindung als farbloser Feststoff erhalten.
LC-MS (Methode 7): Rₜ = 2.91 min., MS (ESIpos): m/z = 325 [M+H]⁺
¹H-NMR (DMSO-d₆, 300 MHz): δ = 1.94 (m, 4H), 2.28 (s, 2H), 3.76 (m, 3H), 3.70 (s, 2H), 3.81 (m, 2H), 4.4 (m, 1H), 7.15 (m, 4H), 12.34 (s, 1H).

### Beispiel 6

### 4-[4-(2-Hydroxyethyl)-1-piperidinyl]-2-(2-methylbenzyl)-6-oxo-1,6-dihydro-5-pyrimidincarbonitril

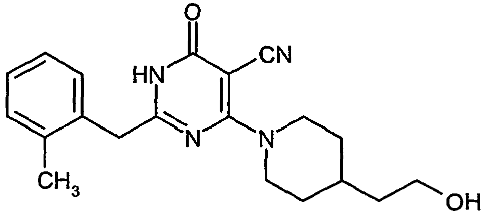

0.1 g (0.37 mmol) 2-(2-Methylbenzyl)-4-(methylsulfanyl)-6-oxo-1,6-dihydro-5 pyrimidincarbonitril werden unter Argon mit 0.14 g (1.1 mmol) 2-(4-Piperidinyl)ethan-1-ol in 3 ml Acetonitril fünf Tage auf 90°C erhitzt. Nach Abkühlen auf Raumtemperatur wird das Rohprodukt mittels präparativer HPLC gereinigt. Es werden 13 mg (10 % d. Th.) der Titelverbindung als farbloser Feststoff erhalten. -
LC-MS (Methode 5): Rₜ= 1.74 min., MS (ESIpos): m/z = 353 [M+H]⁺
¹H-NMR (DMSO-d₆, 300 MHz): δ = 1.06 (m, 2H), 1.34 (m, 2H), 1.68 (m, 3H), 2.30 (s, 3H), 3.00 (t, 2H), 3.42 (t, 2H), 3.80 (s, 2H), 4.51 (d, 2H), 7.16 (m, 4H), 12.33 (s, 1H).

### Beispiel 7

### 2-(2-Fluorbenzyl)-4-[4-(2-hydroxyethyl)-1-piperidinyl]-6-oxo-1,6-dihydro-5-pyrimidincarbonitril

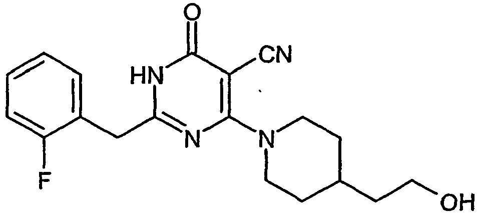

0.1 g (0.37 mmol) 2-(2-Fluorbenzyl)-4-(methylsulfanyl)-6-oxo-1,6-dihydro-5-pyrimidincarbonitril werden unter Argon mit 0.14 g (1.1 mmol) 2-(4-Piperidinyl)ethan-1-ol in 3 ml Acetonitril sechs Tage auf 90°C erhitzt. Nach Abkühlen auf Raumtemperatur wird das Rohprodukt mittels präparativer HPLC gereinigt. Es werden 31 mg (24 % d. Th.) der Titelverbindung als farbloser Feststoff erhalten.
LC-MS (Methode 2): Rₜ = 2.94 min., MS (ESIpos): m/z = 357 [M+H]⁺
¹H-NMR (DMSO-d₆, 300 MHz): δ = 1.01 (m, 2H), 1.33 (m, 2H), 1.67 (m, 3H), 2.97 (t, 2H), 3.41 (dd, 2H), 3.87 (s, 2H), 4.32 (t, 1H), 4.47 (d, 2H), 7.16 (m, 2H), 7.36 (m, 2H), 12.38 (s, 1H).

### Beispiel 8

### 2-(2-Ethoxybenzyl)-4-[4-(2-hydroxyethyl)-1-piperidinyl]-6-oxo-1,6-dihydro-5-pyrimidincarbonitril

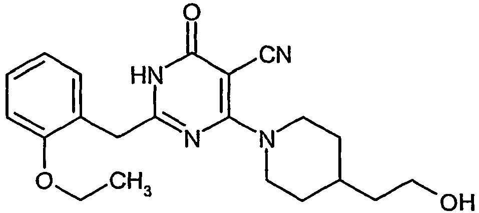

0.1 g (0.37 mmol) 2-(2-Ethoxybenzyl)-4-(methylsulfanyl)-6-oxo-1,6-dihydro-5-pyrimidincarbonitril werden unter Argon mit 0.13 g (0.99 mmol) 2-(4-Piperidinyl)ethan-1-ol in 3 ml Acetonitril fünf Tage auf 90°C erhitzt. Nach Abkühlen auf Raumtemperatur wird das Rohprodukt mittels präparativer HPLC- gereinigt. Es werden 45 mg (43 % d. Th.) der Titelverbindung als farbloser Feststoff erhalten.
LC-MS (Methode 6): Rₜ = 2.01 min., MS (ESIpos): m/z = 383 [M+H]⁺
¹H-NMR (DMSO-d₆, 200 MHz): δ = 1.04 (m, 2H), 1.23 (t, 3H), 1.34 (m, 2H), 1.67 (m, 3H), 2.94 (t, 2H), 3.41 (t, 2H), 3.87 (s, 2H), 3.96 (q, 2H), 4.48 (d, 2H), 6.92 (m, 2H), 7.17 (m, 2H), 12.26 (s, 1H).

### Beispiel 9

### 2-(3-Chlorbenzyl)-6-oxo-4-(propylamino)-1,6-dihydropyrimidin-5-carbonitril

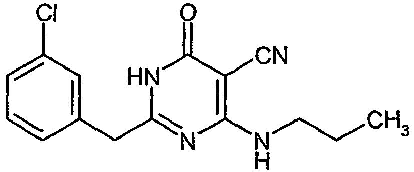

Analog zur Darstellung von Beispiel 1 werden 100 mg (0.34 mmol) 2-(3-Chlorbenzyl)-4-(methyl-sulfanyl)-6-oxo-1,6-dihydropyrimidin-5-carbonitril mit 203 mg (3.43 mmol) n-Propylamin zu 12 mg (12 % d. Th.) der Titelverbindung umgesetzt.
HPLC (Methode 12): Rₜ = 4.4 min.
MS (ESIpos): m/z = 303 [M+H]⁺
¹H-NMR (DMSO-d₆, 200 MHz): δ = 0.75 (t, 3H), 1.40 (m, 2H), 3.23 (m, 2H), 3.83 (s, 2H), 7.23-7.3 (m, 4H), 7.42 (s, 1H), 12.34 (s, 1H).

### Beispiel 10

### 2-(3-Chlorbenzyl)-4-(cyclopentylamino)-6-oxo-1,6-dihydropyrimidin-5-carbonitril

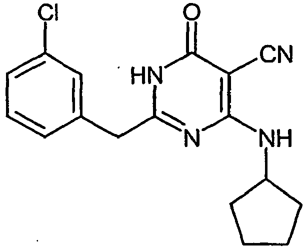

Analog zur Darstellung von Beispiel 1 werden 100 mg (0.34 mmol) 2-(3-Chlorbenzyl)-4-(methyl-sulfanyl)-6-oxo-1,6-dihydropyrimidin-5-carbonitril mit 292 mg (3.43 mmol) Cyclopentylamin zu 10 mg (9 % d. Th.) der Titelverbindung umgesetzt.
HPLC (Methode 12): Rₜ = 4.6 min.
MS (ESIpos): m/z = 329 [M+H]⁺
¹H-NMR (DMSO-d₆, 200 MHz): δ = 1.38-1.85 (m, 8H), 3.82 (s, 2H), 4.20-4.37 (m, 1H), 7.23-7.46 (m, 4H), 7.66-7.80 (s, 1H), 12.25-12.44 (s, 1H).

### Beispiel 11

### 2-(3-Chlorbenzyl)-6-oxo-4-(1-pyrrolidinyl)-1,6-dihydropyrimidin-5-carbonitril

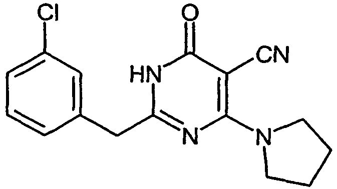

Analog zur Darstellung von Beispiel 1 werden 100 mg (0.34 mmol) 2-(3-Chlorbenzyl)-4-(methyl-sulfanyl)-6-oxo-1,6-dihydropyrimidin-5-carbonitril mit 244 mg (3.43 mmol) Pyrrolidin zu 29 mg (27 % d. Th.) der Titelverbindung umgesetzt.
HPLC (Methode 12): Rₜ = 4.4 min.
MS (ESIpos): m/z = 315 [M+H]⁺
¹H-NMR (DMSO-d₆, 200 MHz): δ = 1.80-1.95 (m, 4H), 3.58-3.71 (m, 4H), 3.79 (s, 2H), 7.25-7.45 (m, 4H), 12.28-12.39 (s, 1H).

### Beispiel 12

### 4-(4,4-Dimethylpiperidin-1-yl)-2-(3-chlorbenzyl)-6-oxo-1,6-dihydropyrimidin-5-carbonitril

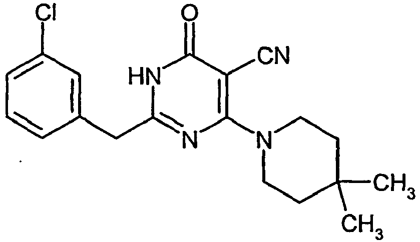

Analog zur Darstellung von Beispiel 1 werden 150 mg (0.51 mmol) 2-(3-Chlorbenzyl)-4-(methyl-sulfanyl)-6-oxo-1,6-dihydropyrimidin-5-carbonitril mit 582 mg (5.14 mmol) 4,4-Dimethylpiperidin zu 96 mg (52 % d. Th.) der Titelverbindung umgesetzt.
HPLC (Methode 12): Rₜ = 4.8 min.
MS (ESIpos): m/z = 357 [M+H]⁺
¹H-NMR (DMSO-d₆, 200 MHz): δ = 0.96 (s, 6H), 1.30-1.41 (m, 4H), 3.75-3.87 (m, 6H, s bei 3.81), 7.24-7.45 (m, 4H), 12.39 (s, 1H).

### Beispiel 13

### 2-(3-Chlorbenzyl)-4-[(2-methoxyethyl)amino]-6-oxo-1,6-dihydropyrimidin-5-carbonitril

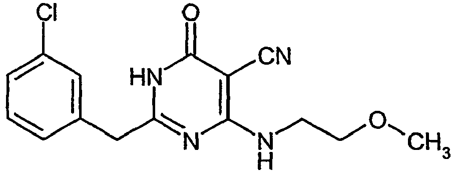

Analog zur Darstellung von Beispiel 1 werden 100 mg (0.34 mmol) 2-(3-Chlorbenzyl)-4-(methyl-sulfanyl)-6-oxo-1,6-dihydropyrimidin-5-carbonitril mit 257 mg (3.43 mmol) 2-Methoxyethylamin zu 61 mg (56 % d. Th.) der Titelverbindung umgesetzt.
HPLC (Methode 12): Rₜ = 4.0 min.
MS (ESIpos): m/z = 319 [M+H]⁺
¹H-NMR (CDCl₃, 300 MHz): δ = 3.38 (s, 3H), 3.53 (t, 2H), 3.75 (q, 2H), 3.88 (s, 2H), 6.00 (t, 1H), 7.25-7.31 (m, 3H), 7.38 (s, 1H), 12.56 (s, 1H).

### Beispiel 14

### 2-(3-Chlorbenzyl)-4-(morpholin-4-yl)-6-oxo-1,6-dihydropyrimidin-5-carbonitril

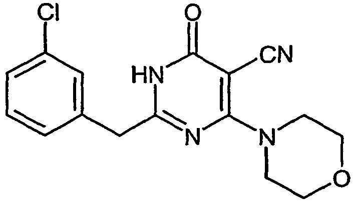

Analog zur Darstellung von Beispiel 1 werden 150 mg (0.51 mmol) 2-(3-Chlorbenzyl)-4-(methyl-sulfanyl)-6-oxo-1,6-dihydropyrimidin-5-carbonitril mit 448 mg (5.14 mmol) Morpholin zu 109 mg (63 % d. Th.) der Titelverbindung umgesetzt.
HPLC (Methode 12): Rₜ = 4.0 min.
MS (ESIpos): m/z =331 [M+H]⁺
¹H-NMR (DMSO-d₆, 200 MHz): δ = 3.59-3.69 (t, 4H), 3.79-3.90 (m, 6H, s bei 3.82), 7.25-7.44 (m, 4H), 12.53 (s, 1H).

### Beispiel 15

### 2-(3-Chlorbenzyl)-4-(4-methylpiperazin-1-yl)-6-oxo-1,6-dihydropyrimidin-5-carbonitril

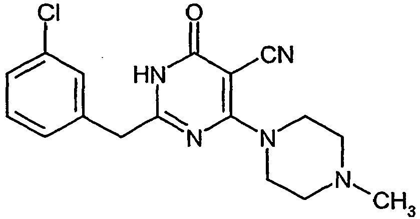

Analog zur Darstellung von Beispiel 1 werden 100 mg (0.34 mmol) 2-(3-Chlorbenzyl)-4-(methyl-sulfanyl)-6-oxo-1,6-dihydropyrimidin-5-carbonitril mit 343 mg (3.43 mmol) N-Methylpiperazin zu 101 mg (84 % d. Th.) der Titelverbindung umgesetzt.
HPLC (Methode 12): Rₜ = 3.5 min.
MS (ESIpos): m/z = 344 [M+H]⁺
¹H-NMR (DMSO-d*₆*, 200 MHz): δ = 2.18 (s, 3H), 2.35 (t, 4H), 3.79-3.88 (m, 6H, s bei 3.82), 7.25-7.44 (m, 4H), 12.48 (s, 1H).

### Beispiel 16

### 2-(3-Chlorbenzyl)-4-[(2-methoxybenzyl)amino]-6-oxo-1,6-dihydropyrimidin-5-carbonitril

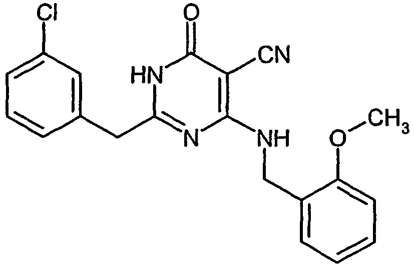

Analog zur Darstellung von Beispiel 1 werden 100 mg (0.34 mmol) 2-(3-Chlorbenzyl)-4-(methyl-sulfanyl)-6-oxo-1,6-dihydropyrimidin-5-carbonitril mit 470 mg (3.43 mmol) 2-Methoxybenzylamin zu 37 mg (28 % d. Th.) der Titelverbindung umgesetzt.
HPLC (Methode 12): Rₜ = 4.6 min.
MS (ESIpos): m/z = 381 [M+H]⁺
¹H-NMR (DMSO-d₆, 300 MHz): δ = 3.77 (s, 2H), 3.79 (s, 3H), 4.52 (d, 2H), 6.84 (t, 1H), 6.92-6.99 (m, 2H), 7.12 (d, 1H), 7.19-7.33 (m, 4H), 8.14 (t, 1H), 12.39 (s, 1H).

### Beispiele 17

### 2-(4-Chlorbenzyl)-4-(cyclobutylamino)-6-oxo-1,6-dihydropyrimidin-5-carbonitril

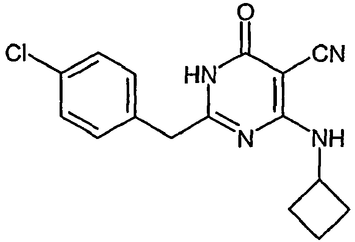

Analog zur Darstellung von Beispiel 1 werden 100 mg (0.34 mmol) 2-(4-Chlorbenzyl)-4-(methyl-sulfanyl)-6-oxo-1,6-dihydropyrimidin-5-carbonitril mit 244 mg (3.43 mmol) Cyclobutylamin zu 15 mg (14 % d. Th.) der Titelverbindung umgesetzt.
HPLC (Methode 12): Rₜ = 4.5 min.
MS (ESIpos): m/z = 315 [M+H]⁺
¹H-NMR (DMSO-d₆, 300 MHz): δ = 1.50-1.67 (m, 2H), 2.03-2.15 (m, 4H), 3.79 (s, 2H), 4.37-4.51 (m, 1H), 7.31-7.43 (m, 4H), 8.00 (s, 1H), 12.33 (s, 1H).

### Beispiel 18

### 2-(4-Chlorbenzyl)-6-oxo-4-(1-pyrrolidinyl)-1,6-dihydropyrimidin-5-carbonitril

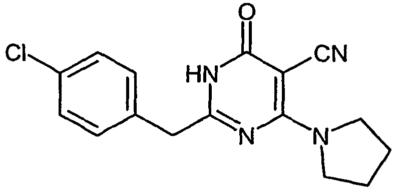

Analog zur Darstellung von Beispiel 1 werden 100 mg (0.34 mmol) 2-(4-Chlorbenzyl)-4-(methyl-sulfanyl)-6-oxo-1,6-dihydropyrimidin-5-carbonitril mit 244 mg (3.43 mmol) Pyrrolidin zu 45 mg (42% d. Th.) der Titelverbindung umgesetzt.
HPLC (Methode 12): Rₜ = 4.4 min.
MS (ESIpos): m/z = 315 [M+H]⁺
¹H-NMR (CD₃OD, 300 MHz): δ = 1.88-2.03 (m, 4H), 3.69-3.86 (m, 6H, s bei 3.82), 7.25-7.35 (m, 4H).

### Beispiel 19

### 4-(Cyclopentylamino)-2-(3-fluorbenzyl)-6-oxo-1,6-dihydropyrimidin-5-carbonitril

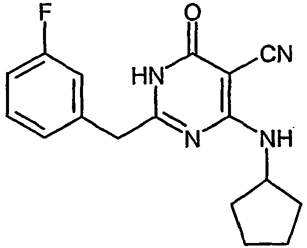

Analog zur Darstellung von Beispiel 1 werden 100 mg (0.36 mmol) 2-(3-Fluorbenzyl)-4-(methyl-sulfanyl)-6-oxo-1,6-dihydropyrimidin-5-carbonitril mit 309 mg (3.63 mmol) Cyclopentylamin zu 12 mg (11 % d. Th.) der Titelverbindung umgesetzt.
HPLC (Methode 12): Rₜ = 4.4 min.
MS (ESIpos): m/z = 313 [M+H]⁺
¹H-NMR (DMSO-d₆, 200 MHz): δ = 1.29-1.71 (m, 8H), 3.89 (s, 2H), 3.98-4.14 (m, 1H), 7.11-7.42 (m, 4H), 7.63-7.75 (s, 1H), 12.34-12.43 (s, 1H).

### Beispiel 20

### 2-(3-Fluorbenzyl)-6-oxo-4-(1-piperidinyl)-1,6-dihydropyrimidin-5-carbonitril

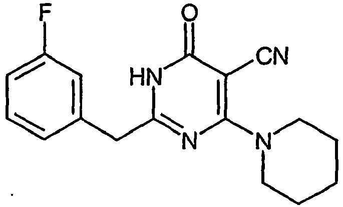

Analog zur Darstellung von Beispiel 1 werden 100 mg (0.36 mmol) 2-(3-Fluorbenzyl)-4-(methyl-sulfanyl)-6-oxo-1,6-dihydropyrimidin-5-carbonitril mit 309 mg (3.63 mmol) Piperidin zu 40 mg (35 % d. Th.) der Titelverbindung umgesetzt.
HPLC (Methode 12): Rₜ = 4.3 min.
MS (ESIpos): m/z = 313 [M+H]⁺
¹H-NMR (DMSO-d₆, 200 MHz): δ = 1.40-1.66 (m, 6H), 3.66-3.76 (m, 4H), 3.76 (s, 2H), 7.12-7.42 (m, 4H), 12.27-12.41 (s, 1H).

### Beispiel 21

### 6-Oxo-4-(1-piperidinyl)-2-[3-(trifluormethyl)benzyl]-1,6-dihydropyrimidin-5-carbonitril

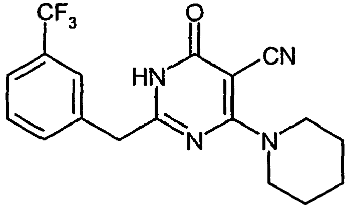

Analog zur Darstellung von Beispiel 1 werden 100 mg (0.31 mmol) 4-(Methylsulfanyl)-6-oxo-2-[3-(trifluormethyl)benzyl]-1,6-dihydropyrimidin-5-carbonitril mit 262 mg (3.07 mmol) Piperidin zu 26 mg (22 % d. Th.) der Titelverbindung umgesetzt.
HPLC (Methode 12): Rₜ = 4.7 min.
MS (ESIpos): m/z = 363 [M+H]⁺
¹H-NMR (DMSO-d₆, 200 MHz): δ = 1.43-1.70 (m, 6H), 3.71-3.83 (m, 4H), 3.93 (s, 2H), 7.51-7.78 (m, 4H), 12.42 (s, 1H).

### Beispiel 22

### 6-Oxo-4-(propylamino)-2-(3-thienylmethyl)-1,6-dihydropyrimidin-5-carbonitril

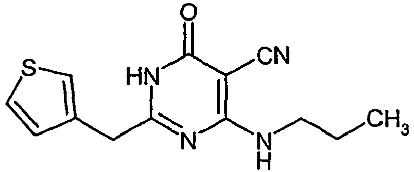

Analog zur Darstellung von Beispiel 1 werden 100 mg (0.38 mmol) 4-(Methylsulfanyl)-6-oxo-2-(3-thienylmethyl)-1,6-dihydropyrimidin-5-carbonitril mit 224 mg (3.80 mmol) n-Propylamin zu 14 mg (13 % d. Th.) der Titelverbindung umgesetzt.
HPLC (Methode 12): Rₜ = 4.1 min.
MS (ESIpos): m/z = 275.2 [M+H]⁺
¹H-NMR (DMSO-d₆, 300 MHz): δ = 0.79 (t, 3H), 1.46 (m, 2H), 3.29 (m, 2H), 3.81 (s, 2H), 7.06 (d, 1H), 7.33 (s, 1H), 7.49 (m, 1H), 7.87 (s, 1H), 12.27 (s, 1H).

### Beispiel 23

### 4-(Cyclopropylamino)-6-oxo-2-(3-thienylmethyl)-1,6-dihydropyrimidin-5-carbonitril

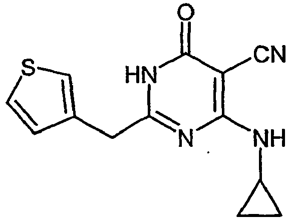

Analog zur Darstellung von Beispiel 1 werden 100 mg (0.38 mmol) 4-(Methylsulfanyl)-6-oxo-2-(3-thienylmethyl)-1,6-dihydropyrimidin-5-carbonitril mit 217 mg (3.80 mmol) Cyclopropylamin zu 44 mg (43 % d. Th.) der Titelverbindung umgesetzt.
HPLC (Methode 12): Rₜ = 3.8 min.
MS (ESIpos): m/z = 273 [M+H]⁺
¹H-NMR (DMSO-d₆, 300 MHz): δ = 0.60-0.78 (m, 4H), 2.84-2.98 (m, 1H), 3.80 (s, 2H), 7.08 (d, 1H), 7.35 (s, 1H), 7.49 (m, 1H), 7.85-8.05 (s, 1H), 12.32 (s, 1H).

### Beispiel 24

### 4-(Cyclopentylamino)-6-oxo-2-(3-thienylmethyl)-1,6-dihydropyrimidin-5-carbonitril

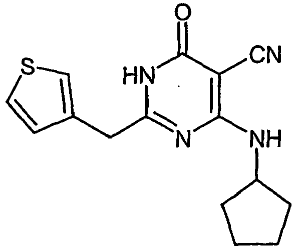

Analog zur Darstellung von Beispiel 1 werden 150 mg (0.57 mmol) 4-(Methylsulfanyl)-6-oxo-2-(3-thienylmethyl)-1,6-dihydropyrimidin-5-carbonitril mit 485 mg (5.70 mmol) Cyclopentylamin zu 46 mg (26 % d. Th.) der Titelverbindung umgesetzt
HPLC (Methode 12): Rₜ = 4.4 min.
MS (ESIpos): m/z = 301.2 [M+H]⁺
¹H-NMR (DMSO-d₆, 300 MHz): δ = 1.40-1.91 (m, 8H), 3.81 (s, 2H), 4.36 (m, 1H), 7.07 (d, 1H), 7.34 (s, 1H), 7.50 (m, 1H), 7.65 (s, 1H), 12.28 (s, 1H).

### Beispiel 25

### 6-Oxo-4-(1-pyrrolidinyl)-2-(3-thienylmethyl)-1,6-dihydropyrimidin-5-carbonitril

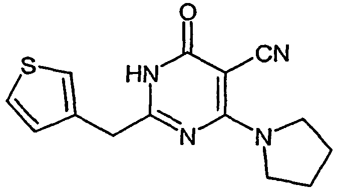

Analog zur Darstellung von Beispiel 1 werden 100 mg (0.38 mmol) 4-(Methylsulfanyl)-6-oxo-2-(3-thienylmethyl)-1,6-dihydropyrimidin-5-carbonitril mit 270 mg (3.80 mmol) Pyrrolidin zu 64 mg (59 % d. Th.) der Titelverbindung umgesetzt.
HPLC (Methode 12): Rₜ = 4.1 min.
MS (ESIpos): m/z = 287 [M+H]⁺
¹H-NMR (DMSO-d₆, 300 MHz): δ = 1.80-1.96 (m, 4H), 3.60-3.76 (m, 4H), 3.78 (s, 2H), 7.08 (d, 1H), 7.35 (s, 1H), 7.48 (m, 1H), 12.27 (s, 1H).

### Beispiel 26

### 4-(4-Methylpiperidin-1-yl)-6-oxo-2-(3-thienylmethyl)-1,6-dihydropyrimidin-5-carbonitril

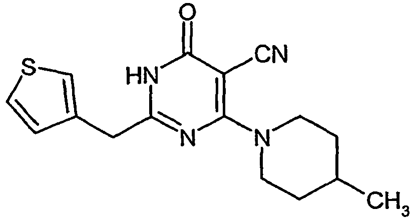

Analog zur Darstellung von Beispiel 1 werden 100 mg (0.38 mmol) 4-(Methylsulfanyl)-6-oxo-2-(3-thienylmethyl)-1,6-dihydropyrimidin-5-carbonitril mit 377 mg (3.80 mmol) 4-Methylpiperidin zu 65 mg (54 % d. Th.) der Titelverbindung umgesetzt.
HPLC (Methode 12): Rₜ = 4.5 min.
MS (ESIpos): m/z = 315 [M+H]⁺
¹H-NMR (DMSO-d₆, 300 MHz): δ = 0.91 (d, 3H), 1.05-1.18 (t, 2H), 1.62-1.77 (m, 3H), 3.06 (t, 2H), 3.80 (s, 2H), 4.61 (d, 2H), 7.07 (d, 1H), 7.34 (s, 1H), 7.49 (m, 1H), 12.32 (s, 1H).

### Beispiel 27

### 4-(4,4-Dimethylpiperidin-1-yl)-6-oxo-2-(3-thienylmethyl)-1,6-dihydropyrimidin-5-carbonitril

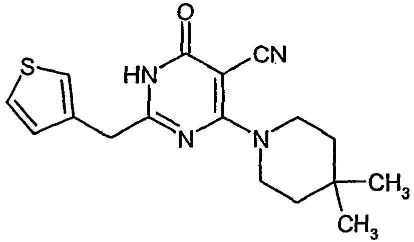

Analog zur Darstellung von Beispiel 1 werden 100 mg (0.38 mmol) 4-(Methylsulfanyl)-6-oxo-2-(3-thienylmethyl)-1,6-dihydropyrimidin-5-carbonitril mit 430 mg (3.80 mmol) 4,4-Dimethyl-piperidin zu 42 mg (34 % d. Th.) der Titelverbindung umgesetzt.
HPLC (Methode 12): Rₜ = 4.6 min.
MS (ESIpos): m/z = 329 [M+H]⁺
¹H-NMR (DMSO-d₆, 300 MHz): δ = 0.97 (s, 6H), 1.37 (t, 4H), 3.77-3.87 (m, 6H, s bei 3.80), 7.06 (d, 1H), 7.34 (s, 1H), 7.49 (m, 1H), 12:31 (s, 1H).

### Beispiel 28

### 4-[4-(tert.-Butyl)piperidin-1-yl]-6-oxo-2-(3-thienylmethyl)-1,6-dihydropyrimidin-5-carbonitril

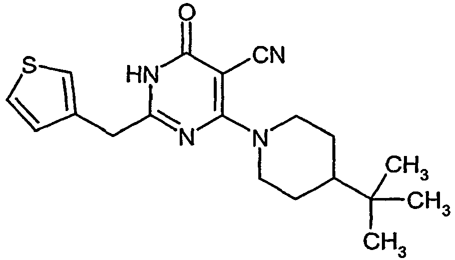

Analog zur Darstellung von Beispiel 1 werden 100 mg (0.38 mmol) 4-(Methylsulfanyl)-6-oxo-2-(3-thienylmethyl)-1,6-dihydropyrimidin-5-carbonitril mit 536 mg (3.80 mmol) 4-*tert*.-Butyl-piperidin zu 57 mg (42 % d. Th.) der Titelverbindung umgesetzt.
HPLC (Methode 12): Rₜ = 4.9 min.
MS (ESIpos): m/z = 357 [M+H]⁺
¹H-NMR (DMSO-d₆, 200 MHz): δ = 0.82 (s, 9H), 1.02-1.42 (m, 3H), 1.74 (d, 2H), 2.96 (t, 2H), 3.78 (s, 2H), 4.72 (d, 2H), 7.06 (d, 1H), 7.33 (s, 1H), 7.49 (m, 1H), 12.35 (s, 1H).

### Beispiel 29

### 4-(4-Hydroxypiperidin-1-yl)-6-oxo-2-(3-thienylmethyl)-1,6-dihydropyrimidin-5-carbonitril

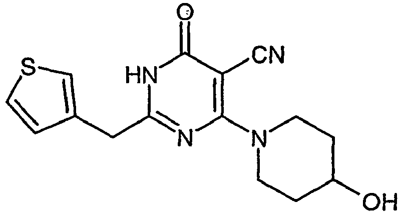

Analog zur Darstellung von Beispiel 1 werden 100 mg (0.38 mmol) 4-(Methylsulfanyl)-6-oxo-2-(3-thienylmethyl)-1,6-dihydropyrimidin-5-carbonitril mit 384 mg (3.80 mmol) Piperidin-4-ol zu 47 mg (39 % d. Th.) der Titelverbindung umgesetzt.
HPLC (Methode 12): Rₜ = 3.50 min.
MS (ESIpos): m/z = 317 [M+H]⁺
¹H-NMR (DMSO-d₆, 200 MHz): δ = 1.29-1.50 (m, 2H), 1.72-1.90 (m, 2H), 3.42-3.60 (m, 2H), 3.68-3.86 (m, 3H, s bei 3.79), 4.10-4.26 (m, 2H), 4.83 (d, 1H, OH), 7.07 (d, 1H), 7.35 (s, 1H), 7.50 (m, 1H), 11.79-12.29 (s, 1H, NH).

### Beispiel 30

### 4-[4-(2-Hydroxyethyl)piperidin-1-yl]-6-oxo-2(3-thienylmethyl)-1,6-dihydropyrimidin-5-carbonitril

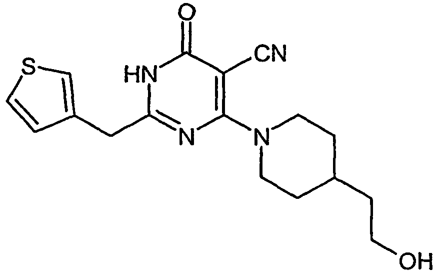

Analog zur Darstellung von Beispiel 1 werden 100 mg (0.38 mmol) 4-(Methylsulfanyl)-6-oxo-2-(3-thienylmethyl)-1,6-dihydropyrimidin-5-carbonitril mit 491 mg (3.80 mmol) 2-(4-Piperidinyl)-ethan-1-ol zu 64 mg (49 % d. Th.) der Titelverbindung umgesetzt
HPLC (Methode 12): Rₜ = 3.70 min.
MS (ESIpos): m/z = 345 [M+H]⁺
¹H-NMR (DMSO-d₆, 300 MHz): δ = 1.02-1.20 (m, 2H), 1.32-1.42 (q, 2H), 1.68-1.81 (m, 3H), 3.05 (t, 2H), 3.44 (q, 2H), 3.80 (s, 2H), 4.34 (t, 1H, OH), 4.62 (d, 2H), 7.06 (d, 1H), 7.34 (s, 1H), 7.49 (m, 1H), 12.31 (s, 1H, NH).

### Beispiel 31

### 4-[(2-Methoxyethyl)amino]-6-oxo-2-(3-thienylmethyl)-1,6-dihydropyrimidin-5-carbonitril

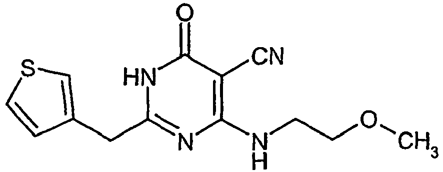

Analog zur Darstellung von Beispiel 1 werden 100 mg (0.38 mmol) 4-(Methylsulfanyl)-6-oxo-2-(3-thienylmethyl)-1,6-dihydropyrimidin-5-carbonitril mit 285 mg (3.80 mmol) 2-Methoxyethyl-amin zu 76 mg (69 % d. Th.) der Titelverbindung umgesetzt.
HPLC (Methode 12): Rₜ = 3.7 min.
MS (ESIpos): m/z = 291 [M+H]⁺
¹H-NMR (DMSO-d₆, 300 MHz): δ = 3.20 (s, 3H), 3.35-3.42 (m, 2H), 3.47-3.56 (m, 2H), 3.82 (s, 2H), 7.07 (d, 1H), 7.34 (s, 1H), 7.49 (m, 1H), 7.79 (s, 1H), 12.33 (s, 1H).

### Beispiel 32

### 4-(1,4-Dioxa-8-azaspiro[4.5]dec-8-yl)-6-oxo-2-(3-thienylmethyl)-1,6-dihydropyrimidin-5-carbonitril

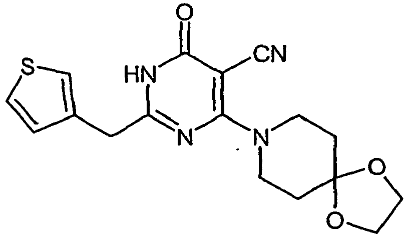

Analog zur Darstellung von Beispiel 1 werden 100 mg (0.38 mmol) 4-(Methylsulfanyl)-6-oxo-2-(3-thienylmethyl)-1,6-dihydropyrimidin-5-carbonitril mit 544 mg (3.80 mmol) 1,4-Dioxa-8-aza-spiro[4.5]decan zu 65 mg (48 % d. Th.) der Titelverbindung umgesetzt.
HPLC (Methode 12): Rₜ = 4.0 min.
MS (ESIpos): m/z = 359 [M+H]⁺
¹H-NMR (DMSO-d₆, 300 MHz): δ = 1.70 (t, 4H), 3.81 (s, 2H), 3.86-3.95 (m, 8H, s bei 3.92), 7.07 (d, 1H), 7.34 (s, 1H), 7.49 (m, 1H), 12.41 (s, 1H).

### Beispiel 33

### 4-(7,11-Dioxa-3-azaspiro[5.5]undec-3-yl)-6-oxo-2-(3-thienylmethyl)-1,6-dihydropyrimidin-5-carbonitril

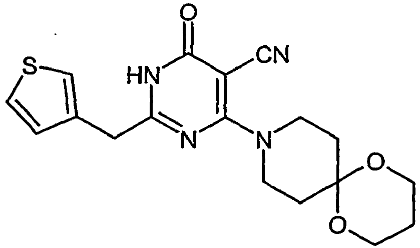

Analog zur Darstellung von Beispiel 1 werden 100 mg (0.38 mmol) 4-(Methylsulfanyl)-6-oxo-2-(3-thienylmethyl)-1,6-dihydropyrimidin-5-carbonitril mit 597 mg (3.80 mmol) 1,5-Dioxa-9-aza-spiro[5.5]undecan zu 86 mg (61 % d. Th.) der Titelverbindung umgesetzt.
HPLC (Methode 12): Rₜ = 4.0 min.
MS (ESIpos): m/z = 373 [M+H]⁺
¹H-NMR (DMSO-d₆, 200 MHz): δ = 1.56 (m, 2H), 1.82-1.94 (m, 4H), 3.75-3.92 (m, 10H, s bei 3.80), 7.07 (d, 1H), 7.35 (s, 1H), 7.50 (m, 1H), 12.43 (br. s, 1H).

### Beispiel 34

### 4-(4-Methylpiperazin-1-yl)-6-oxo-2-(3-thienylmethyl)-1,6-dihydropyrimidin-5-carbonitril

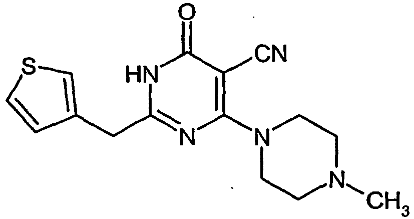

Analog zur Darstellung von Beispiel 1 werden 100 mg (0.38 mmol) 4-(Methylsulfanyl)-6-oxo-2-(3-thienylmethyl)-1,6-dihydropyrimidin-5-carbonitril mit 380 mg (3.80 mmol) N-Methylpiperazin zu 36 mg (30 % d. Th.) der Titelverbindung umgesetzt.
HPLC (Methode 12): Rₜ = 3.2 min.
MS (ESIpos): m/z = 316 [M+H]⁺
¹H-NMR (DMSO-d₆, 200 MHz): δ = 2.19 (s, 3H), 2.37 (t, 4H), 3.77-3.91 (m, 6H, s bei 3.80), 7.07 (d, 1H), 7.35 (s, 1H), 7.50 (m, 1H), 12.43 (s, 1H).

### Beispiel 35

### 6-Oxo-4-(piperazin-1-yl)-2-(3-thienylmethyl)-1,6-dihydropyrimidin-5-carbonitril

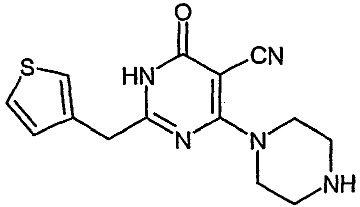

Analog zur Darstellung von Beispiel 1 werden 80 mg (0.30 mmol) 4-(Methylsulfanyl)-6-oxo-2-(3-thienylmethyl)-1,6-dihydropyrimidin-5-carbonitril mit 326 mg (3.80 mmol) Piperazin zu 45 mg (49 % d. Th.) der Titelverbindung umgesetzt.
HPLC (Methode 12): Rₜ = 3.15 min.
MS (ESIpos): m/z = 302 [M+H]⁺
¹H-NMR (DMSO-d₆, 200 MHz): δ = 2.75 (t, 4H), 3.76-3.82 (m, 6H, s bei 3.80), 7.06 (d, 1H), 7.34 (s, 1H), 7.49 (m, 1H).

### Beispiel 36

### 4-(Benzylamino)-6-oxo-2-(3-thienylmethyl)-1,6-dihydropyrimidin-5-carbonitril

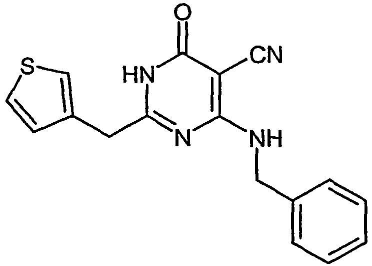

Analog zur Darstellung von Beispiel 1 werden 100 mg (0.38 mmol) 4-(Methylsulfanyl)-6-oxo-2-(3-thienylmethyl)-1,6-dihydropyrimidin-5-carbonitril mit 407 mg (3.80 mmol) Benzylamin zu 43 mg (35 % d. Th.) der Titelverbindung umgesetzt.
HPLC (Methode 12): Rₜ = 4.3 min.
MS (ESIpos): m/z = 323 [M+H]⁺
¹H-NMR (DMSO-d₆, 200 MHz): δ = 3.80 (s, 2H), 4.52 (d, 2H), 6.95 (d, 1H), 7.16-7.32 (m, 6H), 7.45 (m, 1H), 8.49 (t, 1H), 12.40 (s, 1H).

### Beispiel 37

### 4-[(2-Methoxybenzyl)amino]-6-oxo-2-(3-thienylmethyl)-1,6-dihydropyrimidin-5-carbonitril

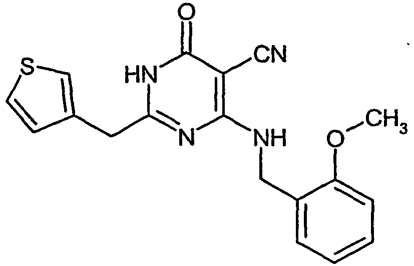

Analog zur Darstellung von Beispiel 1 werden 100 mg (0.38 mmol) 4-(Methylsulfanyl)-6-oxo-2-(3-thienylmethyl)-1,6-dihydropyrimidin-5-carbonitril mit 521 mg (3.80 mmol) 2-Methoxybenzylamin zu 62 mg (46 % d. Th.) der Titelverbindung umgesetzt.
HPLC (Methode 12): Rₜ = 4.4 min.
MS (ESIpos): m/z = 353 [M+H]⁺
¹H-NMR (DMSO-d₆, 300 MHz): δ = 3.75 (s, 2H), 3.80 (s, 3H), 4.56 (d, 2H), 6.84-6.90 (m, 2H), 6.96-7.04 (m, 2H), 7.16 (s, 1H), 7.23 (t, 1H), 7.39 (m, 1H), 8.15 (t, 1H), 12.34 (s, 1H).

### Beispiel 38

### 4-[4-(2-Hydroxyethyl)piperidin-1-yl]-2-[2-(6-methoxypyridin-3-yl)benzyl]-6-oxo-1,6-dihydro-pyrimidin-5-carbonitril

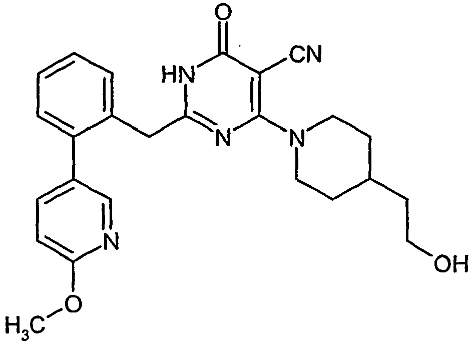

Analog zur Darstellung von Beispiel 1 werden 80 mg (0.22 mmol) 2-[2-(6-Methoxypyridin-3-yl)-benzyl]-4-(methylsulfanyl)-6-oxo-1,6-dihydropyrimidin-5-carbonitril mit 85 mg (0.65 mmol) 2-(4-Piperidinyl)ethan-1-ol zu 62 mg (63 % d. Th.) der Titelverbindung umgesetzt.

Die nachfolgende Verbindung wird nach dem in Schema II dargestellten allgemeinen Syntheseweg hergestellt: 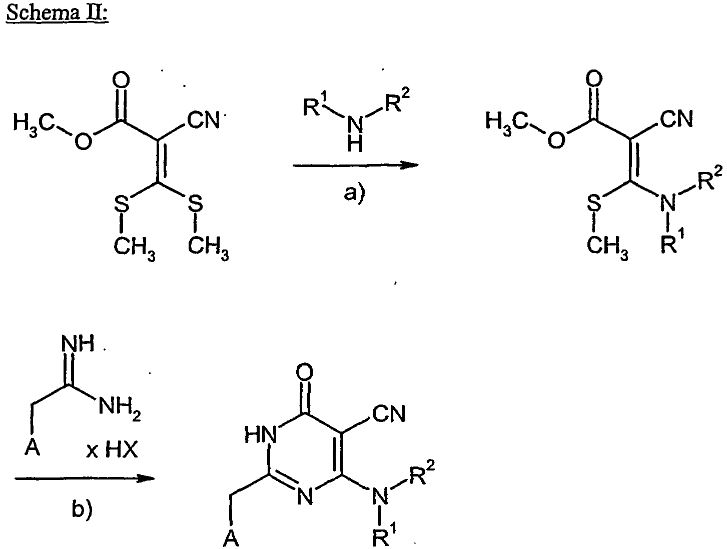 X = Cl, Br
a) 1. Toluol, Bortrifluorid-Etherat, RT, 30 min.; 2. Amin-Komponente R¹R²NH, 150°C, 16 h; oder: Schmelze der Ausgangsverbindungen bei 150°C, 1-16 h; b) DMF, Triethylamin, 100°C, 16 h oder DMF, Kaliumcarbonat, 90°C, 16 h.

### Beispiel 39

### 4-(Cyclohexylamino)-6-oxo-2-(3-thienylmethyl)-1,6-dihydropyrimidin-5-carbonitril

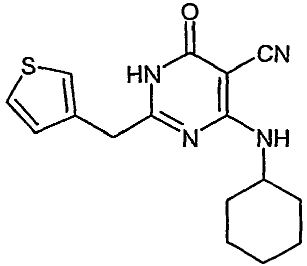

69.5 mg (0.39 mmol) 2-(3-Thienyl)ethanamidin-Hydrochlorid werden mit 100 mg (0.39 mmol) Methyl (2E/Z)-2-cyano-3-(cyclohexylamino)-3-methylthio-prop-2-enoat und 159 mg (1.57 mmol) Triethylamin in 0.5 ml DMF gelöst und über Nacht bei 100°C gerührt. Der abgekühlte Ansatz wird in wenig Wasser aufgenommen und mit Dichlormethan extrahiert. Die Dichlormethan-Phase wird über Natriumsulfat getrocknet, eingeengt und der Rückstand an Kieselgel flash-chromatographiert (Laufmittel: Dichlormethan, dann Dichlormethan/ Methanol 200:1, 100:1). Es werden 23 mg (19 % d. Th.) des Produkts erhalten.
HPLC (Methode 12): Rₜ = 4.55 min.
MS (DCI, NH₃): m/z = 315 [M+H]⁺
¹H-NMR (DMSO-d₆, 400 MHz): δ = 0.98-1.43 (m, 5H), 1.53-1.74 (m, 5H), 3.78-3.94 (m, 3H, s bei 3.82), 7.06 (d, 1H), 7.33 (s, 1H), 7.50 (m, 1H), 7.54 (m, 1H), 12.28 (s, 1H).

### Beispiel 40

### 4-(4-Formylpiperazin-1-yl)-6-oxo-2-(3-thienylmethyl)-1,6-dihydropyrimidin-5-carbonitril

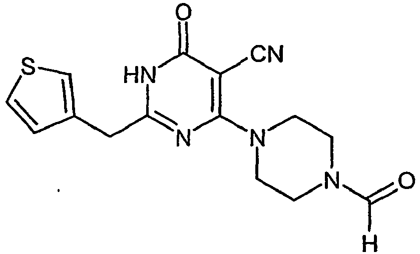

Unter einer Argonatmosphäre werden 11.3 mg (0.17 mmol) Imidazol mit 33.6 mg (0.33 mmol) Triethylamin und 7.64 mg (0.17 mmol) Ameisensäure in 5 ml Dichlormethan vorgelegt und auf 0°C gekühlt. Dann wird eine Lösung von 21.1 mg (0.17 mmol) Oxalylchlorid in Dichlormethan zugetropft und die Mischung im Anschluss 15 min lang gerührt. Es werden 50 mg (0.17 mmol) 6-Oxo-4-(piperazin-1-yl)-2-(3-thienylmethyl)-1,6-dihydropyrimidin-5-carbonitril zugefügt und der Ansatz über Nacht bei RT gerührt. Dann wird mit 1 N Kaliumhydrogensulfat-Lösung gewaschen, die Dichlormethan-Phase über Natriumsulfat getrocknet, eingeengt und der Rückstand an Kieselgel flash-chromatographiert (Laufmittel: Dichlormethan/Methanol 100:1, 80:1, 60:1). Man erhält 16 mg (29 % d. Th.) der Titelverbindung.
HPLC (Methode 12): Rₜ = 3.4 min.
MS (ESIpos): m/z = 330 [M+H]⁺
¹H-NMR (DMSO-d₆, 200 MHz): δ = 3.42-3.54 (m, 4H), 3.79-3.94 (m, 6H, s bei 3.82), 7.09 (d, 1H), 7.36 (s, 1H), 7.51 (m, 1H), 8.06 (s, 1H), 12.55 (s, 1H).

### Beispiel 41

### 4-(4-Acetylpiperazin-1-yl)-6-oxo-2-(3-thienylmethyl)-1,6-dihydropyrimidin-5-carbonitril

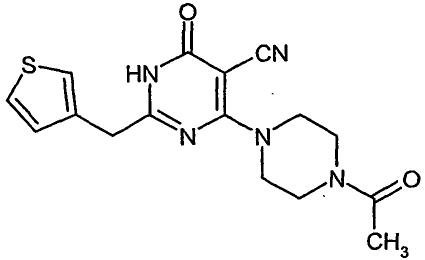

50 mg (0.17 mmol) 6-Oxo-4-(piperazin-1-yl)-2-(3-thienylmethyl)-1,6-dihydropyrimidin-5-carbo-nitril werden mit 34 mg (0.33 mmol) Triethylamin in DMF gelöst und mit 14.3 mg (0.18 mmol) Acetylchlorid über Nacht bei RT gerührt. Anschließend wird der Ansatz mit Dichlormethan verdünnt und mit Wasser gewaschen. Die organische Phase wird abgetrennt, über Natriumsulfat getrocknet und der Rückstand an Kieselgel flash-chromatographiert (Laufmittel: Dichlormethan/ Methanol 100:1, 80:1, 60:1). Man erhält 42 mg (74% d. Th.) der Titelverbindung.
HPLC (Methode 12): Rₜ = 3.5 min.
MS (ESIpos): m/z = 344 [M+H]⁺
¹H-NMR (DMSO-d₆, 300 MHz): δ = 2.02 (s, 3H), 3.50-3.61 (m, 4H), 3.80-3.95 (m, 6H, s bei 3.82), 7.08 (d, 1H), 7.36 (s, 1H), 7.50 (m, 1H), 12.47 (s, 1H).

### Beispiel 42

### 4-(4-Ethylpiperazin-1-yl)-6-oxo-2-(3-thienylmethyl)-1,6-dihydropyrimidin-5-carbonitril

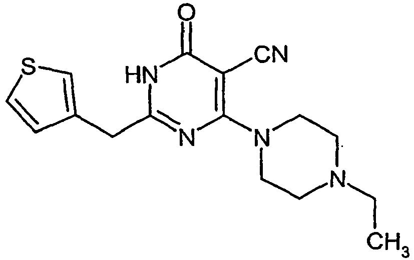

50 mg (0.17 mmol) 6-Oxo-4-(piperazin-1-yl)-2-(3-thienylmethyl)-1,6-dihydropyrimidin-5-carbonitril werden mit 34 mg (0.33 mmol) Triethylamin in DMF gelöst, mit 19.9 mg (0.18 mmol) Bromethan versetzt und über Nacht bei RT gerührt. Dann wird der Ansatz mit Dichlormethan verdünnt und mit Wasser gewaschen. Die organische Phase wird abgetrennt, über Natriumsulfat getrocknet und der Rückstand an Kieselgel flash-chromatographiert (Laufmittel: Dichlormethan/ Methanol 100:1, 80:1, 60:1, 40:1). Man erhält 38 mg (70% d. Th.) der Titelverbindung.
HPLC (Methode 12): Rₜ = 3.3 min.
MS (ESIpos): m/z = 330 [M+H]⁺
¹H-NMR (DMSO-d₆, 300 MHz): δ = 1.00 (t, 3H), 2.34 (q, 2H), 2.42 (t, 4H), 3.80 (s, 2H), 3.86 (t, 4H), 7.06 (d, 1H), 7.34 (s, 1H), 7.49 (m, 1H), 12.39 (s, 1H).

Die in der folgenden Tabelle aufgeführten Ausführungsbeispiele werden in Analogie zu den zuvor beschriebenen Beispielen hergestellt:

| **Beispiel-Nr.** | **Struktur** | **LC-MS: m/z [M+H]⁺** | **Rₜ [min]** | **HPLC- / LC-MS-Methode** |
|---|---|---|---|---|
| **43** | 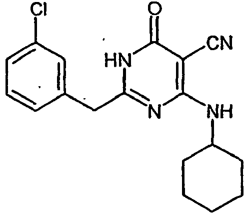 | 344 | 4.8 | 12 |
| **44** | 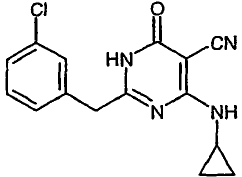 | 302 | 4.2 | 12 |
| **45** | 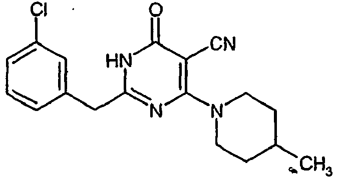 | 344 | 4.8 | 12 |
| **46** | 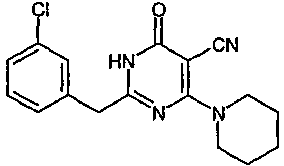 | 330 | 4.6 | 12 |
| **47** | 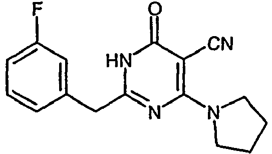 | 299 | 1.99 | 10 |
| **48** | 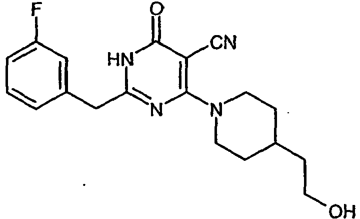 | 357 | 1.83 | 6 |
| **49** | 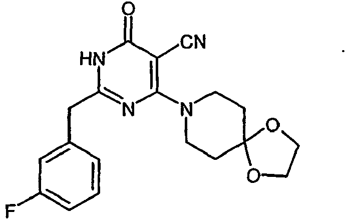 | 371 | 3.17 | 7 |
| **50** | 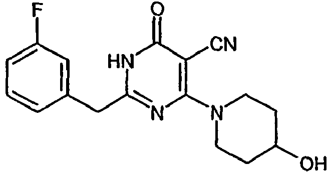 | 329 | 1.45 | 5 |
| **51** | 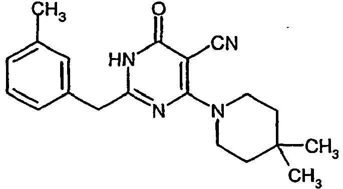 | 337 | 3.19 | 2 |
| **52** | 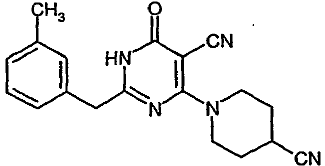 | 334 | 2.54 | 7 |
| **53** | 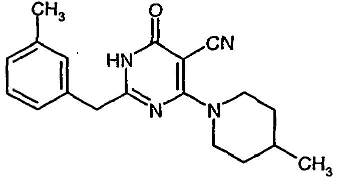 | 323 | 2.36 | 10 |
| **54** | 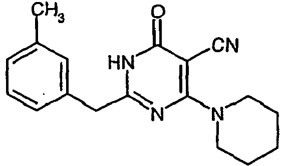 | 309 | 2.87 | 7 |
| **55** | 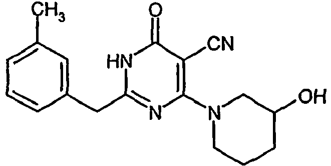 | 325 | 2.38 | 2 |
| **56** | 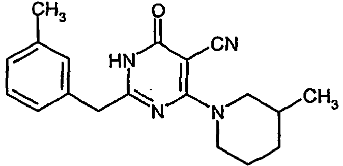 | 323 | 3.06 | 2 |
| **57** | 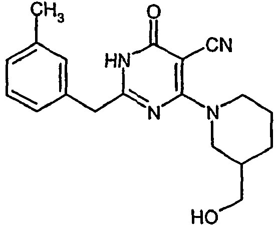 | 339 | 2.99 | 7 |
| **58** | 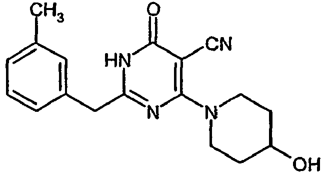 | 325 | 1.74 | 10 |
| **59** | 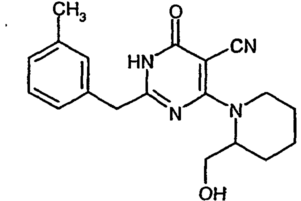 | 339 | 1.79 | 5 |
| **60** | 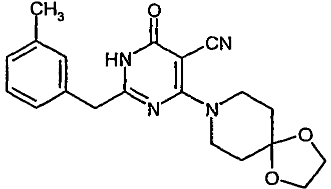 | 367 | 2.7 | 2 |
| **61** | 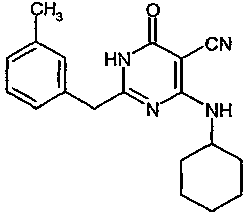 | 323 | 3.1 | 7 |
| **62** | 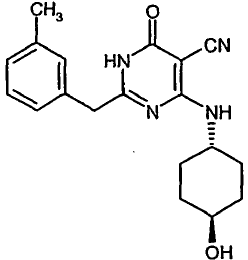 | 339 | 2.19 | 7 |
| **63** | 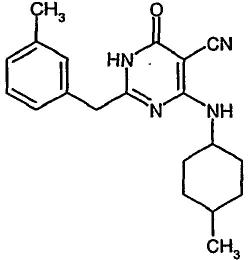 | 337 | 3.3 | 7 |
| **64** | 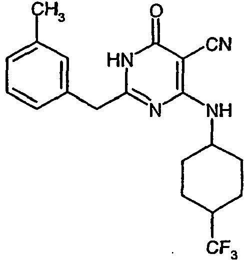 | 391 | 2.6 | 4 |
| **65** | 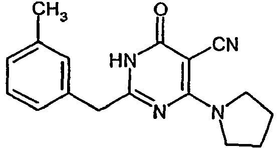 | 295 | 2.75 | 2 |
| **66** | 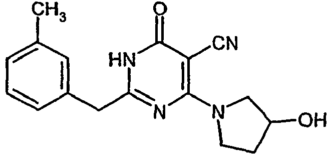 | 311 | 1.73 | 10 |
| **67** | 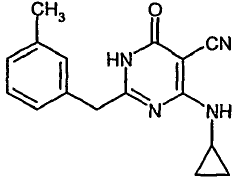 | 281 | 3.18 | 7 |
| **68** | 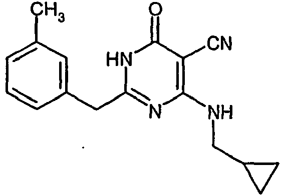 | 295 | 2.73 | 7 |
| **69** | 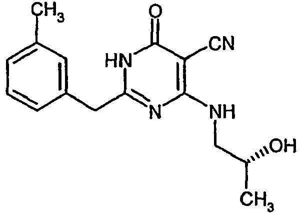 | 299 | 2.22 | 7 |
| **70** | 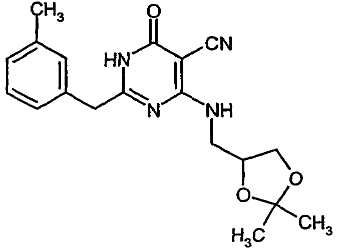 | 355 | 2.63 | 2 |
| **71** | 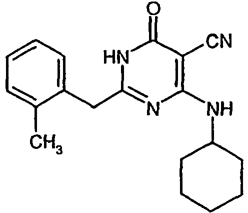 | 323 | 2.37 | 10 |
| **72** | 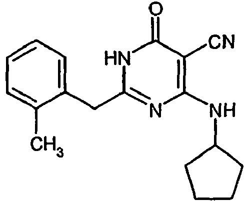 | 309 | 2.27 | 10 |
| **73** | 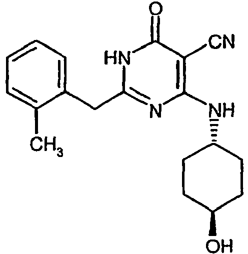 | 339 | 2.19 | 2 |
| **74** | 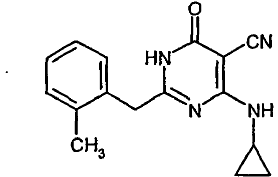 | 281 | 2.02 | 6 |
| **75** | 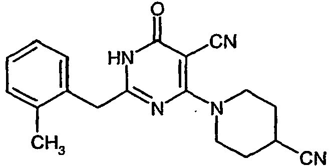 | 334 | 2.47 | 7 |
| **76** | 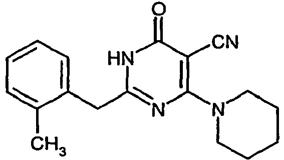 | 309 | 2.19 | 10 |
| **77** | 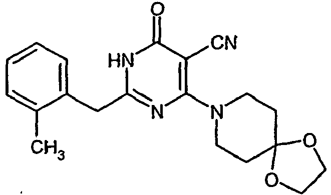 | 367 | 2.81 | 1 |
| **78** | 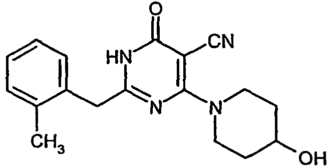 | 325 | 2.35 | 2 |
| **79** | 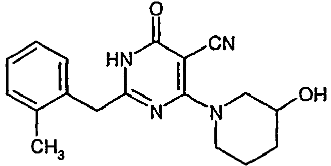 | 325 | 2.35 | 2 |
| **80** | 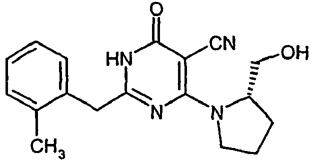 | 325 | 1.76 | 4 |
| **81** | 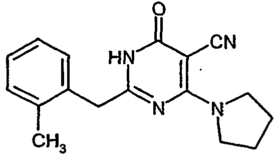 | 295 | 3.24 | 7 |
| **82** | 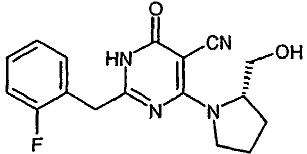 | 329 | 1.69 | 10 |
| **83** | 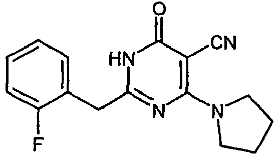 | 299 | 3.19 | 7 |
| **84** | 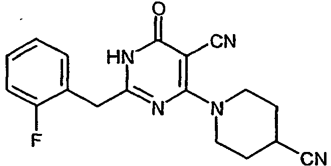 | 338 | 1.88 | 10 |
| **85** | 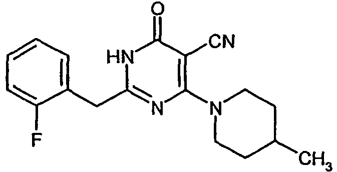 | 327 | 3.5 | 2 |
| **86** | 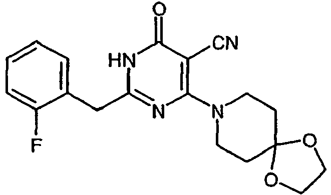 | 371 | 1.95 | 10 |
| **87** | 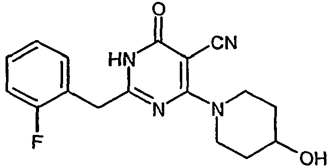 | 329 | 1.63 | 10 |
| **88** | 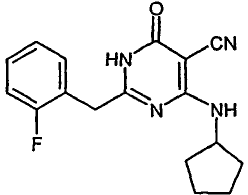 | 313 | 3.4 | 2 |
| **89** | 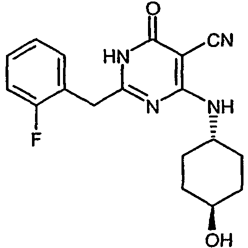 | 343 | 2.71 | 7 |
| **90** | 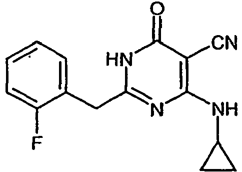 | 285 | 1.84 | 10 |
| **91** | 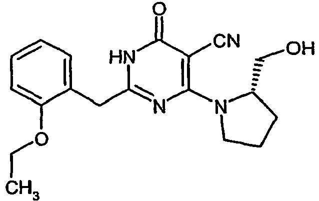 | 355 | 1.97 | 6 |
| **92** | 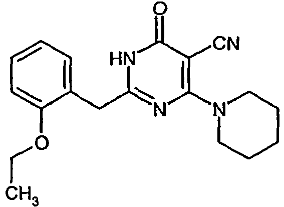 | 339 | 2.19 | 6 |
| **93** | 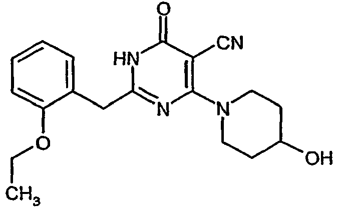 | 255 | 1.61 | 6 |
| **94** | 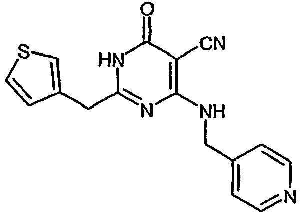 | 324 | 3.2 | 12 |
| **95** | 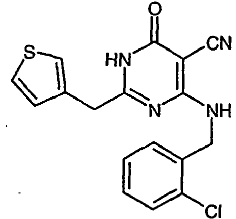 | 358 | 4.5 | 12 |
| **96** | 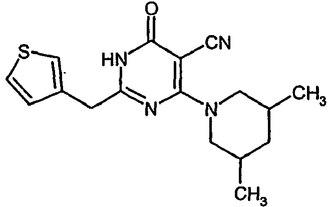 | 329 | 4.7 | 12 |
| **97** | 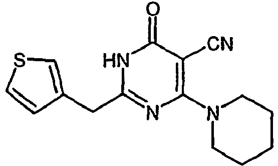 | 301 | 4.3 | 12 |
| **98** | 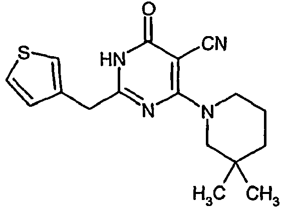 | 329 | 4.6 | 12 |
| **99** | 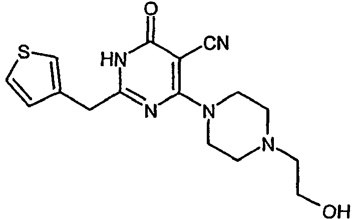 | 346 | 3.2 | 12 |
| **100** | 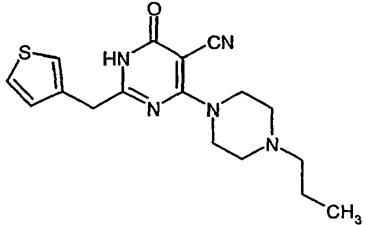 | 344 | 3.4 | 12 |
| **101** | 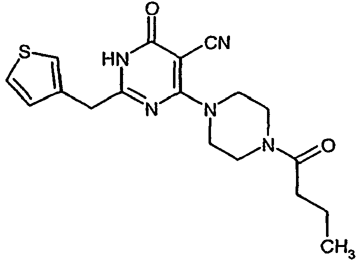 | 372 | 3.9 | 12 |
| **102** | 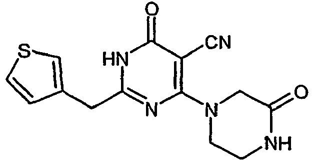 | 316 | 4.3 | 13 |
| **103** | 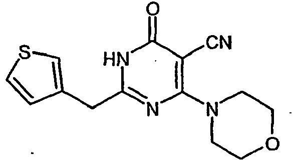 | 303 | 3.8 | 12 |
| **104** | 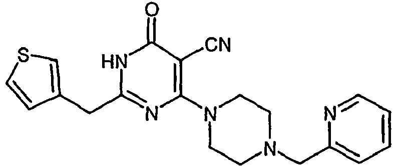 | 393 | 3.5 | 12 |
| **105** | 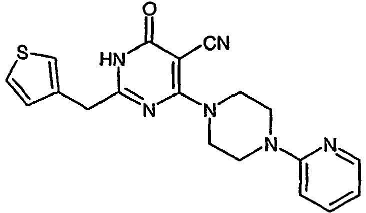 | 379 | 3.5 | 12 |
| **106** | 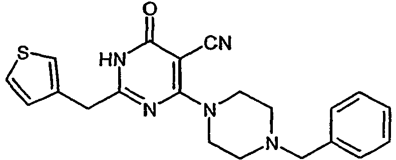 | 392 | 3.8 | 12 |
| **107** | 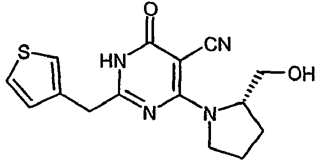 | 317 | 1.46 | 5 |
| **108** | 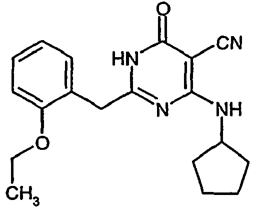 | 339 | 2.42 | 6 |
| **109** | 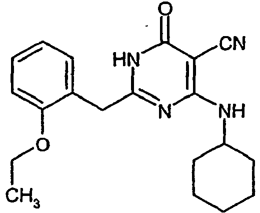 | 353 | 2.54 | 6 |
| **110** | 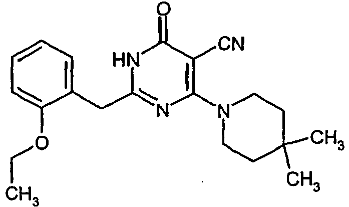 | 367 | 2.63 | 6 |
| **111** | 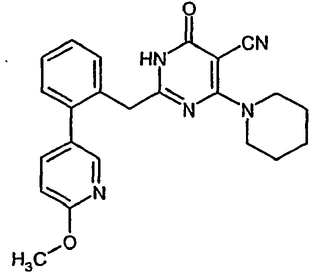 | 402 | 2.15 | 5 |
| **112** | 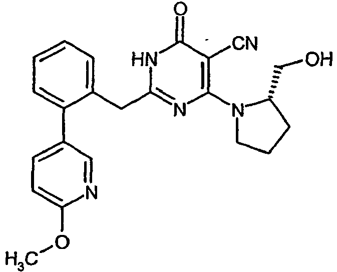 | 418 | 1.92 | 6 |
| **113** | 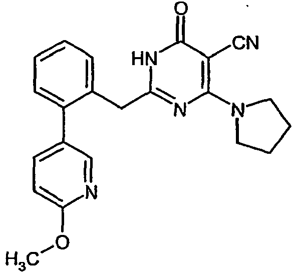 | 388 | 2.02 | 5 |
| **114** | 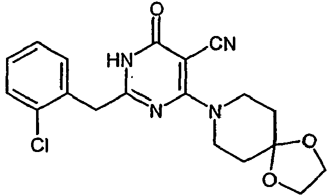 | 387 | 1.9 | 5 |
| **115** | 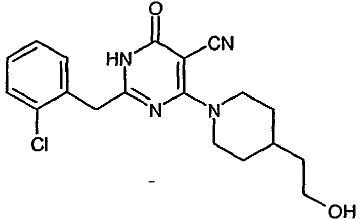 | 373 | 1.94 | 6 |
| **116** | 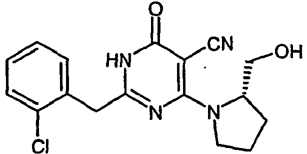 | 345 | 1.84 | 6 |
| **117** | 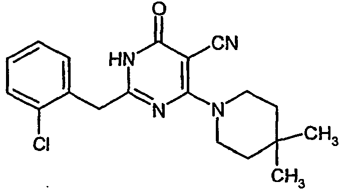 | 357 | 2.61 | 6 |
| **118** | 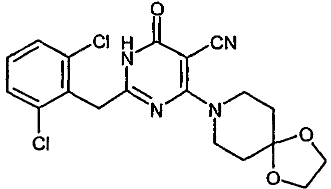 | 421 | 1.99 | 5 |
| **119** | 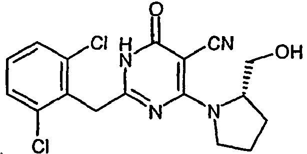 | 379 | 1.89 | 6 |
| **120** | 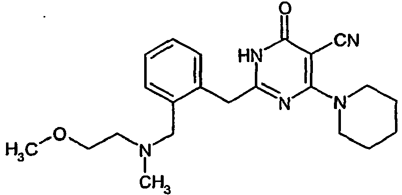 | 396 | 1.18 | 5 |
| **121** | 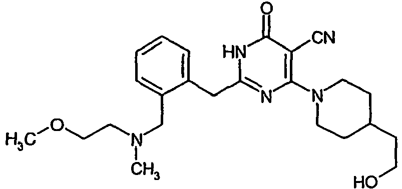 | 440 | 1.03 | 5 |
| **122** | 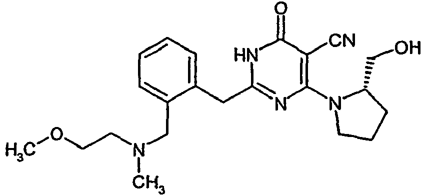 | 412 | 0.92 | 5 |
| **123** | 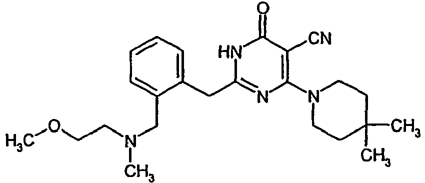 | 424 | 1.46 | 5 |
| **124** | 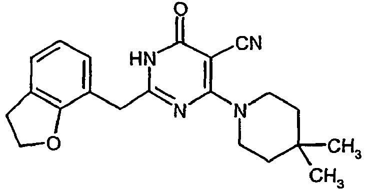 | 365 | 2.31 | 5 |
| **125** | 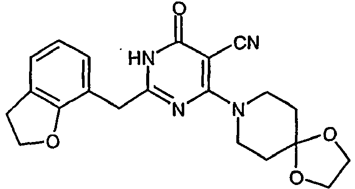 | 395 | 1.85 | 5 |
| **126** | 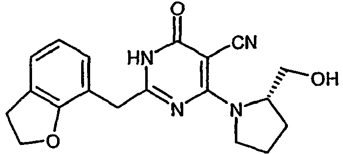 | 353 | 1.54 | 5 |
| **127** | 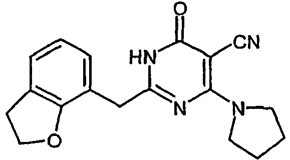 | 323 | 1.89 | 5 |
| **128** | 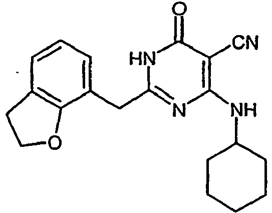 | 351 | 2.2 | 5 |
| **129** | 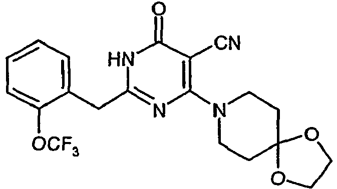 | 437 | 2.28 | 6 |
| **130** | 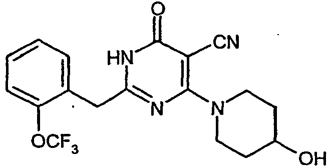 | 395 | 1.86 | 4 |
| **131** | 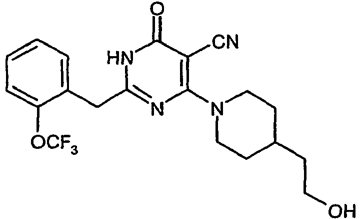 | 423 | 2.01 | 4 |
| **132** | 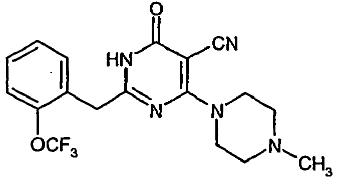 | 393 | 1.3 | 4 |
| **133** | 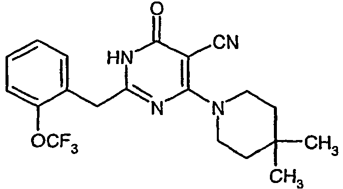 | 407 | 2.5 | 5 |
| **134** | 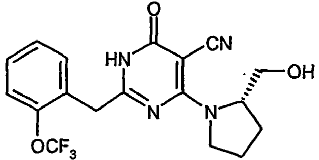 | 395 | 1.74 | 5 |
| **135** | 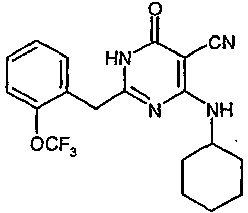 | 393 | 2.63 | 6 |
| **136** | 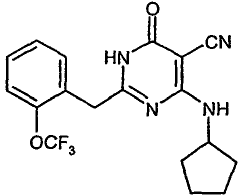 | 379 | 2.31 | 5 |
| **137** | 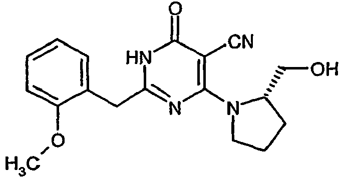 | 341 | 1.54 | 5 |
| **138** | 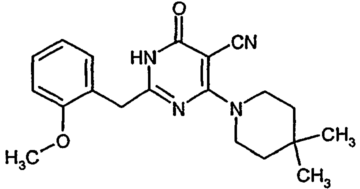 | 353 | 2.5 | 4 |
| **139** | 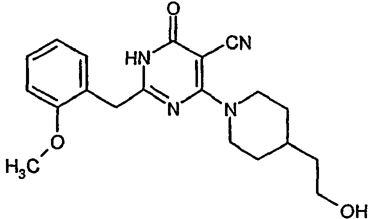 | 369 | 1.84 | 4 |
| **140** | 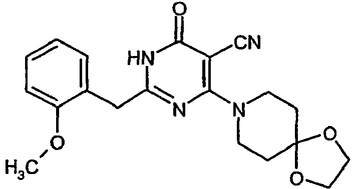 | 383 | 2.1 | 6 |
| **141** | 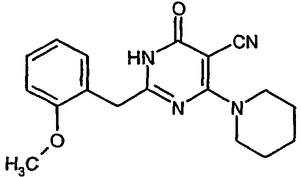 | 325 | 2.04 | 5 |
| **142** | 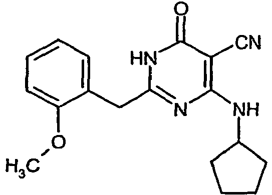 | 325 | 2.28 | 4 |
| **143** | 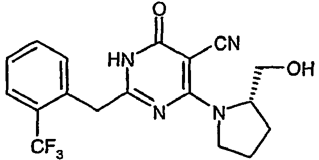 | 379 | 1.85 | 4 |
| **144** | 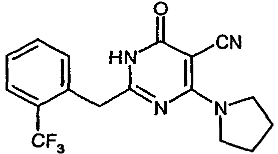 | 349 | 2.07 | 5 |
| **145** | 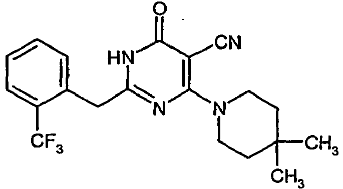 | 391 | 2.44 | 5 |
| **146** | 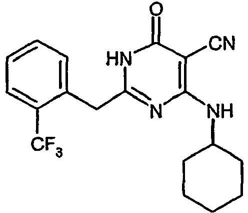 | 377 | 2.48 | 4 |
| **147** | 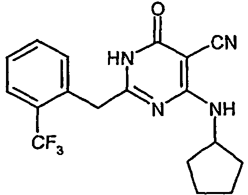 | 363 | 2.45 | 6 |
| **148** | 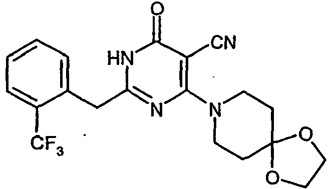 | 421 | 2.15 | 4 |
| **149** | 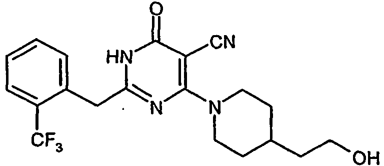 | 407 | 1.94 | 4 |
| **150** | 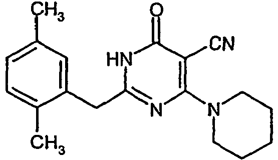 | 323 | 2.47 | 6 |
| **151** | 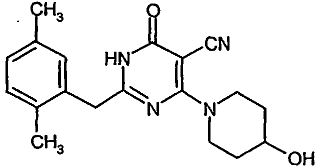 | 339 | 1.67 | 5 |
| **152** | 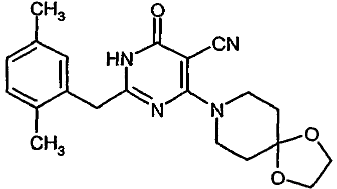 | 381 | 2.07 | 5 |
| **153** | 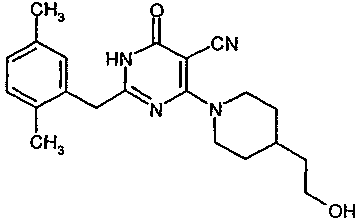 | 367 | 1.99 | 4 |
| **154** | 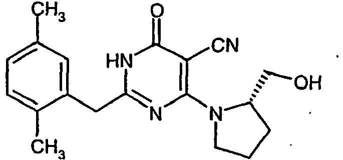 | 229 | 1.98 | 6 |
| **155** | 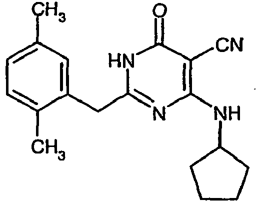 | 323 | 2.53 | 6 |
| **156** | 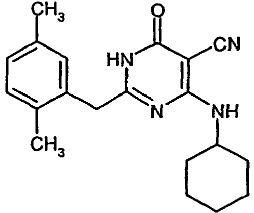 | 337 | 2.65 | 6 |
| **157** | 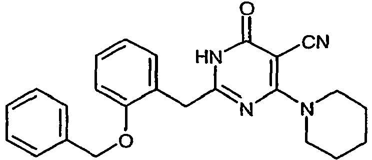 | 401 | 2.59 | 6 |
| **158** | 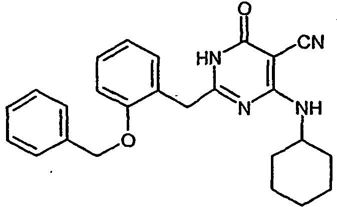 | 415 | 2.75 | 6 |
| **159** | 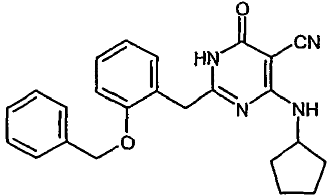 | 401 | 2.43 | 5 |
| **160** | 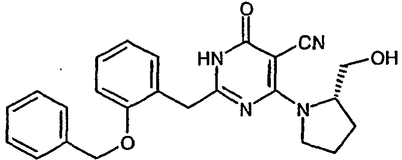 | 417 | 1.9 | 5 |
| **161** | 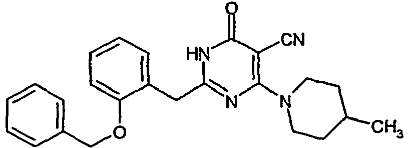 | 415 | 2.72 | 6 |
| **162** | 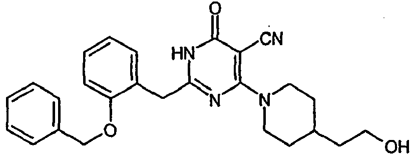 | 445 | 1.97 | 5 |
| **163** | 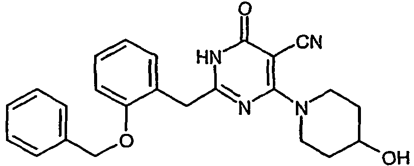 | 417 | 1.82 | 5 |
| **164** | 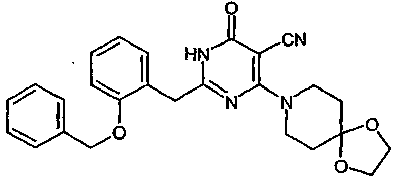 | 459 | 2.2 | 5 |

## Patentansprüche

1. Verfahren zur Herstellung von Verbindungen nach der Formel 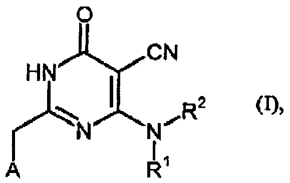 in welcher
A Phenyl, Heteroaryl, das einen aromatischen, monocyclischen Rest mit 5 bis 6 Ring-atomen und bis zu 3 Heteroatomen aus der Reihe S, O und/oder N darstellt, oder eine Gruppe der Formel 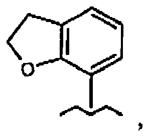 wobei Phenyl und Heteroaryl, gegebenenfalls mit bis zu 2 Resten unabhängig voneinander ausgewählt aus der Gruppe Heteroaryl, das einen aromatischen, monocyclischen Rest mit 5 bis 6 Ringatomen und bis zu 3 Heteroatomen aus der Reihe S, O und/oder N darstellt, Halogen, C₁-C₆-Alkyl, C₁-C₆-Alkoxy, Trifluormethyl, Trifluormethoxy, Benzyloxy und Benzyl substituiert sind,
wobei C₁-C₆-Alkyl gegebenenfalls mit einer Gruppe der Formel -NR³R⁴, in welcher R³ C₁-C₆-Alkyl und R⁴ Wasserstoff oder C₁-C₆-Alkoxy(C₁-C₆)alkyl bedeuten, und
Heteroaryl gegebenenfalls mit C₁-C₆-Alkoxy substituiert ist,
R¹ C₃-C₈-Cycloalkyl, C₁-C₆-Alkyl, C₁-C₆-Alkoxy(C₁-C₆)alkyl, Benzyl oder eine Gruppe der Formel 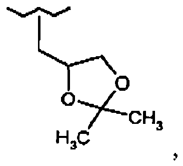 wobei C₃-C₈-Cycloalkyl gegebenenfalls mit Hydroxy, C₁-C₆-Alkyl oder Trifluormethyl,
C₁-C₆-Alkyl gegebenenfalls mit Heteroaryl, das einen aromatischen, monocyclischen Rest mit 5 bis 6 Ringatomen und bis zu 3 Heteroatomen aus der Reihe S, O und/oder N darstellt, C₃-C₈-Cycloalkyl oder Hydroxy,
und Benzyl gegebenenfalls mit C₁-C₆-Alkoxy oder Halogen substituiert ist,
R² Wasserstoff,
oder
R¹ und R² zusammen mit dem Stickstoffatom, an dem sie gebunden sind, ein 5- bis 6-gliedriges Heterocyclyl bilden, wobei besagtes 5- bis 6-gliedriges Heterocyclyl ein monocyclischer, gesättigter oder partiell ungesättigter heterocyclischer Rest mit 5 bis 6 Ringatomen und bis zu 2 Heteroatomen aus der Reihe N, O, S ist, welcher gegebenenfalls mit bis zu 2 Substituenten unabhängig voneinander ausgewählt aus der Gruppe C₁-C₆-Alkyl, Hydroxy, Cyano, Oxo, Heteroaryl, das einen aromatischen, monocyclischen Rest mit 5 bis 6 Ringatomen und bis zu 3 Heteroatomen aus der Reihe S, O und/oder N darstellt, Benzyl, Formyl, C₁-C₆-Alkylcarbonyl und einer der folgenden Gruppen 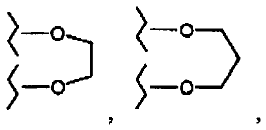 die über die beiden Sauerstoffatome an eines der Kohlenstoffatome im Heterocyclus gebunden sind, substituiert ist,
wobei C₁-C₆-Alkyl gegebenenfalls mit Hydroxy oder Heteroaryl, das einen aromatischen, monocyclischen Rest mit 5 bis 6 Ringatomen und bis zu 3 Heteroatomen aus der Reihe S, O und/oder N darstellt, substituiert ist,
bedeuten, sowie deren Salze, Solvate und/oder Solvate der Salze, **dadurch gekennzeichnet, dass** man entweder
[A] eine Verbindung der Formel 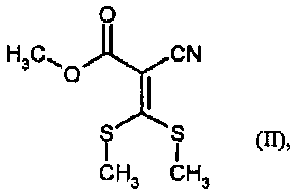 zunächst mit einer Verbindung der Formel
HNR¹R² (III),
in welcher
R¹ und R² die oben angegebenen Bedeutungen aufweisen,
bei erhöhter Temperatur in einem inerten Lösemittel oder auch in Abwesenheit eines Lösemittels in eine Verbindung der Formel 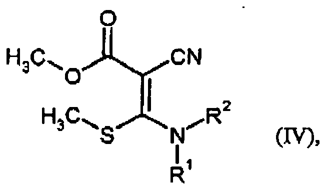 in welcher
R¹ und R² die oben angegebenen Bedeutungen aufweisen,
überführt und diese dann in einem inerten Lösemittel in Gegenwart einer Base mit einer Verbindung der Formel 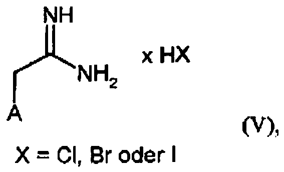 in welcher
A die oben angegebenen Bedeutungen aufweist,
umsetzt
oder in veränderter Reihenfolge der Reaktionspartner
[B] eine Verbindung der Formel (II) zunächst mit einer Verbindung der Formel (V) in einem inerten Lösemittel in Gegenwart einer Base in eine Verbindung der Formel 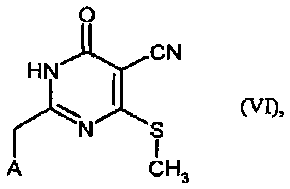 in welcher
A die oben angegebenen Bedeutungen aufweist,
überführt und diese dann bei erhöhter Temperatur in einem inerten Lösemittel oder auch in Abwesenheit eines Lösemittels mit einer Verbindung der Formel (III) umsetzt,
und die jeweils resultierenden Verbindungen der Formel (I) gegebenenfalls mit den entsprechenden (i) Lösemitteln und/oder (ii) Basen oder Säuren zu ihren Solvaten, Salzen und/oder Solvaten der Salze umsetzt.

2. Verfahren nach Anspruch 1, wobei
A Phenyl, Heteroaryl, das einen aromatischen, monocyclischen Rest mit 5 bis 6 Ring-atomen und bis zu 3 Heteroatomen aus der Reihe S, O und/oder N darstellt, oder eine Gruppe der Formel 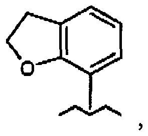 wobei Phenyl und Heteroaryl gegebenenfalls mit bis zu 2 Resten unabhängig voneinander ausgewählt aus der Gruppe Heteroaryl, das einen aromatischen, monocyclischen Rest mit 5 bis 6 Ringatomen und bis zu 3 Heteroatomen aus der Reihe S, O und/oder N darstellt, Halogen, C₁-C₄-Alkyl, C₁-C₄-Alkoxy, Trifluormethyl, Trifluormethoxy, Benzyloxy und Benzyl substituiert sind,
wobei C₁-C₄-Alkyl gegebenenfalls mit einer Gruppe der FormelNR³R⁴, in welcher R³ C₁-C₄-Alkyl und R⁴ Wasserstoff oder C₁-C₄-Alkoxy(C₁-C₄)alkyl bedeuten, und
Heteroaryl gegebenenfalls mit C₁-C₄-Alkoxy substituiert ist,
R¹ C₃-C₆-Cycloalkyl, C₁-C₄-Alkyl, C₁-C₄-Alkoxy(C₁-C₄)alkyl, Benzyl oder eine Gruppe der Formel 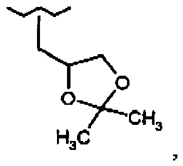 wobei C₃-C₆-Cycloalkyl gegebenenfalls mit Hydroxy, C₁-C₄-Alkyl oder Trifluormethyl,
C₁-C₄-Alkyl gegebenenfalls mit Heteroaryl, das einen aromatischen, monocyclischen Rest mit 5 bis 6 Ringatomen und bis zu 3 Heteroatomen aus der Reihe S, O und/oder N darstellt, C₃-C₆-Cycloalkyl oder Hydroxy,
und Benzyl gegebenenfalls mit C₁-C₄-Alkoxy oder Halogen substituiert ist,
R² Wasserstoff,
oder
R¹ und R² zusammen mit dem Stickstoffatom, an dem sie gebunden sind, ein 5- bis 6-gliedriges Heterocyclyl bilden, wobei besagtes 5- bis 6-gliedriges Heterocyclyl ein monocyclischer, gesättigter oder partiell ungesättigter heterocyclischer Rest mit 5 bis 6 Ringatomen und bis zu 2 Heteroatomen aus der Reihe N, O, S ist, welcher gegebenenfalls mit bis zu 2 Substituenten unabhängig voneinander ausgewählt aus der Gruppe C₁-C₄-Alkyl, Hydroxy, Cyano, Oxo, Heteroaryl, das einen aromatischen, monocyclischen Rest mit 5 bis 6 Ringatomen und bis zu 3 Heteroatomen aus der Reihe S, O und/oder N darstellt, Benzyl, Formyl, C₁-C₄-Alkylcarbonyl und einer der folgenden Gruppen 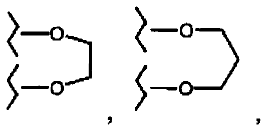 die über die beiden Sauerstoffatome an eines der Kohlenstoffatome im Heterocyclus gebunden sind, substituiert ist,
wobei C₁-C₄-Alkyl gegebenenfalls mit Hydroxy oder Heteroaryl, das einen aromatischen, monocyclischen Rest mit 5 bis 6 Ringatomen und bis zu 3 Heteroatomen aus der Reihe S, O und/oder N darstellt, substituiert ist,
bedeuten, sowie deren Salze, Solvate und/oder Solvate der Salze,

3. Verfahren nach Ansprüchen 1 und 2, wobei
A Phenyl, Thienyl oder eine Gruppe der Formel 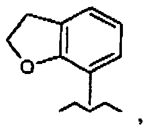 wobei Phenyl und Thienyl gegebenenfalls mit bis zu 2 Resten unabhängig voneinander ausgewählt aus der Gruppe Pyridyl, Fluor, Chlor, Brom, C₁-C₄-Alkyl, C₁-C₄-Alkoxy, Trifluormethyl, Trifluormethoxy, Benzyloxy und Benzyl substituiert sind,
wobei C₁-C₄-Alkyl gegebenenfalls mit einer Gruppe der Formel - NR³R⁴, in welcher R³ C₁-C₄-Alkyl und R⁴ Wasserstoff oder C₁-C₄-Alkoxy(C₁-C₄)alkyl bedeuten, und
Pyridyl gegebenenfalls mit C₁-C₄-Alkoxy substituiert ist,
R¹ C₃-C₆-Cycloalkyl, C₁-C₄-Alkyl, C₁-C₄-Alkoxy(C₁-C₄)alkyl, Benzyl oder eine Gruppe der Formel 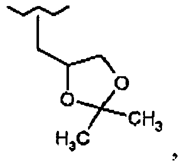 wobei C₃-C₆-Cycloalkyl gegebenenfalls mit Hydroxy, C₁-C₄-Alkyl oder Trifluormethyl,
C₁-C₄-Alkyl gegebenenfalls mit Pyridyl, C₃-C₆-Cycloakyl oder Hydroxy, und Benzyl gegebenenfalls mit C₁-C₄-Alkoxy, Fluor, Chlor oder Brom substituiert ist,
R² Wasserstoff,
oder
R¹ und R² zusammen mit dem Stickstoffatom, an dem sie gebunden sind, ein 5- bis 6-gliedriges Heterocyclyl ausgewählt aus der Gruppe Pyrrolidinyl, Piperidinyl, Piperazinyl und Morpholinyl bilden, welches gegebenenfalls mit bis zu 2 Sub-stituenten unabhängig voneinander ausgewählt aus der Gruppe C₁-C₄-Alkyl, Hydroxy, Cyano, Oxo, Heteroaryl, das einen aromatischen, monocyclischen Rest mit 5 bis 6 Ringatomen und bis zu 3 Heteroatomen aus der Reihe S, O und/oder N darstellt, Benzyl, Formyl, C₁-C₄-Alkylcarbonyl und einer der folgenden Gruppen 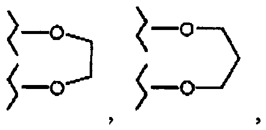 die über die beiden Sauerstoffatome an eines der Kohlenstoffatome im Heterocyclus gebunden sind, substituiert ist,
wobei C₁-C₄-Alkyl gegebenenfalls mit Hydroxy oder Pyridyl substituiert ist,
bedeuten, sowie deren Salze, Solvate und/oder Solvate der Salze.

4. Verfahren nach Ansprüchen 1, 2 und 3, wobei
A Phenyl, Thienyl oder eine Gruppe der Formel 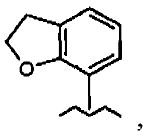 wobei Phenyl gegebenenfalls mit bis zu 2 Resten unabhängig voneinander ausgewählt aus der Gruppe Pyridyl, Fluor, Chlor, Methyl, Methoxy, Ethoxy, Trifluormethyl, Trifluormethoxy, Benzyloxy und Benzyl substituiert ist,
wobei Methyl gegebenenfalls mit einer Gruppe der Formel -NR³R⁴, in welcher R³ Methyl und R⁴ Wasserstoff oder 2-Methoxyethyl bedeuten, und
Pyridyl gegebenenfalls mit Methoxy substituiert ist,
R¹ C₃-C₆-Cycloalkyl, Methyl, Ethyl, Propyl, 2-Methoxyethyl, Benzyl oder eine Gruppe der Formel 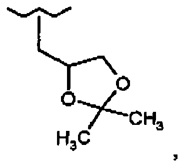 wobei C₃-C₆-Cycloalkyl gegebenenfalls mit Hydroxy, Methyl oder Trifluor-methyl,
Methyl, Ethyl, Propyl gegebenenfalls mit Pyridyl, Cyclopropyl oder Hydroxy,
und Benzyl gegebenenfalls mit Methoxy, Ethoxy, Fluor oder Chlor substituiert ist,
R² Wasserstoff,
oder
R¹ und R² zusammen mit dem Stickstoffatom, an dem sie gebunden sind, ein 5- bis 6-gliedriges Heterocyclyl ausgewählt aus der Gruppe Pyrrolidinyl, Piperidinyl, Piperazinyl und Morpholinyl bilden, welches gegebenenfalls mit bis zu 2 Substituenten unabhängig voneinander ausgewählt aus der Gruppe Methyl, Ethyl, Propyl, tert.-Butyl, Hydroxy, Cyano, Oxo, Pyridyl, Benzyl, Formyl, Methylcarbonyl, Ethylcarbonyl, Propylcarbonyl und einer der folgenden Gruppen 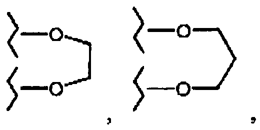 die über die beiden Sauerstoffatome an eines der Kohlenstoffatome im Heterocyclus gebunden sind, substituiert ist,
wobei Methyl, Ethyl und Propyl gegebenenfalls mit Hydroxy oder Pyridyl substituiert sind,
bedeuten, sowie deren Salze, Solvate und/oder Solvate der Salze.

5. Verbindungen nach der Formel 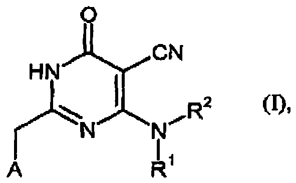 in welcher die Reste A, R¹ und R² wie in den Ansprüchen 1 bis 4 definiert sind sowie deren Salze, Solvate und/oder Solvate der Salze, wobei die nachfolgende Verbindung ausgenommen ist:

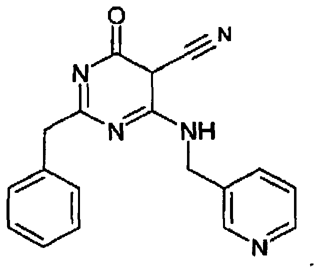

6. Eine Verbindung ausgewählt aus der Gruppe bestehend aus:
| **Verbindung** | **Struktur** | **Verbindung** | **Struktur** |
|---|---|---|---|
| **1** | 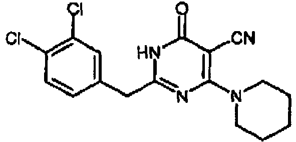 | **2** | 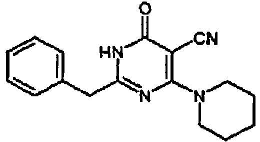 |
| **3** | 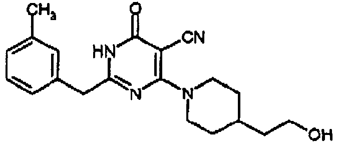 | **4** | 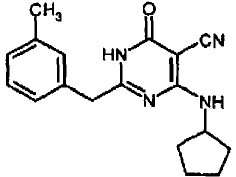 |
| **5** | 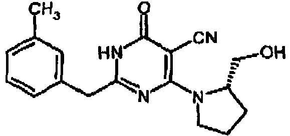 | **6** | 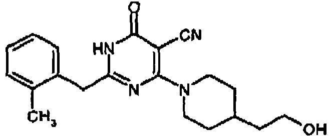 |
| **7** | 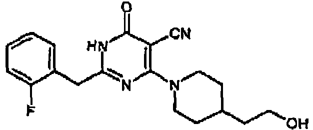 | **8** | 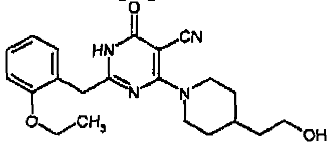 |
| **9** | 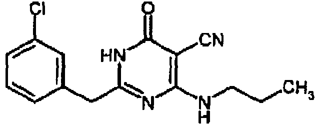 | **10** | 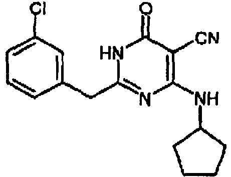 |
| **11** | 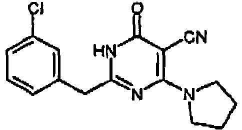 | **12** | 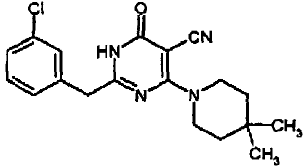 |
| **13** | 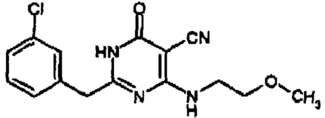 | **14** | 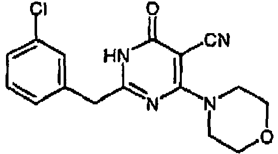 |
| **15** | 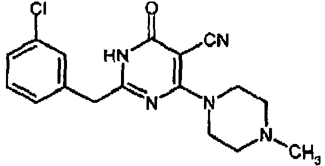 | **16** | 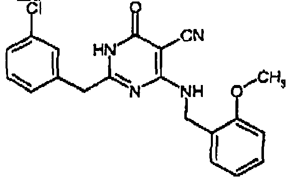 |
| **17** | 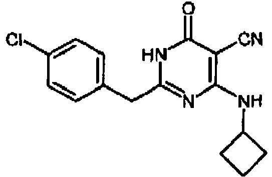 | **18** | 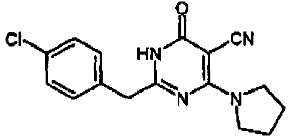 |
| **19** | 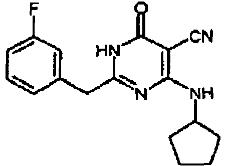 | **20** | 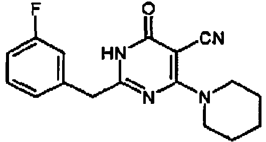 |
| **21** | 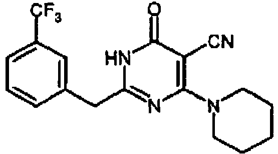 | **22** | 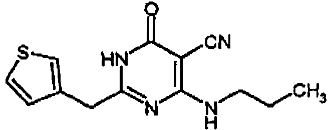 |
| **23** | 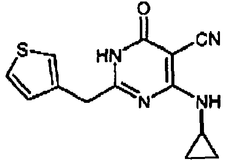 | **24** | 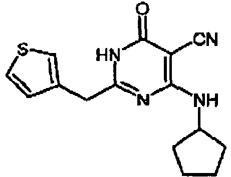 |
| **25** | 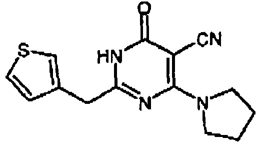 | **26** | 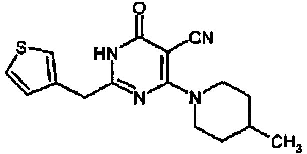 |
| **27** | 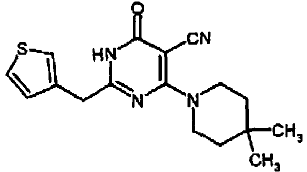 | **28** | 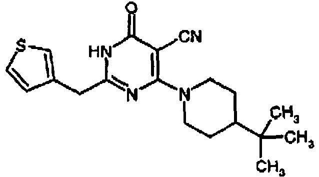 |
| **29** | 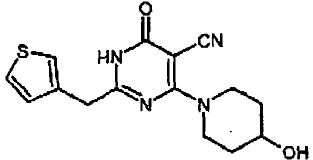 | **30** | 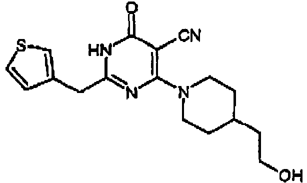 |
| **31** | 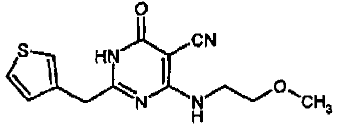 | **32** | 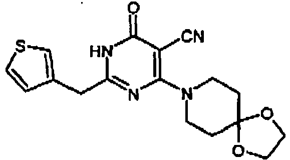 |
| **33** | 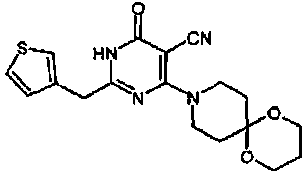 | **34** | 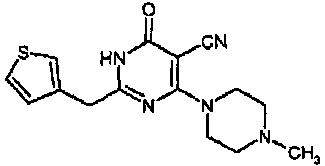 |
| **35** | 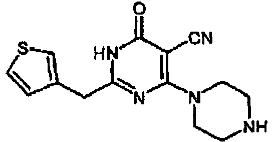 | **36** | 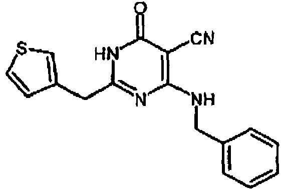 |
| **37** | 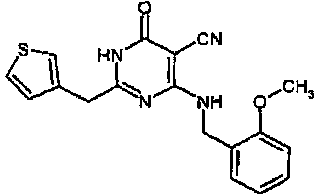 | **38** | 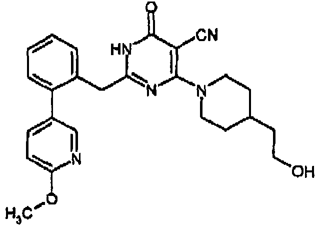 |
| **39** | 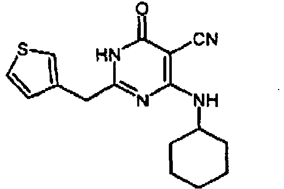 | **40** | 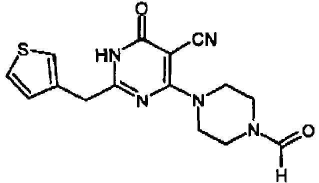 |
| **41** | 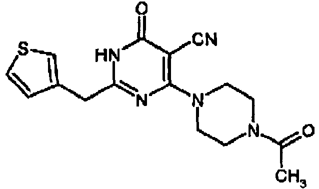 | **42** | 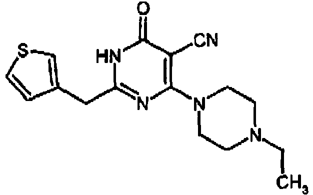 |
| **43** | 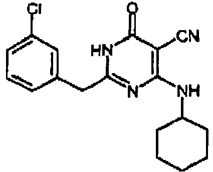 | **44** | 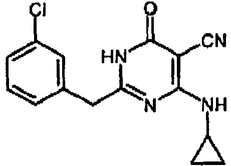 |
| **45** | 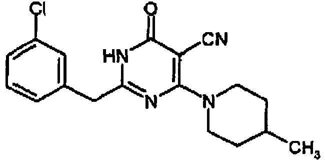 | **46** | 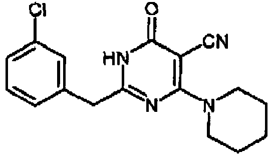 |
| **47** | 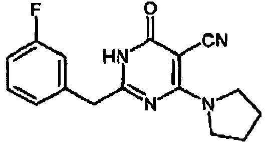 | **48** | 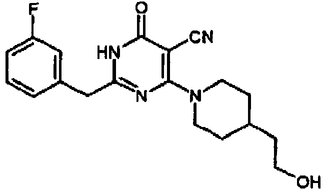 |
| **49** | 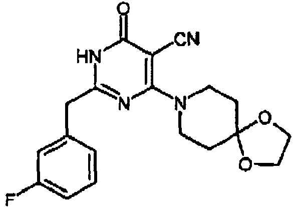 | **50** | 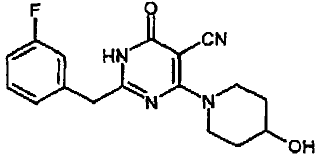 |
| **51** | 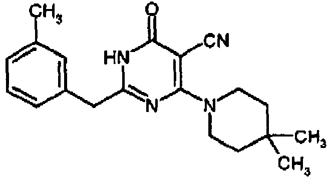 | **52** | 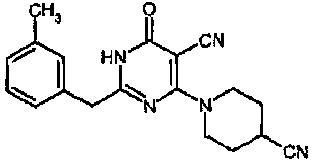 |
| **53** | 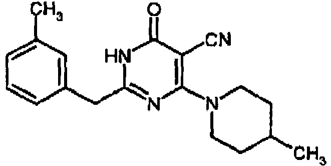 | **54** | 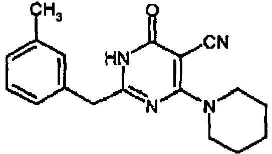 |
| **55** | 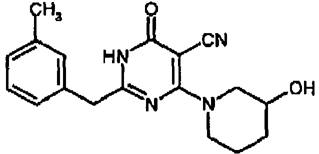 | **56** | 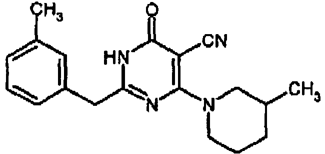 |
| **57** | 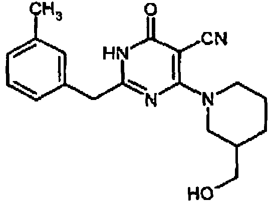 | **58** | 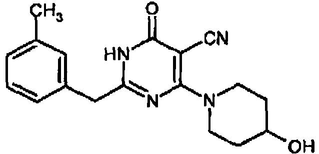 |
| **59** | 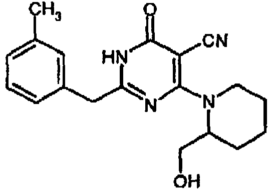 | **60** | 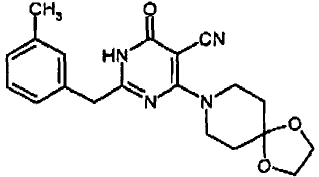 |
| **61** | 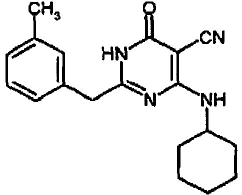 | **62** | 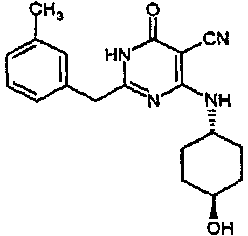 |
| **63** | 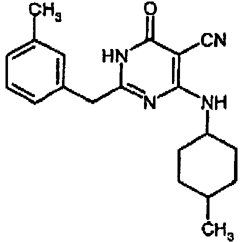 | **64** | 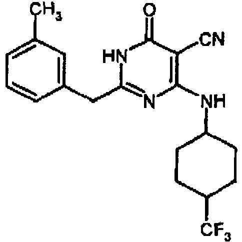 |
| **65** | 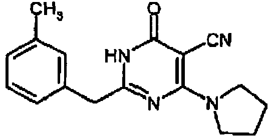 | **66** | 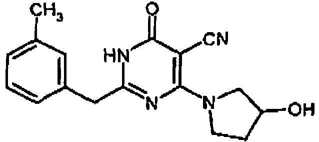 |
| **67** | 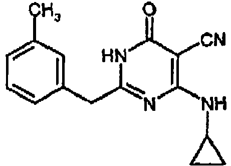 | **68** | 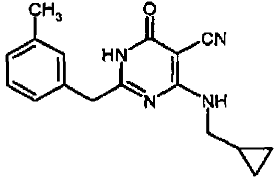 |
| **69** | 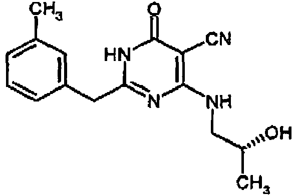 | **70** | 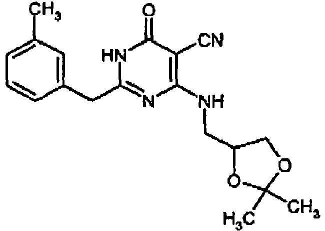 |
| **71** | 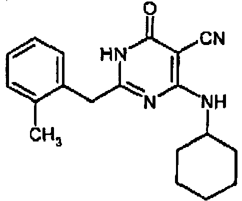 | **72** | 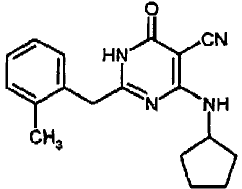 |
| **73** | 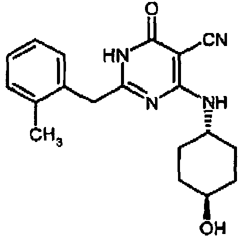 | **74** | 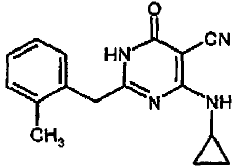 |
| **75** | 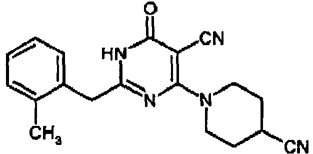 | **76** | 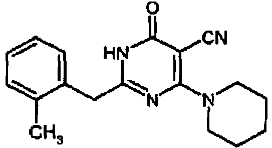 |
| **77** | 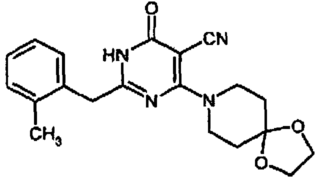 | **78** | 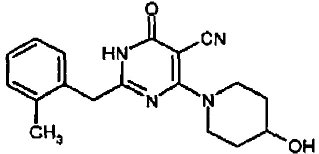 |
| **79** | 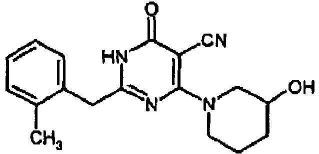 | **80** | 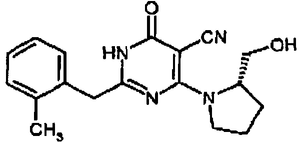 |
| **81** | 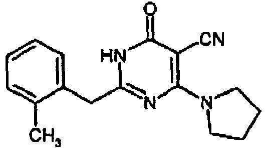 | **82** | 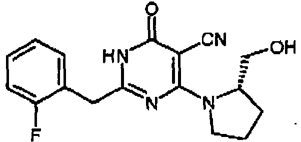 |
| **83** | 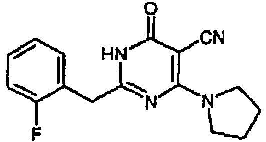 | **84** | 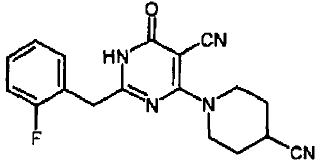 |
| **85** | 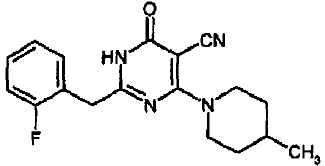 | **86** | 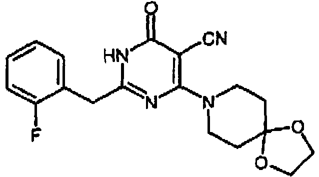 |
| **87** | 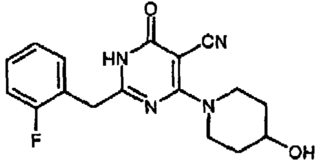 | **88** | 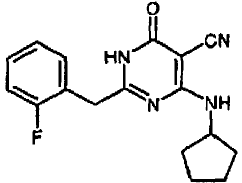 |
| **89** | 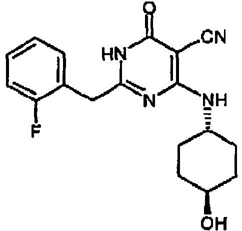 | **90** | 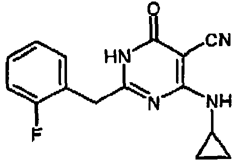 |
| **91** | 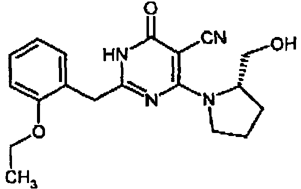 | **92** | 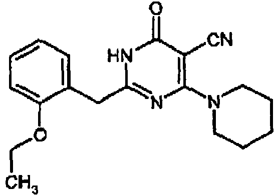 |
| **93** | 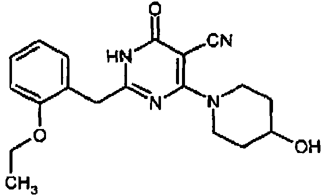 | **94** | 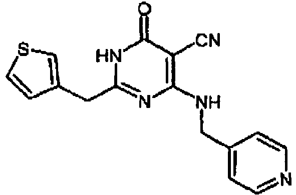 |
| **95** | 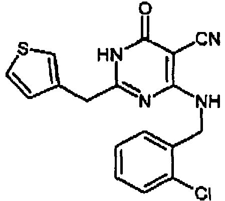 | **96** | 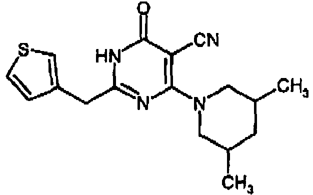 |
| **97** | 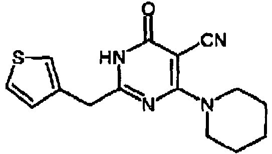 | **98** | 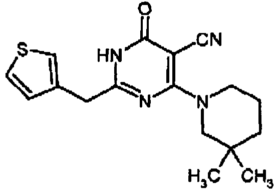 |
| **99** | 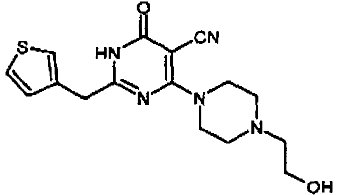 | **100** | 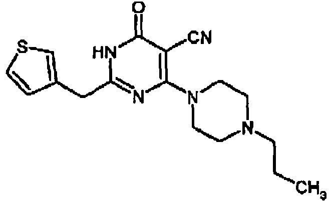 |
| **101** | 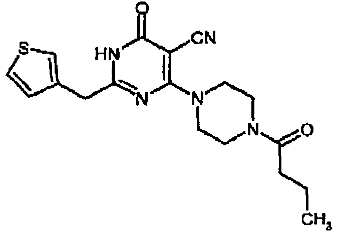 | **102** | 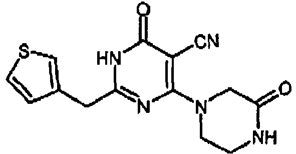 |
| **103** | 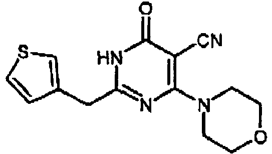 | **104** | 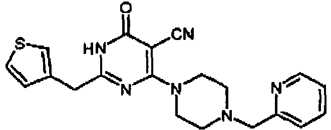 |
| **105** | 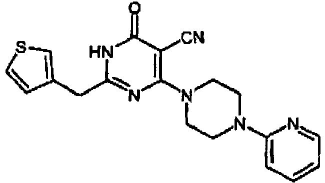 | **106** | 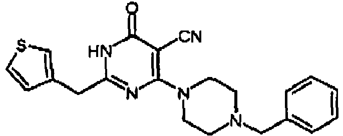 |
| **107** | 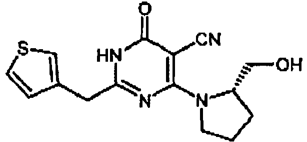 | **108** | 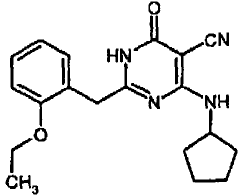 |
| **109** | 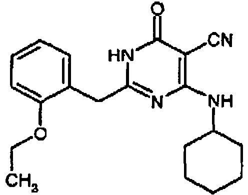 | **110** | 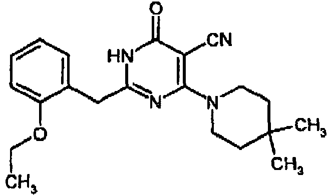 |
| **111** | 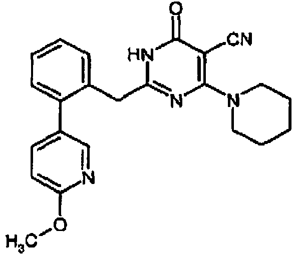 | **112** | 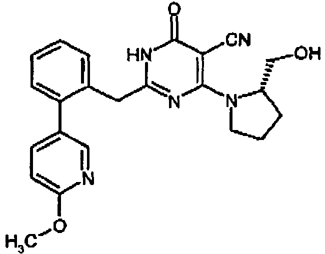 |
| **113** | 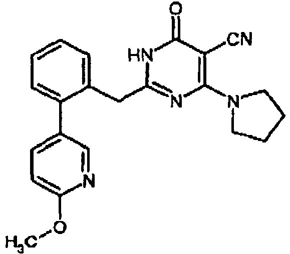 | **114** | 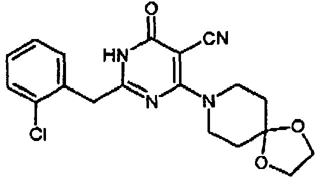 |
| **115** | 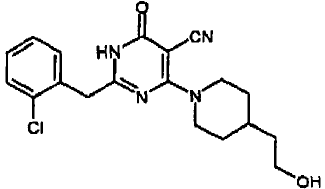 | **116** | 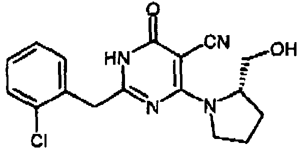 |
| **117** | 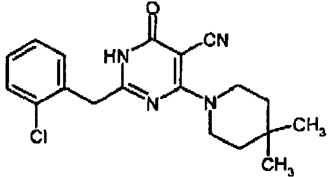 | **118** | 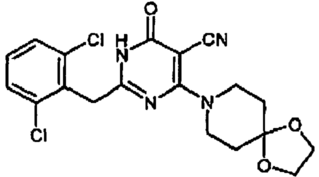 |
| **119** | 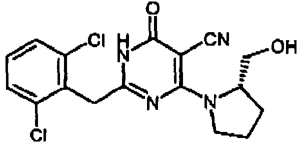 | **120** | 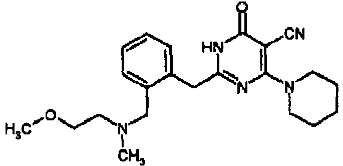 |
| **121** | 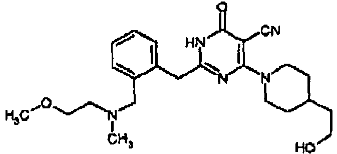 | **122** | 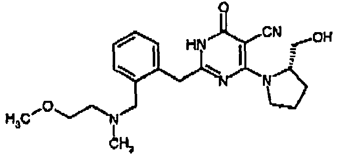 |
| **123** | 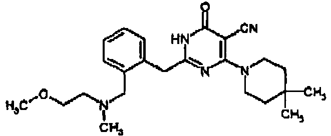 | **124** | 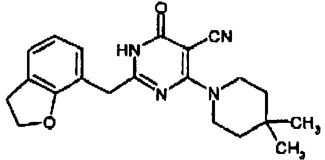 |
| **125** | 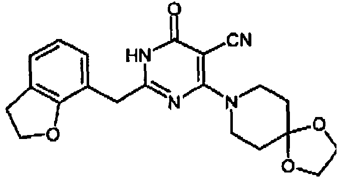 | **126** | 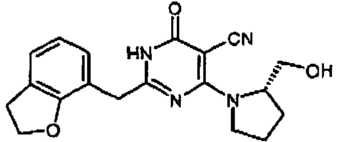 |
| **127** | 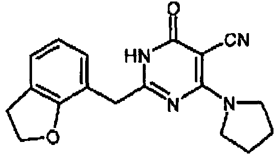 | **128** | 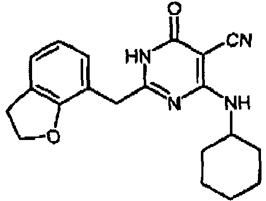 |
| **129** | 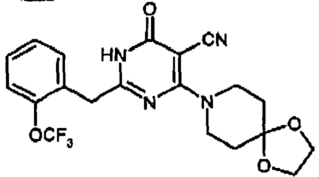 | **130** | 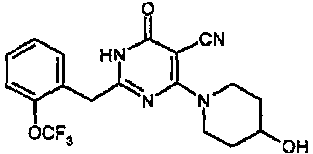 |
| **131** | 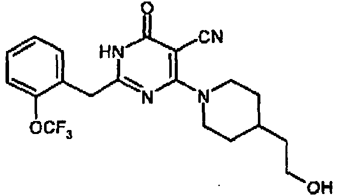 | **132** | 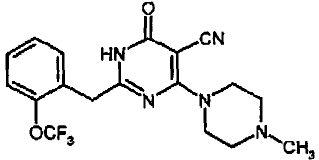 |
| **133** | 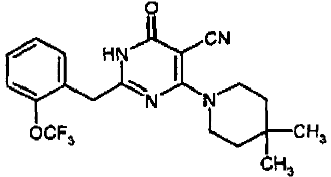 | **134** | 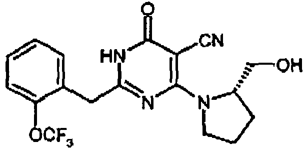 |
| **135** | 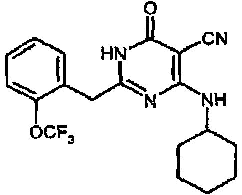 | **136** | 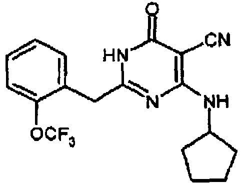 |
| **137** | 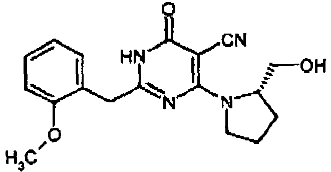 | **138** | 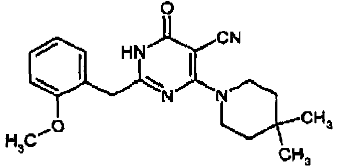 |
| **139** | 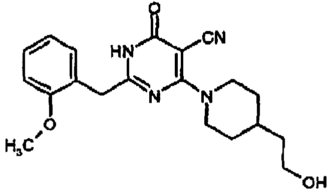 | **140** | 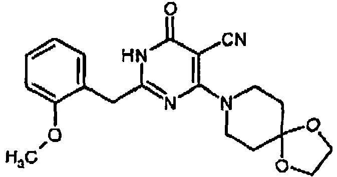 |
| **141** | 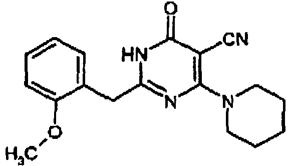 | **142** | 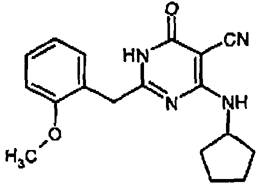 |
| **143** | 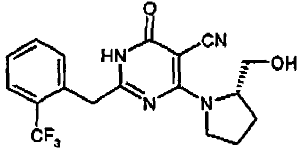 | **144** | 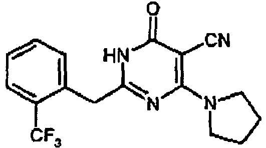 |
| **145** | 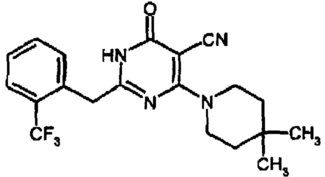 | **146** | 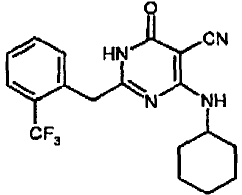 |
| **147** | 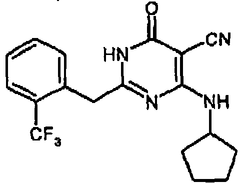 | **148** | 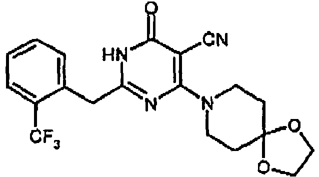 |
| **149** | 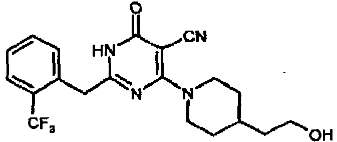 | **150** | 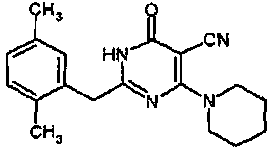 |
| **151** | 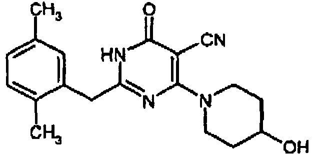 | **152** | 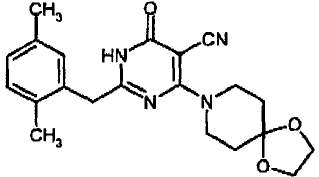 |
| **153** | 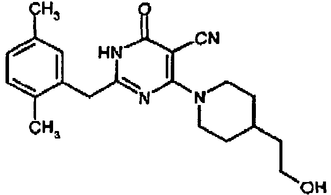 | **154** | 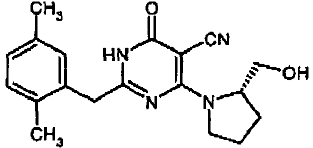 |
| **155** | 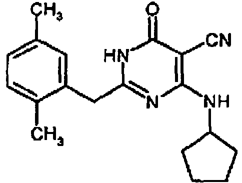 | **156** | 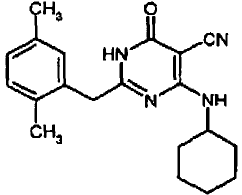 |
| **157** | 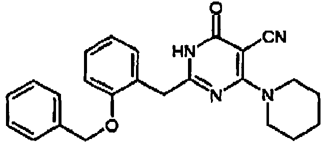 | **158** | 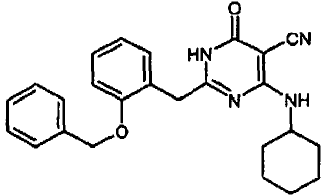 |
| **159** | 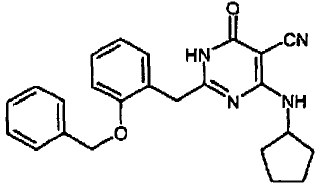 | **160** | 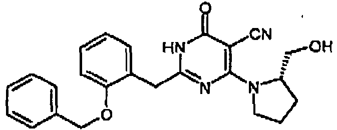 |
| **161** | 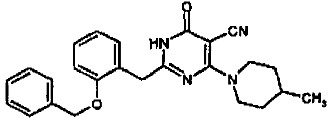 | **162** | 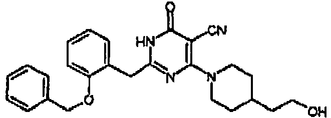 |
| **163** | 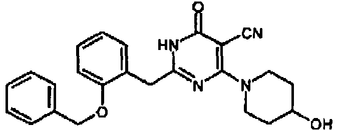 | **164** | 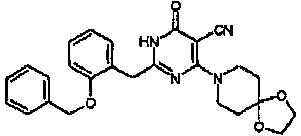 |

7. Verwendung der Verbindungen nach einem der Ansprüche 5 oder 6 zur Herstellung eines Arzneimittels zur Prophylaxe und/oder Behandlung von Störungen der Wahrnehmung, Konzentrationsleistung, Lern- und/oder Gedächtnisleistung.

8. Verwendung nach Anspruch 7, wobei die Störung eine Folge der Alzheimer'schen Krankheit ist.

9. Verwendung der Verbindungen nach einem der Ansprüche 5 oder 6 zur Herstellung eines Arzneimittels zur Verbesserung der Wahrnehmung, Konzentrationsleistung, Lern- und/oder Gedächtnisleistung.

## Claims

1. Process for preparing compounds of formula 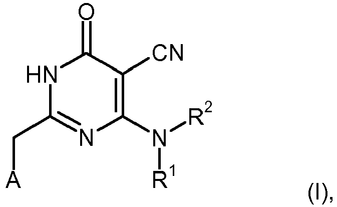 wherein
A denotes phenyl, heteroaryl, which constitutes an aromatic monocyclic group having 5 or 6 ring atoms and up to 3 heteroatoms selected from among S, O and/or N, or a group of the formula 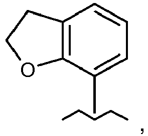 wherein phenyl and heteroaryl are optionally substituted by up to 2 groups selected independently of one another from among heteroaryl constituting an aromatic monocyclic group with 5 to 6 ring atoms and up to 3 heteroatoms selected from among S, O and/or N, halogen, C₁-C₆-alkyl, C₁-C₆-alkoxy, trifluoromethyl, trifluoromethoxy, benzyloxy and benzyl,
wherein C₁-C₆-alkyl is optionally substituted by a group of the formula -NR³R⁴, wherein R³ denotes C₁-C₆-alkyl and R⁴ denotes hydrogen or C₁-C₆-alkoxy(C₁-C₆-alkyl), and
heteroaryl is optionally substituted by C₁-C₆-alkoxy,
R¹ denotes C₃-C₈-cycloalkyl, C₁-C₆-alkyl, C₁-C₆-alkoxy(C₁-C₆-alkyl), benzyl or a group of the formula 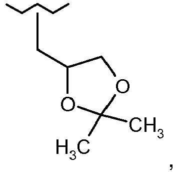 wherein C₃-C₈-cycloalkyl is optionally substituted by hydroxy, C₁-C₆-alkyl or trifluoromethyl,
C₁-C₆-alkyl is optionally substituted by heteroaryl, which constitutes an aromatic monocyclic group with 5 to 6 ring atoms and up to 3 heteroatoms selected from among S, O and/or N, C₃-C₈-cycloalkyl or hydroxy,
and benzyl is optionally substituted by C₁-C₆-alkoxy or halogen,
R² denotes hydrogen,
or
R¹ and R² together with the nitrogen atom to which they are bound denote a 5- to 6-membered heterocyclyl, said 5- to 6-membered heterocyclyl being a monocyclic, saturated or partially unsaturated heterocyclic group with 5 to 6 ring atoms and up to 2 heteroatoms selected from among N, O, S, which is optionally substituted by up to 2 substitutents selected independently of one another from among C₁-C₆-alkyl, hydroxy, cyano, oxo, heteroaryl, which constitutes an aromatic monocyclic group with 5 to 6 ring atoms and up to 3 heteroatoms, selected from among S, O and/or N, benzyl, formyl, C₁-C₆-alkylcarbonyl and one of the following groups 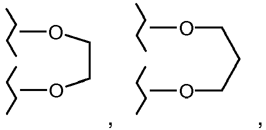 which are bound by means of one of the two oxygen atoms to one of the carbon atoms in the heterocyclic group,
wherein C₁-C₆-alkyl is optionally substituted by hydroxyl or heteroaryl which constitutes an aromatic, monocyclic group with 5 or 6 ring atoms and up to 3 heteroatoms selected from among S, O and/or N,
and the salts, solvates and/or solvates of the salts thereof,
**characterised in that** either
[A] a compound of formula 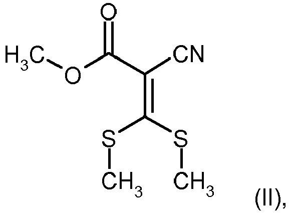 is first converted with a compound of formula
HNR¹R² (III),
wherein
R¹ and R² are as hereinbefore defined,
at elevated temperature in an inert solvent or in the absence of a solvent, into a compound of formula 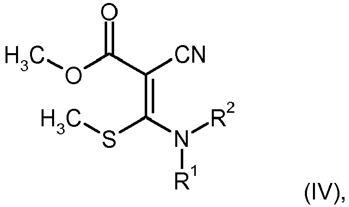 wherein
R¹ and R² are as hereinbefore defined,
and this is then reacted, in an inert solvent in the presence of a base, with a compound of formula 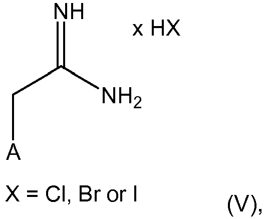 wherein
A is as hereinbefore defined,
or, in a different order of reactants,
[B] a compound of formula (II) is first converted with a compound of formula (V) in an inert solvent in the presence of a base into a compound of formula 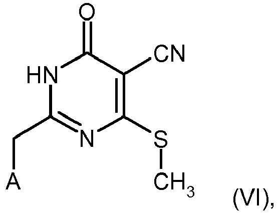 wherein
A is as hereinbefore defined,
and this is then reacted at elevated temperature in an inert solvent or also in the absence of a solvent, with a compound of formula (III),
and the resulting compounds of formula (I) are optionally reacted with the corresponding (i) solvents and/or (ii) bases or acids to form the solvates, salts and/or solvates of the salts thereof.

2. Process according to claim 1, wherein
A denotes phenyl, heteroaryl, which constitutes an aromatic monocyclic group having 5 or 6 ring atoms and up to 3 heteroatoms selected from among S, O and/or N, or a group of the formula 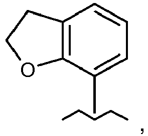
wherein phenyl and heteroaryl are optionally substituted by up to 2 groups selected independently of one another from among heteroaryl, which constitutes an aromatic monocyclic group with 5 to 6 ring atoms and up to 3 heteroatoms selected from among S, O and/or N, halogen, C₁-C₄-alkyl, C₁-C₄-alkoxy, trifluoromethyl, trifluoromethoxy, benzyloxy and benzyl,
wherein C₁-C₄-alkyl is optionally substituted by a group of the formula -NR³R⁴wherein R³ denotes C₁-C₄-alkyl and R⁴ denotes hydrogen or C₁-C₄-alkoxy(C₁-C₄-alkyl), and
heteroaryl is optionally substituted by C₁-C₄-alkoxy,
R¹ denotes C₃-C₆-cycloalkyl, C₁-C₄-alkyl, C₁-C₄-alkoxy(C₁-C₄-alkyl), benzyl or a group of the formula 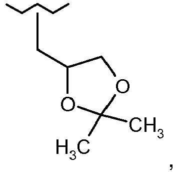
wherein C₃-C₆-cycloalkyl is optionally substituted by hydroxy, C₁-C₄-alkyl or trifluoromethyl,
C₁-C₄-alkyl is optionally substituted by heteroaryl, which constitutes an aromatic monocyclic group with 5 to 6 ring atoms and up to 3 heteroatoms selected from among S, O and/or N, C₃-C₆-cycloalkyl or hydroxy,
and benzyl is optionally substituted by C₁-C₄-alkoxy or halogen,
R² denotes hydrogen,
or
R¹ and R² together with the nitrogen atom to which they are bound denote a 5- to 6-membered heterocyclyl, said 5- to 6-membered heterocyclyl being a monocyclic, saturated or partially unsaturated heterocyclic group with 5 to 6 ring atoms and up to 2 heteroatoms selected from among N, O, S, which is optionally substituted by up to 2 substituents selected independently of one another from among C₁-C₄-alkyl, hydroxyl, cyano, oxo, heteroaryl, which constitutes an aromatic monocyclic group with 5 to 6 ring atoms and up to 3 heteroatoms selected from among S, O and/or N, benzyl, formyl, C₁-C₄-alkylcarbonyl and one of the following groups 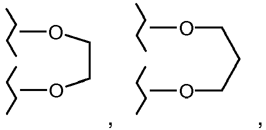 which are bound by means of the two oxygen atoms to one of the carbon atoms in the heterocyclic group,
wherein C₁-C₄-alkyl is optionally substituted by hydroxyl or heteroaryl, which constitutes an aromatic, monocyclic group with 5 or 6 ring atoms and up to 3 heteroatoms selected from among S, O and/or N,
and the salts, solvates and/or solvates of the salts thereof.

3. Process according to claims 1 and 2, wherein
A denotes phenyl, thienyl or a group of the formula 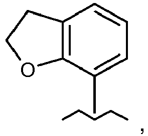 wherein phenyl and thienyl are optionally substituted by up to 2 groups selected independently of one another from among pyridyl, fluorine, chlorine, bromine, C₁-C₄-alkyl, C₁-C₄-alkoxy, trifluoromethyl, trifluoromethoxy, benzyloxy and benzyl,
wherein C₁-C₄-alkyl is optionally substituted by a group of the formula -NR³R⁴wherein R³ denotes C₁-C₄-alkyl and R⁴ denotes hydrogen or C₁-C₄-alkoxy(C₁-C₄)alkyl and
pyridyl is optionally substituted by C₁-C₄-alkoxy,
R¹ denotes C₃-C₆-cycloalkyl, C₁-C₄-alkyl, C₁-C₄-alkoxy(C₁-C₄)alkyl, benzyl or a group of the formula 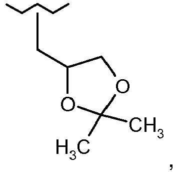
wherein C₃-C₆-cycloalkyl is optionally substituted by hydroxy, C₁-C₄-alkyl or trifluoromethyl,
C₁-C₄-alkyl is optionally substituted by pyridyl, C₃-C₆-cycloalkyl or hydroxy,
and benzyl is optionally substituted by C₁-C₄-alkoxy, fluorine, chlorine or bromine,
R² denotes hydrogen,
or
R¹ and R² together with the nitrogen atom to which they are bound denote a 5- to 6-membered heterocyclyl selected from among pyrrolidinyl, piperidinyl, piperazinyl and morpholinyl, which is optionally substituted by up to 2 substitutents selected independently of one another from among C₁-C₄-alkyl, hydroxy, cyano, oxo, heteroaryl, which constitutes an aromatic monocyclic group with 5 to 6 ring atoms and up to 3 heteroatoms selected from among S, O and/or N, benzyl, formyl, C₁-C₄-alkylcarbonyl and one of the following groups 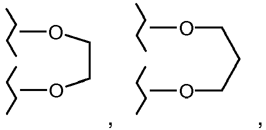 which are bound by means of the two oxygen atoms to one of the carbon atoms in the heterocyclic group,
wherein C₁-C₄-alkyl is optionally substituted by hydroxyl or pyridyl,
and the salts, solvates and/or solvates of the salts thereof.

4. Process according to claims 1, 2 and 3, wherein
A denotes phenyl, thienyl or a group of the formula 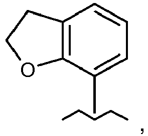
wherein phenyl is optionally substituted by up to 2 groups selected independently of one another from among pyridyl, fluorine, chlorine, methyl, methoxy, ethoxy, trifluoromethyl, trifluoromethoxy, benzyloxy and benzyl,
wherein methyl is optionally substituted by a group of the formula -NR³R⁴wherein R³ denotes methyl and R⁴ denotes hydrogen or 2-methoxyethyl, and
pyridyl is optionally substituted by methoxy,
R¹ denotes C₃-C₆-cycloalkyl, methyl, ethyl, propyl, 2-methoxyethyl, benzyl or a group of the formula 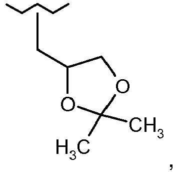
wherein C₃-C₆-cycloalkyl is optionally substituted by hydroxy, methyl or trifluoromethyl,
methyl, ethyl, propyl is optionally substituted by pyridyl, cyclopropyl or hydroxy,
and benzyl is optionally substituted by methoxy, ethoxy, fluorine or chlorine,
R² denotes hydrogen,
or
R¹ and R² together with the nitrogen atom to which they are bound denote a 5- to 6-membered heterocyclyl selected from among pyrrolidinyl, piperidinyl, piperazinyl and morpholinyl, which is optionally substituted by up to 2 substituents selected independently of one another from among methyl, ethyl, propyl, tert. butyl, hydroxy, cyano, oxo, pyridyl, benzyl, formyl, methylcarbonyl, ethylcarbonyl, propylcarbonyl and one of the following groups 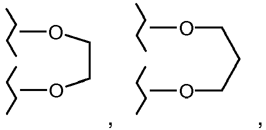 which are bound by means of the two oxygen atoms to one of the carbon atoms in the heterocyclic group,
wherein methyl, ethyl and propyl are optionally substituted by hydroxy or pyridyl,
and the salts, solvates and/or solvates of the salts thereof.

5. Compounds according to the formula 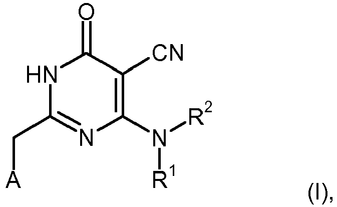 wherein the groups A, R¹ and R² are defined as in claims 1 to 4 and the salts, solvates and/or solvates of the salts thereof, with the exception of the following compound:

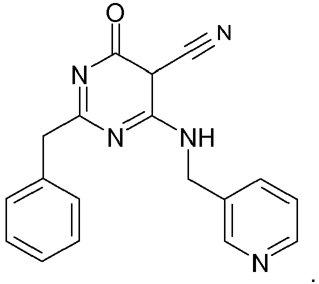

6. A compound selected from among:
| Compound | Structure | Compound | Structure |
|---|---|---|---|
| **1** | 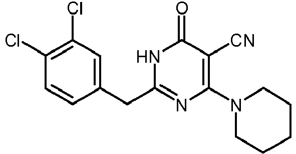 | **2** | 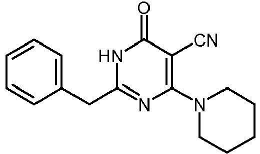 |
| **3** | 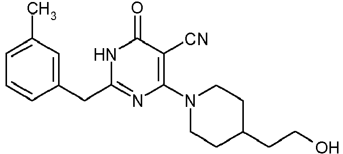 | **4** | 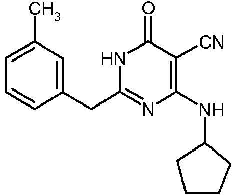 |
| **5** | 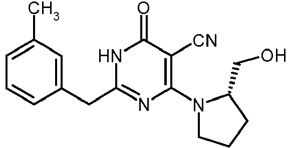 | **6** | 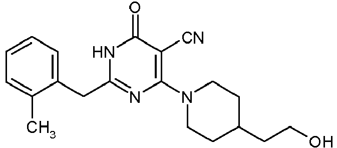 |
| **7** | 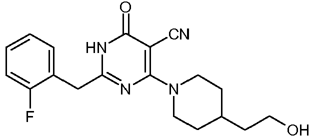 | **8** | 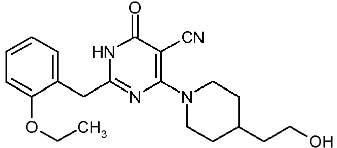 |
| **9** | 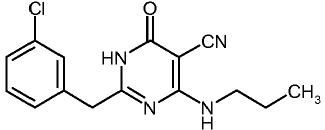 | **10** | 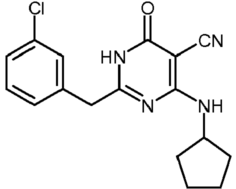 |
| **11** | 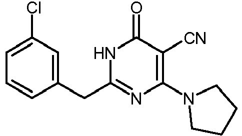 | **12** | 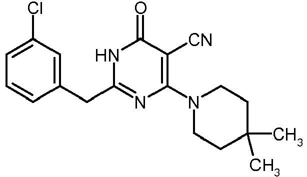 |
| **13** | 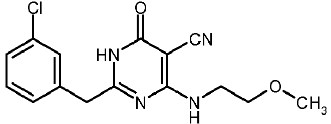 | **14** | 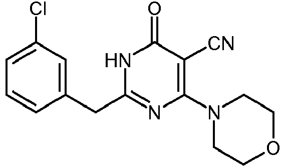 |
| **15** | 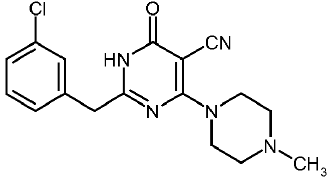 | **16** | 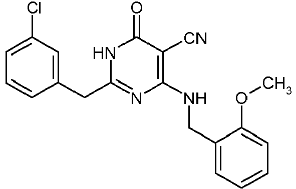 |
| **17** | 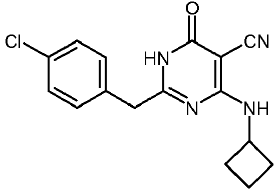 | **18** | 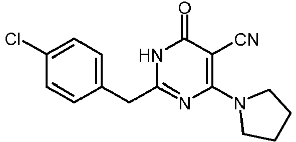 |
| **19** | 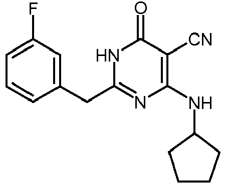 | **20** | 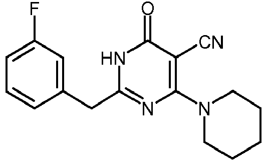 |
| **21** | 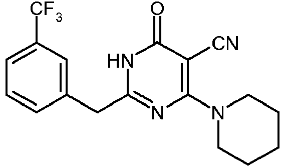 | **22** | 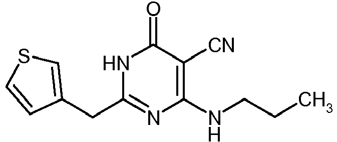 |
| **23** | 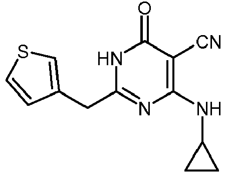 | **24** | 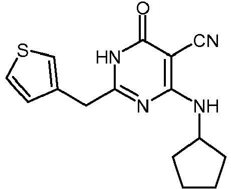 |
| **25** | 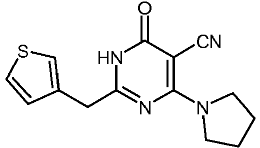 | **26** | 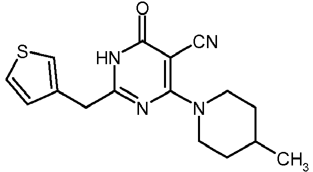 |
| **27** | 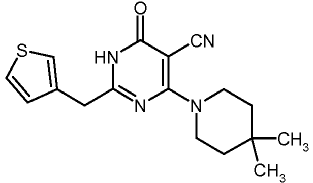 | **28** | 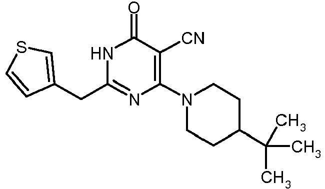 |
| **29** | 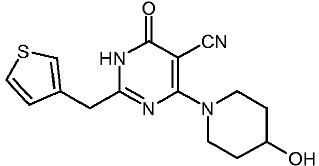 | **30** | 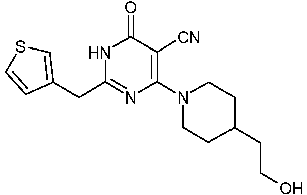 |
| **31** | 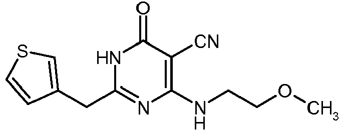 | **32** | 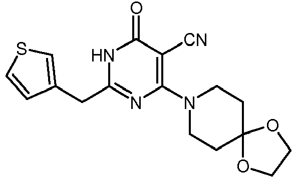 |
| **33** | 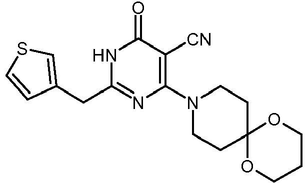 | **34** | 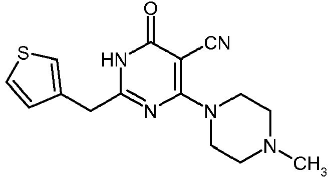 |
| **35** | 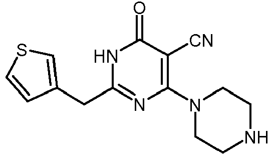 | **36** | 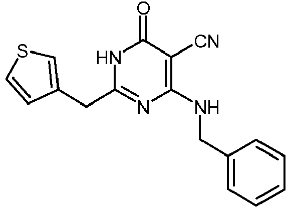 |
| **37** | 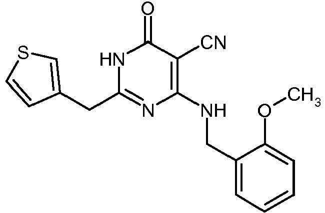 | **38** | 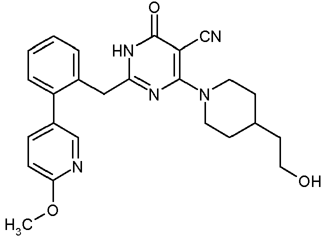 |
| **39** | 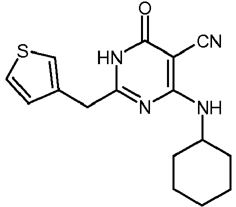 | **40** | 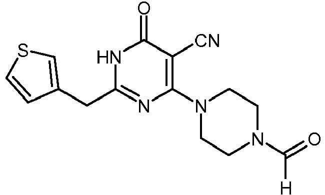 |
| **41** | 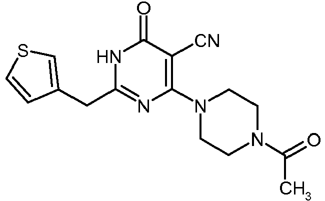 | **42** | 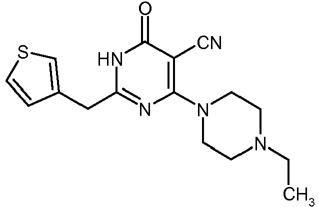 |
| **43** | 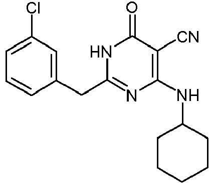 | **44** | 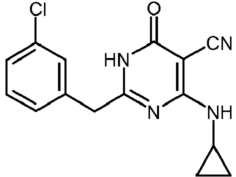 |
| **45** | 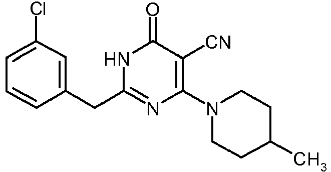 | **46** | 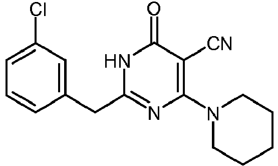 |
| **47** | 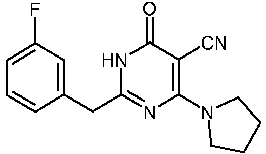 | **48** | 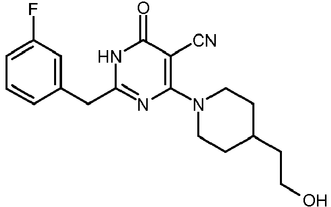 |
| **49** | 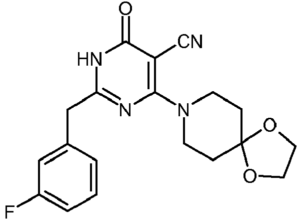 | **50** | 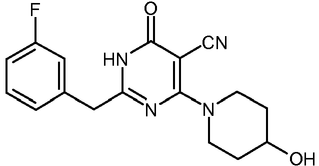 |
| **51** | 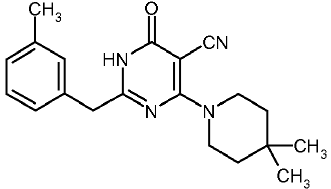 | **52** | 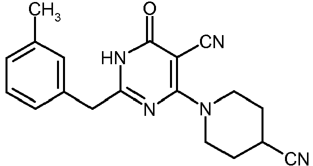 |
| **53** | 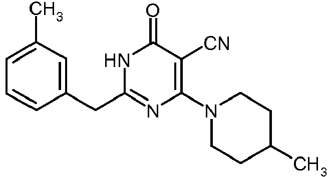 | **54** | 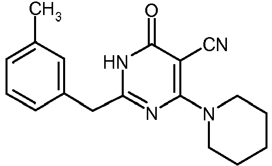 |
| **55** | 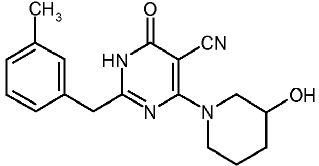 | **56** | 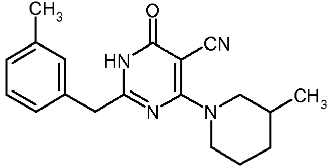 |
| **57** | 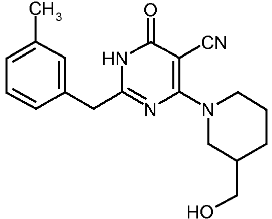 | **58** | 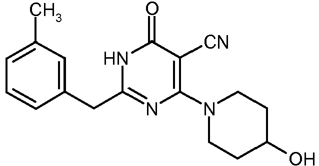 |
| **59** | 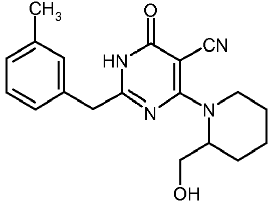 | **60** | 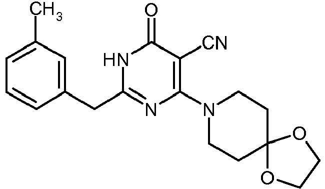 |
| **61** | 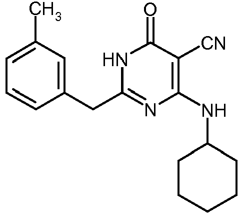 | **62** | 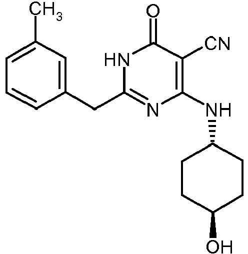 |
| **63** | 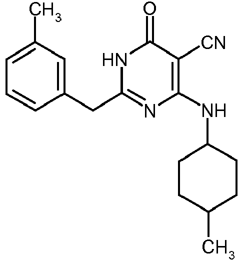 | **64** | 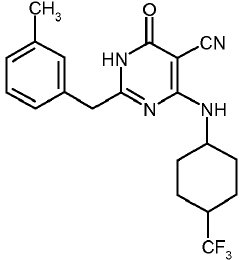 |
| **65** | 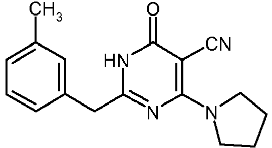 | **66** | 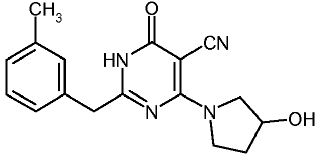 |
| **67** | 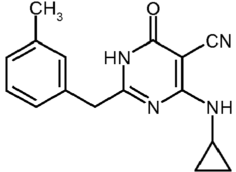 | **68** | 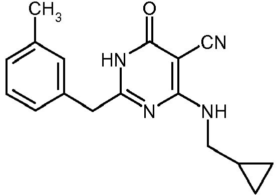 |
| **69** | 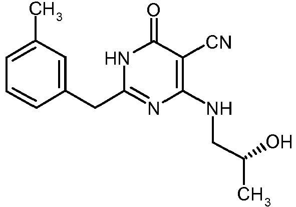 | **70** | 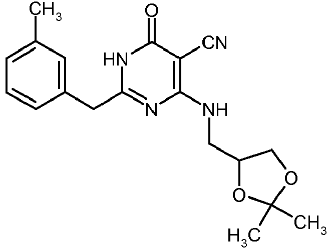 |
| **71** | 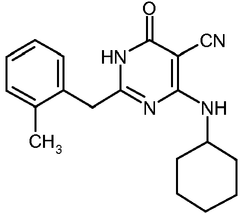 | **72** | 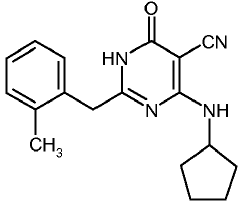 |
| **73** | 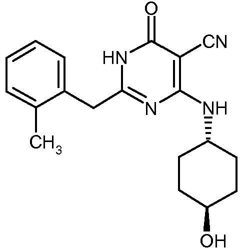 | **74** | 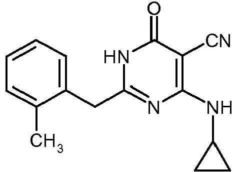 |
| **75** | 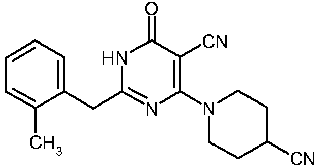 | **76** | 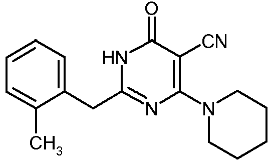 |
| **77** | 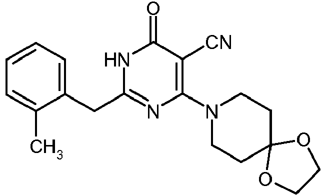 | **78** | 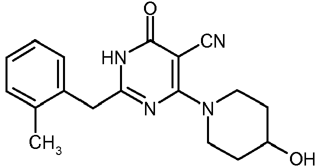 |
| **79** | 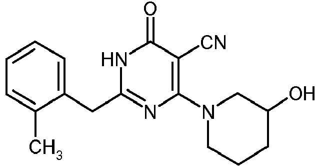 | **80** | 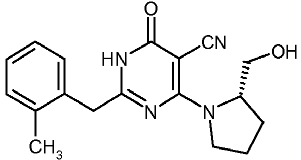 |
| **81** | 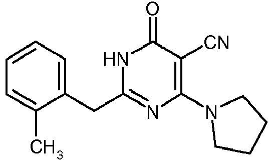 | **82** | 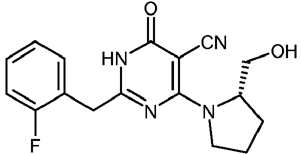 |
| **83** | 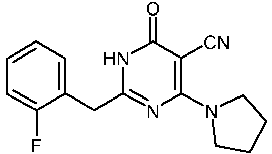 | **84** | 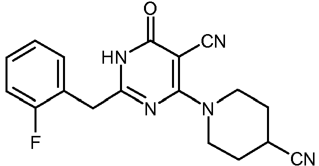 |
| **85** | 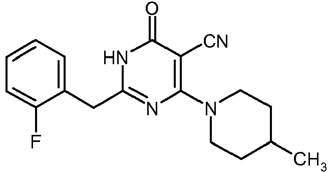 | **86** | 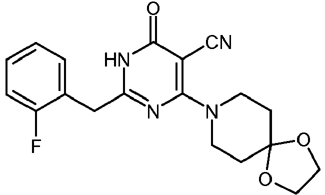 |
| **87** | 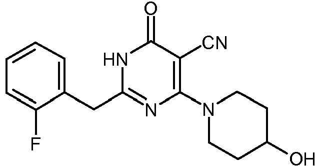 | **88** | 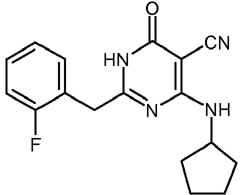 |
| **89** | 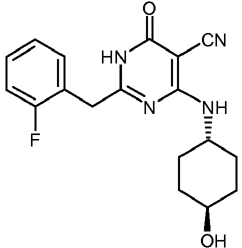 | **90** | 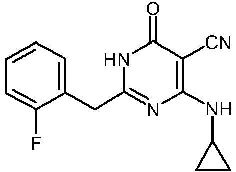 |
| **91** | 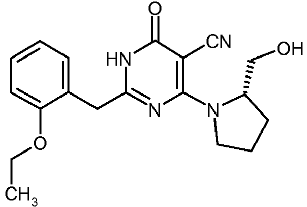 | **92** | 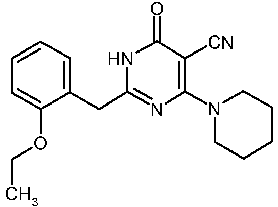 |
| **93** | 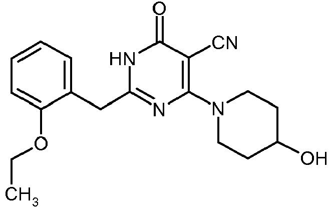 | **94** | 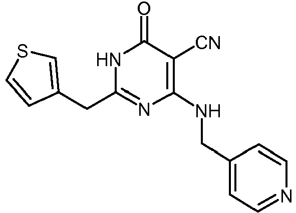 |
| **95** | 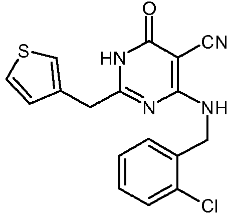 | **96** | 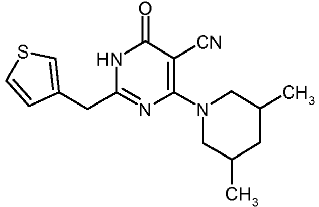 |
| **97** | 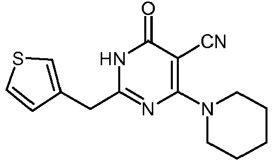 | **98** | 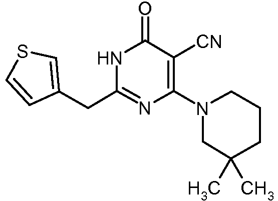 |
| **99** | 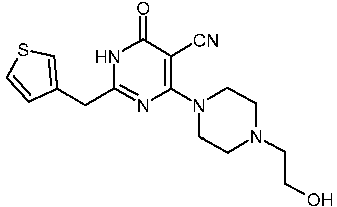 | **100** | 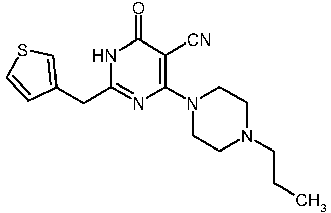 |
| **101** | 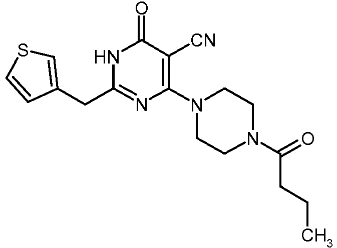 | **102** | 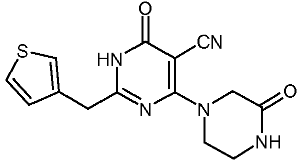 |
| **103** | 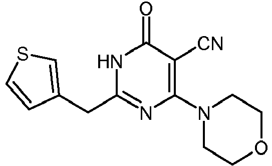 | **104** | 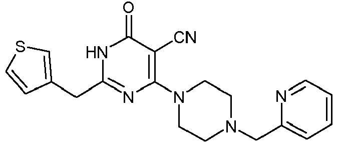 |
| **105** | 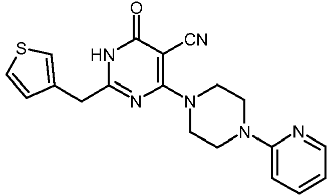 | **106** | 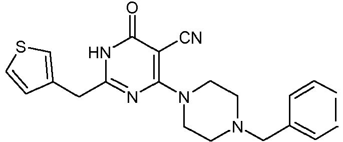 |
| **107** | 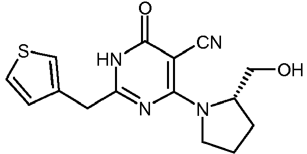 | **108** | 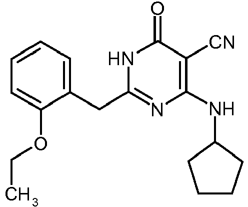 |
| **109** | 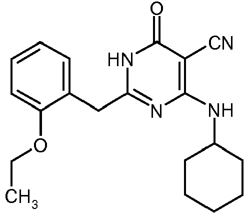 | **110** | 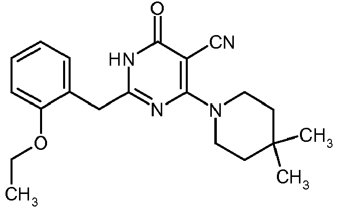 |
| **111** | 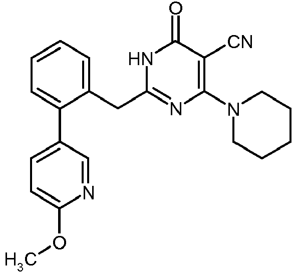 | **112** | 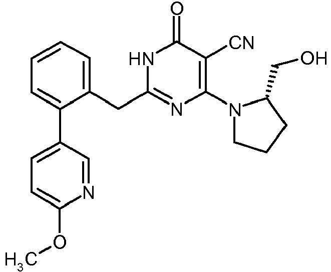 |
| **113** | 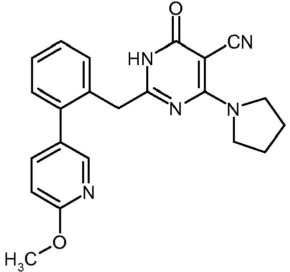 | **114** | 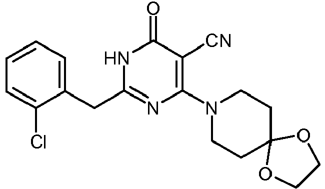 |
| **115** | 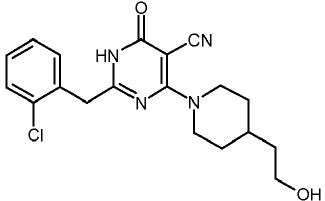 | **116** | 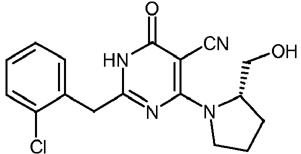 |
| **117** | 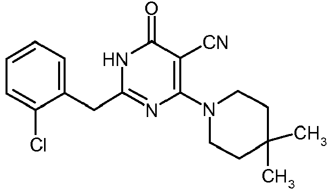 | **118** | 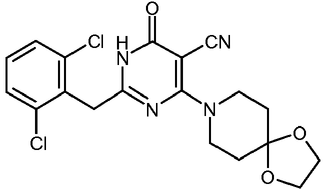 |
| **119** | 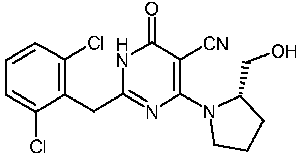 | **120** | 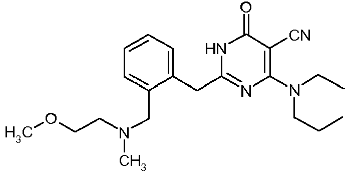 |
| **121** | 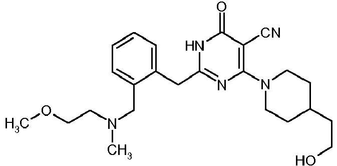 | **122** | 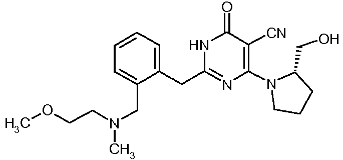 |
| **123** | 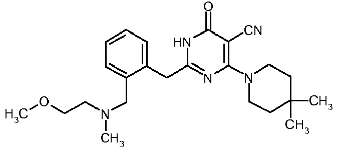 | **124** | 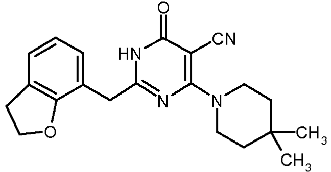 |
| **125** | 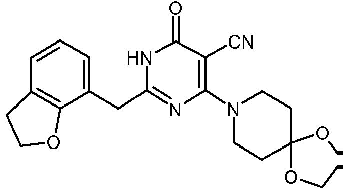 | **126** | 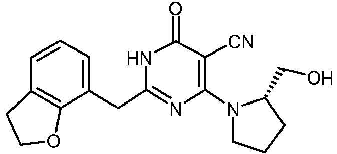 |
| **127** | 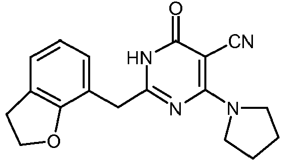 | **128** | 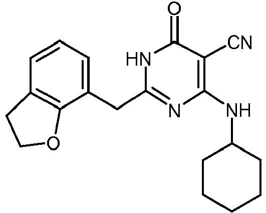 |
| **129** | 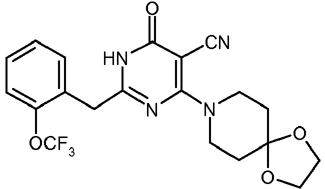 | **130** | 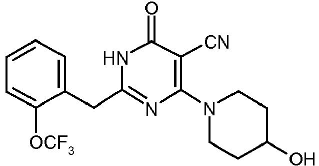 |
| **131** | 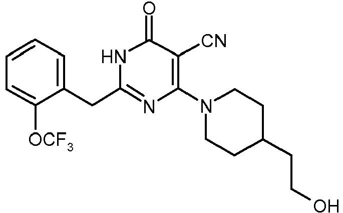 | **132** | 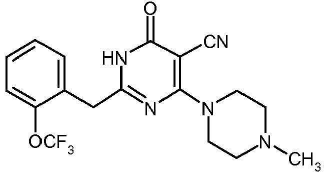 |
| **133** | 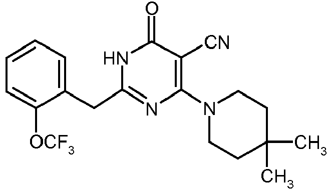 | **134** | 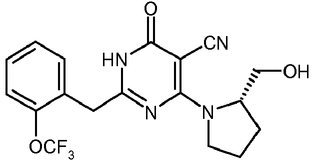 |
| **135** | 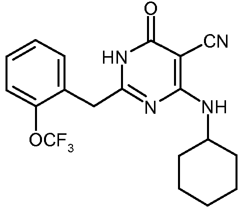 | **136** | 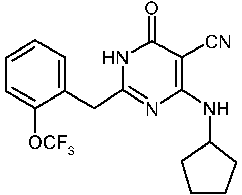 |
| **137** | 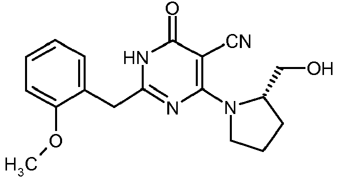 | **138** | 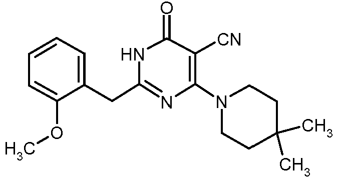 |
| **139** | 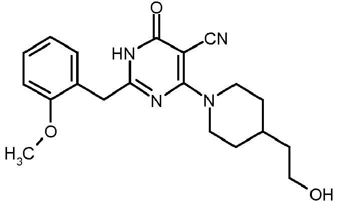 | **140** | 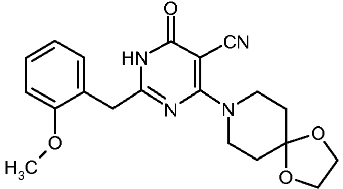 |
| **141** | 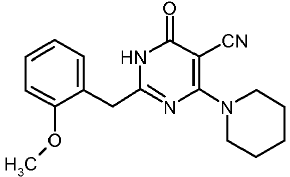 | **142** | 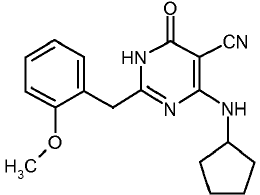 |
| **143** | 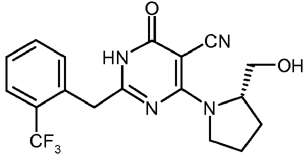 | **144** | 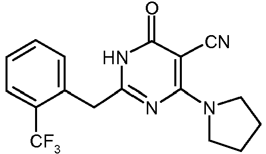 |
| **145** | 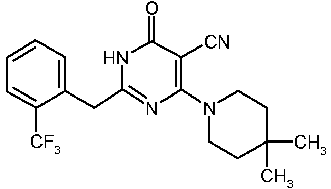 | **146** | 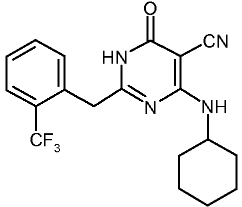 |
| **147** | 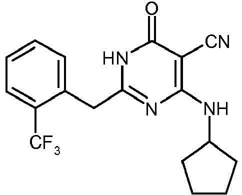 | **148** | 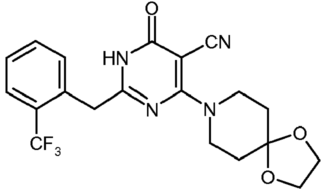 |
| **149** | 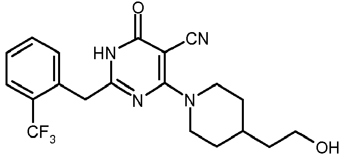 | **150** | 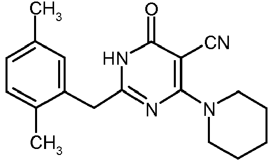 |
| **151** | 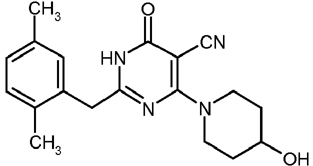 | **152** | 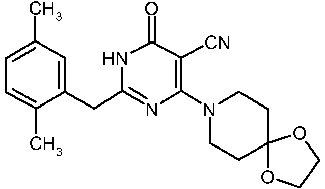 |
| **153** | 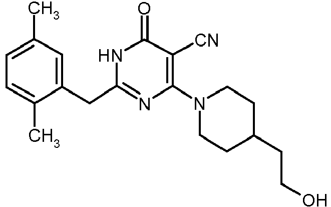 | **154** | 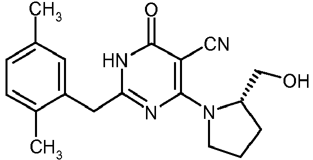 |
| **155** | 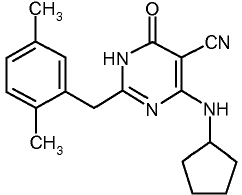 | **156** | 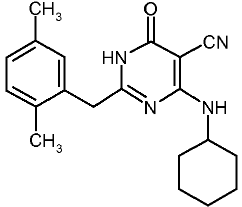 |
| **157** | 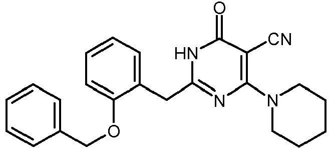 | **158** | 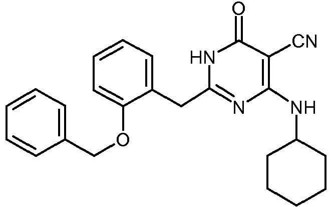 |
| **159** | 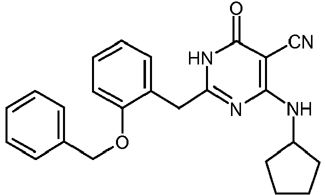 | **160** | 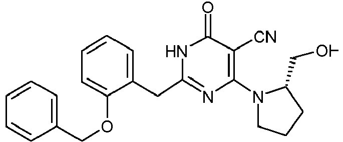 |
| **161** | 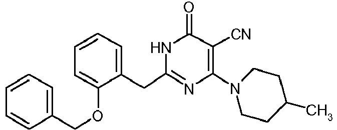 | **162** | 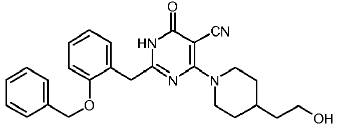 |
| **163** | 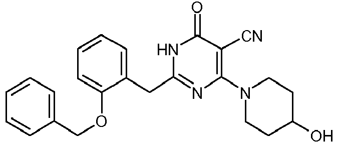 | **164** | 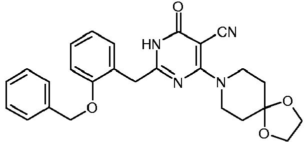 |

7. Use of the compounds according to one of claims 5 or 6 for preparing a medicament for the prevention and/or treatment of disorders of perception, concentration, learning and/or memory.

8. Use according to claim 7 wherein the disorder is a consequence of Alzheimer's disease.

9. Use of the compounds according to one of claims 5 or 6 for preparing a medicament for improving perception, concentration, learning and/or memory.

## Revendications

1. Procédé pour la production de composés selon la formule 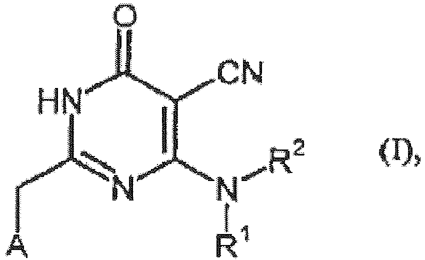 dans laquelle
A est un groupe phényle, hétéroaryle qui représente un radical aromatique monocyclique comportant 5 à 6 atomes cycliques et jusqu'à 3 hétéroatomes de la série S, O et/ou N, ou un groupe de formule 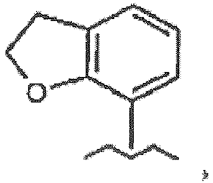 les groupes phényle et hétéroaryle étant substitués éventuellement par jusqu'à 2 radicaux choisis indépendamment l'un de l'autre dans le groupe hétéroaryle qui représente un radical aromatique monocyclique comportant 5 à 6 atomes cycliques et jusqu'à 3 hétéroatomes de la série S, O et/ou N, un atome d'halogène, un groupe alkyle en C₁ à C₆, un groupe alcoxy en C₁ à C₆, trifluorométhyle, trifluorométhoxy, benzyloxy et benzyle,
le groupe alkyle en C₁ à C₆ étant substitué éventuellement par un groupe de formule -NR³R⁴ dans lequel R³ est un groupe alkyle en C₁ à C₆ et R⁴ est un atome d'hydrogène ou un groupe alcoxy en C₁ à C₆-alkyle (en C₁ à C₆), et
le groupe hétéroaryle est éventuellement substitué par un groupe alcoxy en C₁ à C₆,
R¹ est un groupe cycloalkyle en C₃ à C₈, alkyle en C₁ à C₆, alcoxy en C₁ à C₆-alkyle (en C₁ à C₆), benzyle ou un groupe de formule 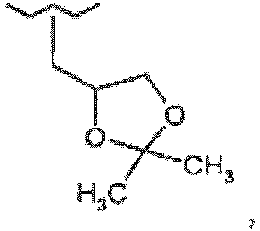
dans laquelle le groupe cycloalkyle en C₃ à C₈ est substitué éventuellement par un groupe hydroxy, alkyle en C₁ à C₆ ou trifluorométhyle,
le groupe alkyle en C₁ à C₆ est substitué éventuellement par un groupe hétéroaryle qui représente un radical aromatique monocyclique comportant 5 à 6 atomes cycliques et jusqu'à 3 hétéroatomes de la série S, O et/ou N, un groupe cycloalkyle en C₃ à C₈ ou hydroxy,
et le groupe benzyle est substitué éventuellement par un groupe alcoxy en C₁ à C₆ ou un atome d'halogène,
R² est un atome d'hydrogène
ou
R¹ et R² conjointement avec l'atome d'azote auquel ils sont liés, forment un groupe hétérocyclyle de 5 à 6 chaînons, ledit groupe hétérocyclyle de 5 à 6 chaînons étant un radical hétérocyclique, monocyclique, saturé ou partiellement insaturé comportant 5 à 6 atomes cycliques et jusqu'à 2 hétéroatomes de la série N, O, S, qui est substitué éventuellement par jusqu'à 2 substituants choisis indépendamment l'un de l'autre dans le groupe comprenant les groupes alkyle en C₁ à C₆, hydroxy, cyano, oxo, hétéroaryle qui représente un radical aromatique monocyclique comportant 5 à 6 atomes cycliques et jusqu'à 3 hétéroatomes de la série S, O et/ou N, benzyle, formyle, alkylcarbonyle en C₁ à C₆ et l'un des groupes suivants 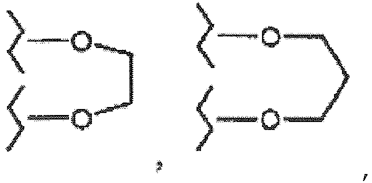 qui sont liés par les deux atomes d'oxygène à l'un des atomes de carbone dans l'hétérocycle,
le groupe alkyle en C₁ à C₆ étant substitué éventuellement par un groupe hydroxy ou hétéroaryle qui est un radical aromatique monocyclique comportant 5 à 6 atomes cycliques et jusqu'à 3 hétéroatomes de la série S, O et/ou N
et leurs sels, leurs solvates et/ou les solvates de leurs sels, **caractérisé en ce que**, soit
[A] on convertit un composé de formule 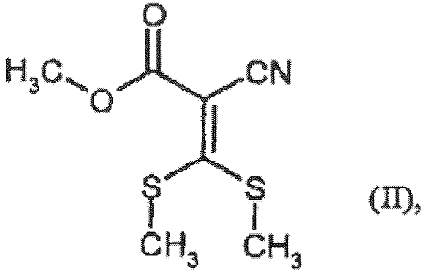 tout d'abord avec un composé de formule
HNR¹R² (III),
dans laquelle
R¹ et R² ont les significations mentionnées ci-dessus,
à température élevée dans un solvant inerte ou également en l'absence d'un solvant, en un composé de formule 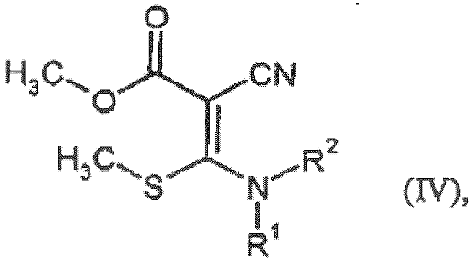 dans laquelle
R¹ et R² ont les significations mentionnées ci-dessus,
et on fait ensuite réagir celui-ci dans un solvant inerte en présence d'une base avec un composé de formule 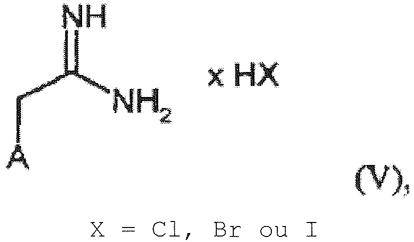 dans laquelle
A a les significations mentionnées ci-dessus, soit dans un ordre différent des partenaires réactionnels,
[B] on convertit un composé de formule (II) tout d'abord avec un composé de formule (V) dans un solvant inerte en présence d'une base en un composé de formule 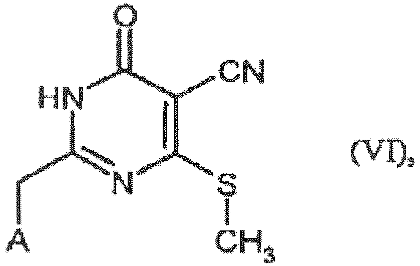 dans laquelle
A a les significations mentionnées ci-dessus,
et ensuite, on fait réagir celui-ci à température élevée dans un solvant inerte ou également en l'absence de solvant avec un composé de formule (III)
et on fait réagir les composés de formule (I) respectivement obtenus éventuellement avec (i) les solvants et/ou (ii) les bases ou acides correspondants en leurs solvates, leurs sels et/ou les solvates de leurs sels.

2. Procédé selon la revendication 1, dans lequel
A est un groupe phényle, hétéroaryle qui représente un radical aromatique monocyclique comportant 5 à 6 atomes cycliques et jusqu'à 3 hétéroatomes de la série S, O et/ou N, ou un groupe de formule 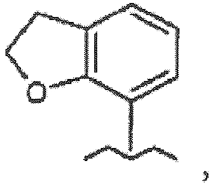 les groupes phényle et hétéroaryle étant substitués éventuellement par jusqu'à 2 radicaux choisis indépendamment l'un de l'autre dans le groupe hétéroaryle qui représente un radical aromatique monocyclique comportant 5 à 6 atomes cycliques et jusqu'à 3 hétéroatomes de la série S, O et/ou N, un halogène, un groupe alkyle en C₁ à C₄, un groupe alcoxy en C₁ à C₄, trifluorométhyle, trifluorométhoxy, benzyloxy et benzyle,
le groupe alkyle en C₁ à C₄ étant substitué éventuellement par un groupe de formule -NR³R⁴ dans lequel R³ est un groupe alkyle en C₁ à C₄ et R⁴ est un atome d'hydrogène ou un groupe alcoxy en C₁ à C₄-alkyle (en C₁ à C₄), et
le groupe hétéroaryle est éventuellement substitué par un groupe alcoxy en C₁ à C₄,
R¹ est un groupe cycloalkyle en C₃ à C₆, un groupe alkyle en C₁ à C₄, un groupe alcoxy en C₁ à C₄-alkyle (en C₁ à C₄), benzyle ou un groupe de formule 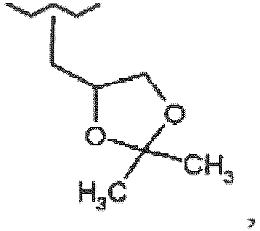
dans laquelle le groupe cycloalkyle en C₃ à C₆ est substitué éventuellement par un groupe hydroxy, un groupe alkyle en C₁ à C₄ ou trifluorométhyle,
le groupe alkyle en C₁ à C₄ est substitué éventuellement par un groupe hétéroaryle qui représente un radical aromatique monocyclique comportant 5 à 6 atomes cycliques et jusqu'à 3 hétéroatomes de la série S, O et/ou N, un groupe cycloalkyle en C₃ à C₆ ou hydroxy,
et le groupe benzyle est substitué éventuellement par un groupe alcoxy en C₁ à C₄ ou un atome d'halogène,
R² est un atome d'hydrogène
ou
R¹ et R² conjointement avec l'atome d'azote auquel ils sont liés, forment un groupe hétérocyclyle de 5 à 6 chaînons, ledit groupe hétérocyclyle de 5 à 6 chaînons étant un radical hétérocyclique, monocyclique, saturé ou partiellement insaturé comportant 5 à 6 atomes cycliques et jusqu'à 2 hétéroatomes de la série N, O, S, qui est substitué éventuellement par jusqu'à 2 substituants choisis indépendamment l'un de l'autre dans le groupe comprenant les groupes alkyle en C₁ à C₄, hydroxy, cyano, oxo, hétéroaryle qui représente un radical aromatique monocyclique comportant 5 à 6 atomes cycliques et jusqu'à 3 hétéroatomes de la série S, O et/ou N, benzyle, formyle, alkylcarbonyle en C₁ à C₄ et l'un des groupes suivants 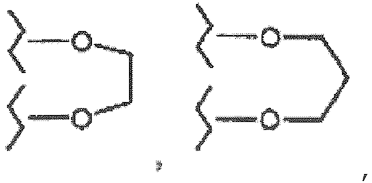 qui sont liés par les deux atomes d'oxygène à l'un des atomes de carbone dans l'hétérocycle,
le groupe alkyle en C₁ à C₄ étant substitué éventuellement par un groupe hydroxy ou hétéroaryle qui est un radical aromatique monocyclique comportant 5 à 6 atomes cycliques et jusqu'à 3 hétéroatomes de la série S, O et/ou N
et leurs sels, leurs solvates et/ou les solvates de leurs sels.

3. Procédé selon les revendications 1 ou 2, dans lequel
A est un groupe phényle, thiényle ou un groupe
de formule 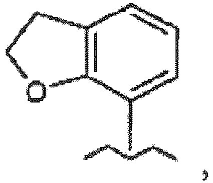 les groupes phényle et thiényle étant substitués éventuellement par jusqu'à 2 radicaux choisis indépendamment l'un de l'autre dans le groupe comprenant un groupe pyridyle, un atome de fluor, de chlore, de brome, un groupe alkyle en C₁ à C₄, alcoxy en C₁ à C₄, trifluorométhyle, trifluorométhoxy, benzyloxy et benzyle,
le groupe alkyle en C₁ à C₄ étant substitué éventuellement par un groupe de formule -NR³R⁴ dans lequel R³ est un groupe alkyle en C₁ à C₄ et R⁴ est un atome d'hydrogène ou un groupe alcoxy en C₁ à C₄-alkyle (en C₁ à C₄), et
le groupe pyridyle est éventuellement substitué par un groupe alcoxy en C₁ à C₄,
R¹ est un groupe cycloalkyle en C₃ à C₆, un groupe alkyle en C₁ à C₄, un groupe alcoxy en C₁ à C₄-alkyle (en C₁ à C₄), benzyle ou un groupe de formule 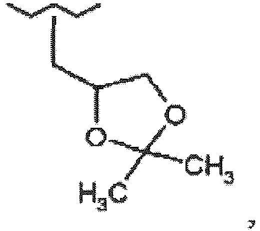
dans laquelle le groupe cycloalkyle en C₃ à C₆ est substitué éventuellement par un groupe hydroxy, alkyle en C₁ à C₄ ou trifluorométhyle,
le groupe alkyle en C₁ à C₄ est substitué éventuellement par un groupe pyridyle, cycloalkyle en C₃ à C₆ ou hydroxy,
et le groupe benzyle est substitué éventuellement par un groupe alcoxy en C₁ à C₄, un atome de fluor, de chlore ou de brome,
R² est un atome d'hydrogène
ou
R¹ et R² conjointement avec l'atome d'azote auquel ils sont liés, forment un groupe hétérocyclyle de 5 à 6 chaînons choisi dans le groupe comprenant les groupes pyrrolidinyle, pipéridinyle, pipérazinyle et morpholinyle, qui est substitué éventuellement par jusqu'à 2 substituants choisis indépendamment l'un de l'autre dans le groupe comprenant les groupes alkyle en C₁ à C₄, hydroxy, cyano, oxo, hétéroaryle qui représente un radical aromatique monocyclique comportant 5 à 6 atomes cycliques et jusqu'à 3 hétéroatomes de la série S, O et/ou N, benzyle, formyle, alkylcarbonyle en C₁ à C₄ et l'un des groupes suivants 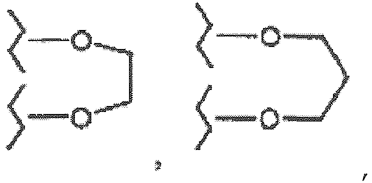 qui sont liés par les deux atomes d'oxygène à l'un des atomes de carbone dans l'hétérocycle,
le groupe alkyle en C₁ à C₄ étant substitué éventuellement par un groupe hydroxy ou pyridyle
et leurs sels, leurs solvates et/ou les solvates de leurs sels.

4. Procédé selon les revendications 1, 2 et 3, dans lequel
A est un groupe phényle, thiényle ou un groupe de formule 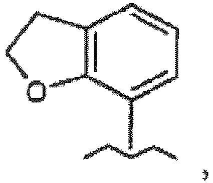 le groupe phényle étant substitué éventuellement par jusqu'à 2 radicaux choisis indépendamment l'un de l'autre dans le groupe comprenant un groupe pyridyle, un atome de fluor, de chlore, un groupe méthyle, méthoxy, éthoxy, trifluorométhyle, trifluorométhoxy, benzyloxy et benzyle,
le groupe méthyle étant substitué éventuellement par un groupe de formule -NR³R⁴ dans lequel R³ est un groupe méthyle et R⁴ est un atome d'hydrogène ou un groupe 2-méthoxyéthyle, et
le groupe pyridyle est éventuellement substitué par un groupe méthoxy,
R¹ est un groupe cycloalkyle en C₃ à C₆, méthyle, éthyle, propyle, 2-méthoxyéthyle, benzyle ou un groupe de formule 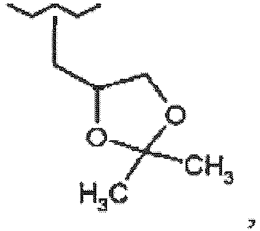
dans laquelle le groupe cycloalkyle en C₃ à C₆ est substitué éventuellement par un groupe hydroxy, méthyle ou trifluorométhyle,
le groupe méthyle, éthyle, propyle est substitué éventuellement par un groupe pyridyle, cyclopropyle ou hydroxy,
et le groupe benzyle est substitué éventuellement par un groupe méthoxy, éthoxy, un atome de fluor ou de chlore,
R² est un atome d'hydrogène
ou
R¹ et R² conjointement avec l'atome d'azote auquel ils sont liés, forment un groupe hétérocyclyle de 5 à 6 chaînons choisi dans le groupe comprenant les groupes pyrrolidinyle, pipéridinyle, pipérazinyle et morpholinyle, qui est substitué éventuellement par jusqu'à 2 substituants choisis indépendamment l'un de l'autre dans le groupe comprenant les groupes méthyle, éthyle, propyle, tert-butyle, hydroxy, cyano, oxo, pyridyle, benzyle, formyle, méthyl-carbonyle, éthylcarbonyle, propylcarbonyle et l'un des groupes suivants 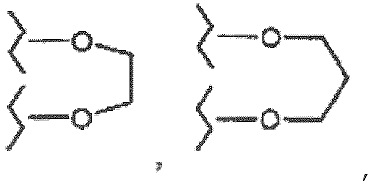 qui sont liés par les deux atomes d'oxygène à l'un des atomes de carbone dans l'hétérocycle,
les groupes méthyle, éthyle et propyle étant substitués éventuellement par un groupe hydroxy ou pyridyle,
et leurs sels, leurs solvates et/ou les solvates de leurs sels.

5. Composés de formule 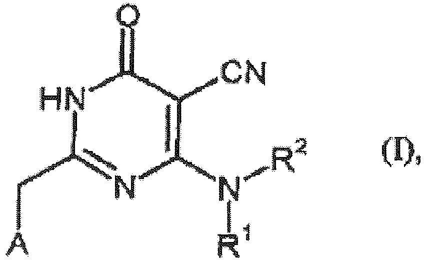 dans laquelle les radicaux A, R¹ et R² sont tels que définis dans les revendications 1 à 4 et leurs sels, leurs solvates et/ou les solvates de leurs sels, le composé suivant étant exclu :

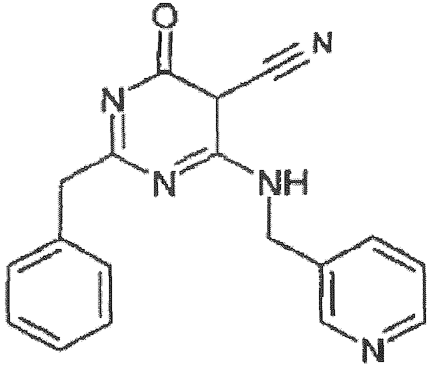

6. Composé choisi dans le groupe constitué de
| Composé | Structure | Composé | Structure |
|---|---|---|---|
| 1 | 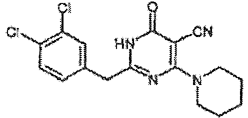 | 2 | 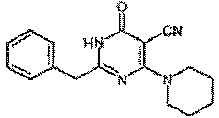 |
| 3 | 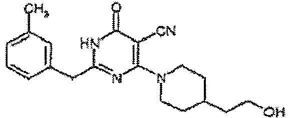 | 4 | 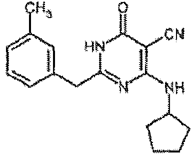 |
| 5 | 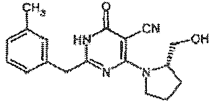 | 6 | 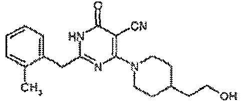 |
| 7 | 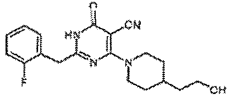 | 8 | 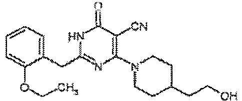 |
| 9 | 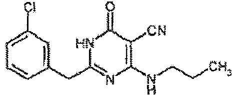 | 10 | 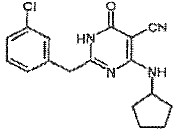 |
| 11 | 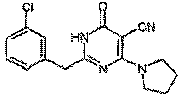 | 12 | 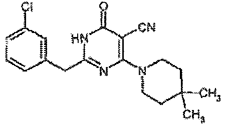 |
| 13 | 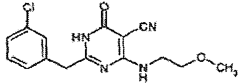 | 14 | 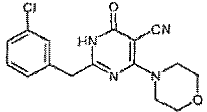 |
| 15 | 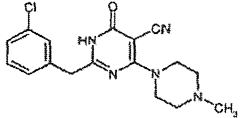 | 16 | 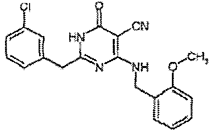 |
| 17 | 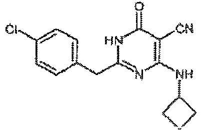 | 18 | 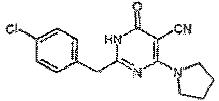 |
| 19 | 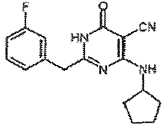 | 20 | 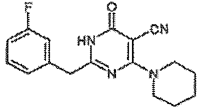 |
| 21 | 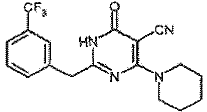 | 22 | 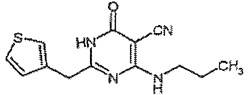 |
| 23 | 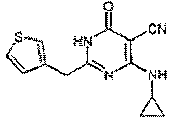 | 24 | 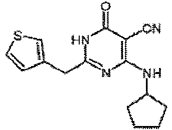 |
| 25 | 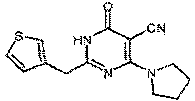 | 26 | 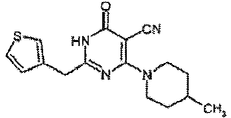 |
| 27 | 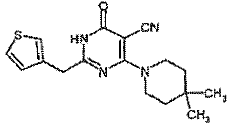 | 28 | 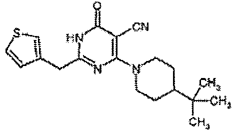 |
| 29 | 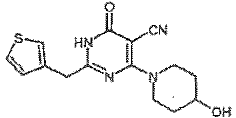 | 30 | 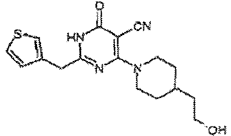 |
| 31 | 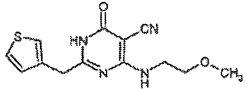 | 32 | 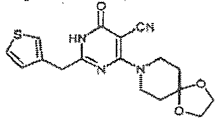 |
| 33 | 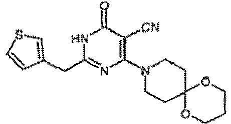 | 34 | 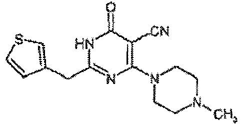 |
| 35 | 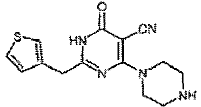 | 36 | 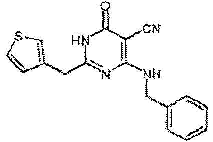 |
| 37 | 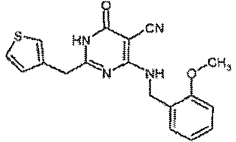 | 38 | 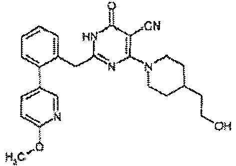 |
| 39 | 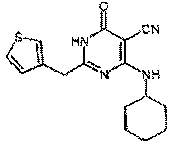 | 40 | 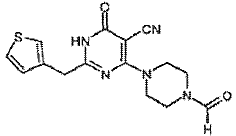 |
| 41 | 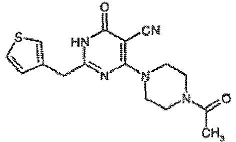 | 42 | 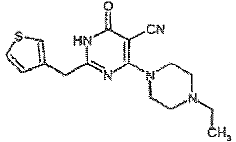 |
| 43 | 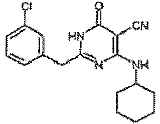 | 44 | 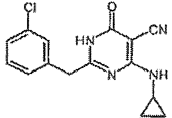 |
| 45 | 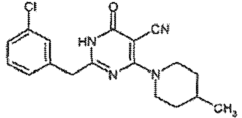 | 46 | 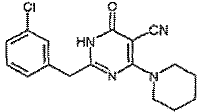 |
| 47 | 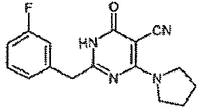 | 48 | 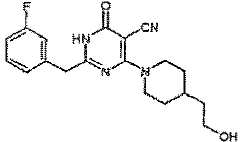 |
| 49 | 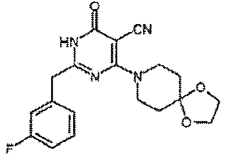 | 50 | 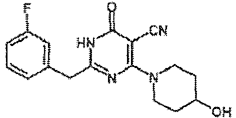 |
| 51 | 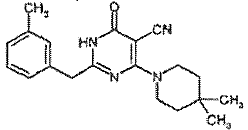 | 52 | 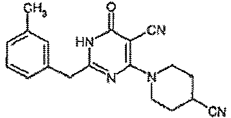 |
| 53 | 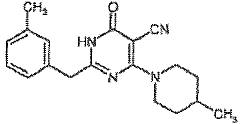 | 54 | 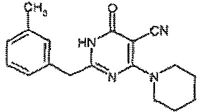 |
| 55 | 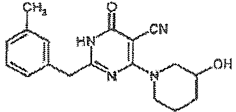 | 56 | 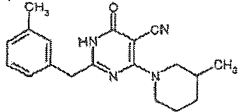 |
| 57 | 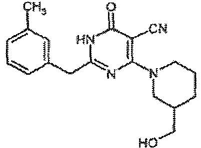 | 58 | 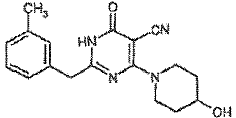 |
| 59 | 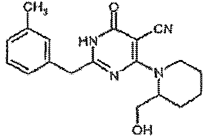 | 60 | 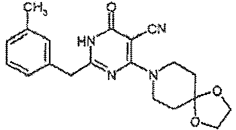 |
| 61 | 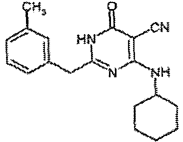 | 62 | 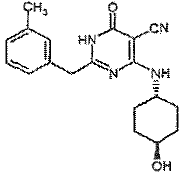 |
| 63 | 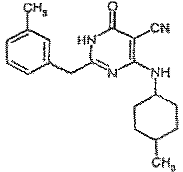 | 64 | 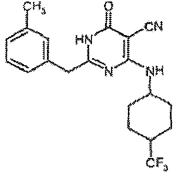 |
| 65 | 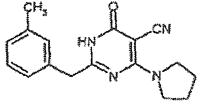 | 66 | 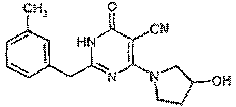 |
| 67 | 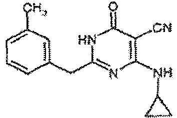 | 68 | 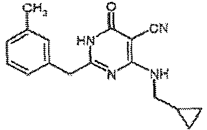 |
| 69 | 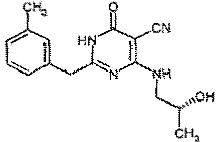 | 70 | 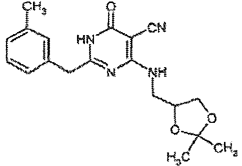 |
| 71 | 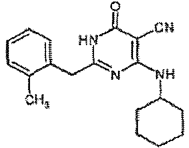 | 72 | 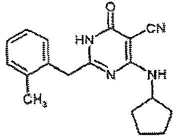 |
| 13 | 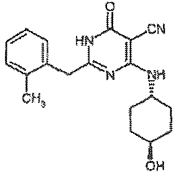 | 74 | 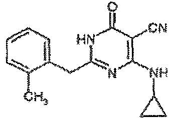 |
| 75 | 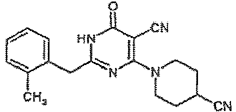 | 76 | 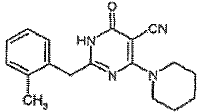 |
| 77 | 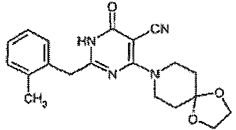 | 78 | 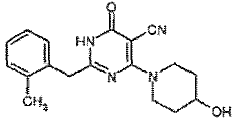 |
| 79 | 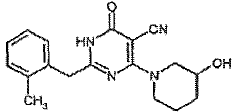 | 80 | 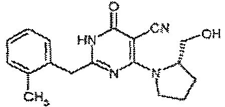 |
| 81 | 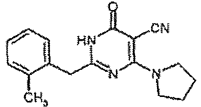 | 82 | 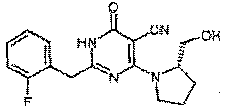 |
| 83 | 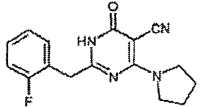 | 84 | 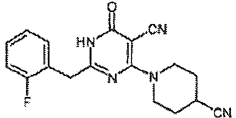 |
| 85 | 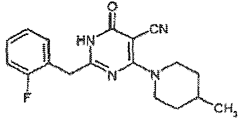 | 86 | 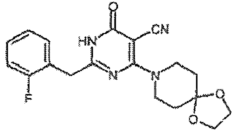 |
| 87 | 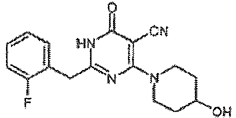 | 88 | 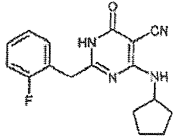 |
| 89 | 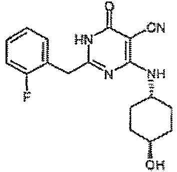 | 90 | 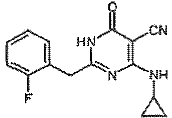 |
| 91 | 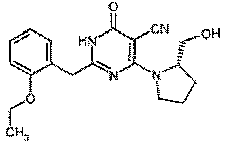 | 92 | 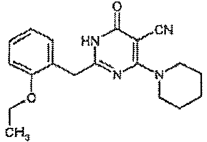 |
| 93 | 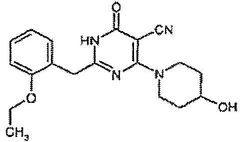 | 94 | 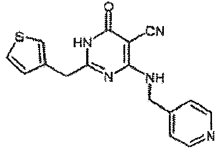 |
| 95 | 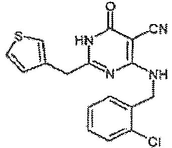 | 96 | 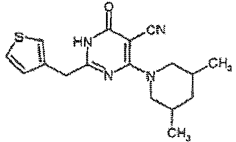 |
| 97 | 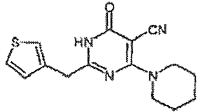 | 98 | 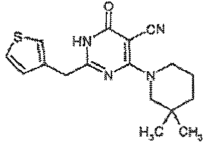 |
| 99 | 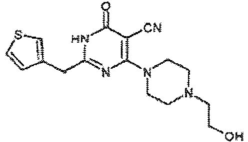 | 100 | 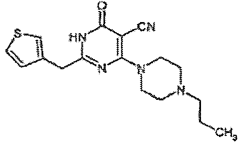 |
| 101 | 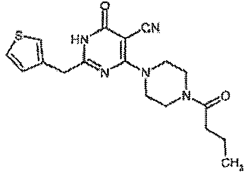 | 102 | 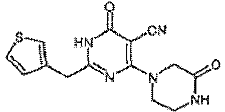 |
| 103 | 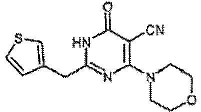 | 104 | 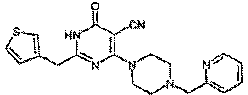 |
| 105 | 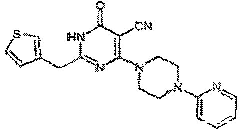 | 106 | 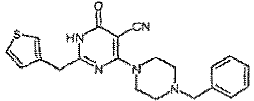 |
| 107 | 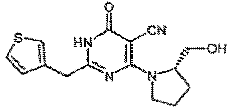 | 108 | 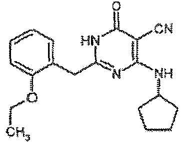 |
| 109 | 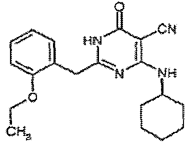 | 110 | 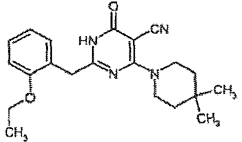 |
| 111 | 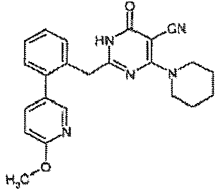 | 112 | 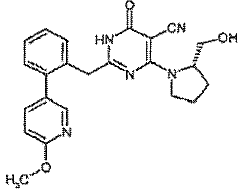 |
| 113 | 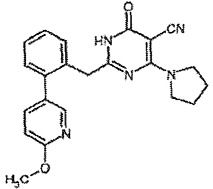 | 114 | 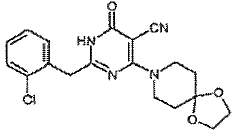 |
| 115 | 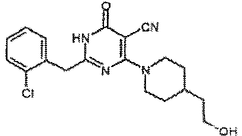 | 116 | 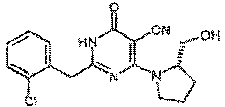 |
| 117 | 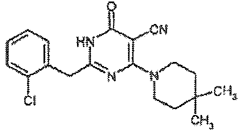 | 118 | 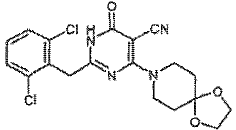 |
| 119 | 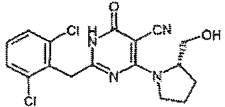 | 120 | 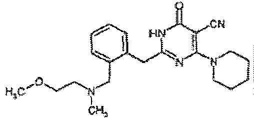 |
| 121 | 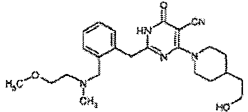 | 122 | 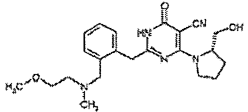 |
| 123 | 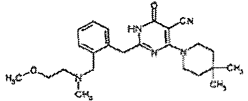 | 124 | 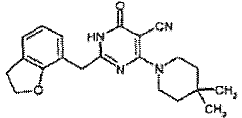 |
| 125 | 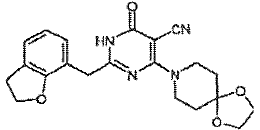 | 126 | 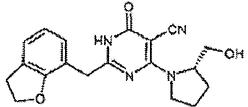 |
| 127 | 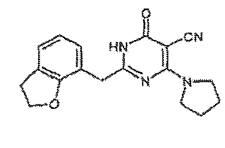 | 128 | 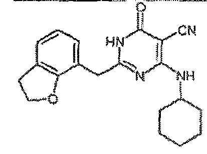 |
| 129 | 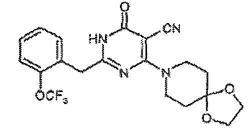 | 130 | 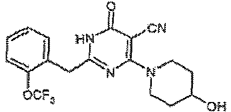 |
| 131 | 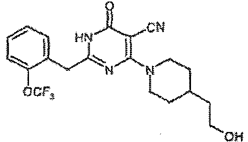 | 132 | 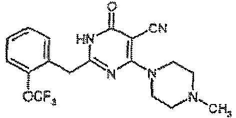 |
| 133 | 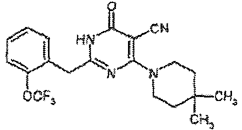 | 134 | 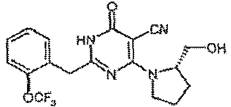 |
| 135 | 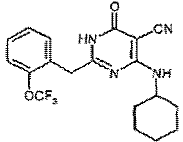 | 136 | 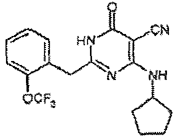 |
| 137 | 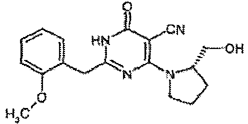 | 138 | 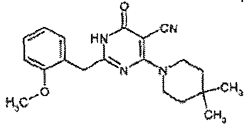 |
| 139 | 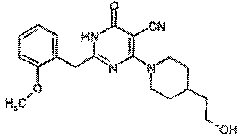 | 140 | 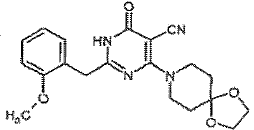 |
| 141 | 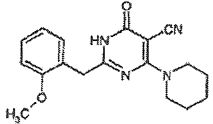 | 142 | 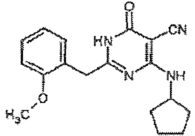 |
| 143 | 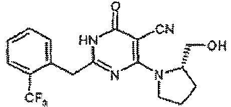 | 144 | 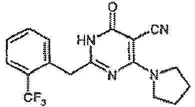 |
| 145 | 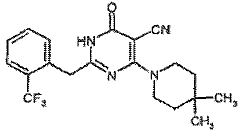 | 146 | 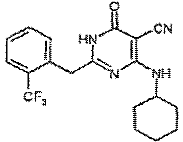 |
| 147 | 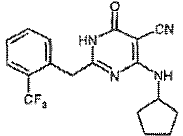 | 148 | 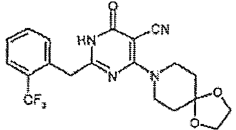 |
| 149 | 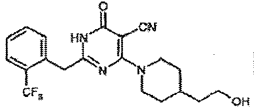 | 150 | 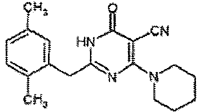 |
| 151 | 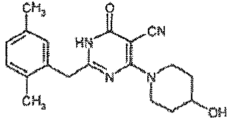 | 152 | 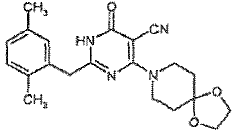 |
| 153 | 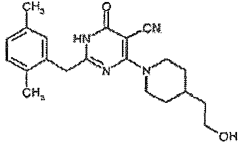 | 154 | 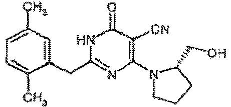 |
| 155 | 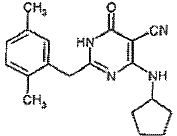 | 156 | 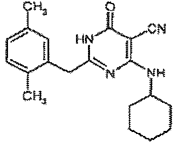 |
| 157 | 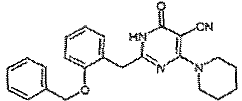 | 158 | 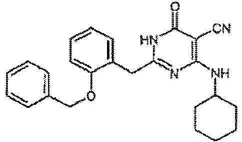 |
| 159 | 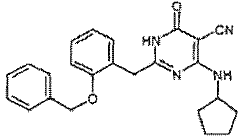 | 160 | 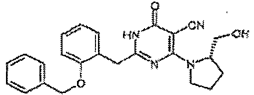 |
| 161 | 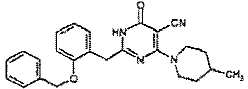 | 162 | 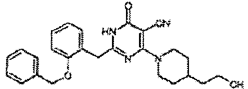 |
| 163 | 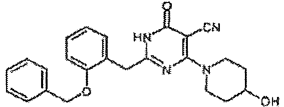 | 164 | 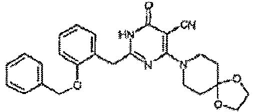 |

7. Utilisation des composés selon l'une des revendications 5 ou 6, pour la production d'un médicament pour la prophylaxie et/ou le traitement des troubles de la perception, de concentration, d'apprentissage et/ou de mémorisation.

8. Utilisation selon la revendication 7, dans laquelle le trouble est une conséquence de la maladie d'Alzheimer.

9. Utilisation des composés selon l'une des revendications 5 ou 6, pour la production d'un médicament pour améliorer la perception, la concentration, l'apprentissage et/ou la mémorisation.
